# EUROPEAN PATENT APPLICATION

(11) **EP 4 011 392 A1**
(43) Date of publication of application: **15.06.2022**
(21) Application number: 20849639.8
(22) Date of filing: 05.08.2020
(51) Int. Cl.: A61K 45/00, A61P 25/28

(54) **METHOD FOR TREATING ALZHEIMER'S DISEASE BY REGULATING INTESTINAL MICROORGANISMS**

(30) Priority: 06.08.2019 CN 201910720486
(71) Applicant: Shanghai Green Valley Pharmaceutical Co., Ltd., Pudong New Area, Shanghai 201203 (CN); Shanghai Institute of Materia Medica, Chinese Academy of Sciences, Shanghai 201203 (CN)
(72) Inventor: GENG, Meiyu, Shanghai 201203 (CN); SUN, Guangqiang, Shanghai 201203 (CN); WANG, Xinyi, Shanghai 201203 (CN); ZHANG, Jing, Shanghai 201203 (CN); FENG, Teng, Shanghai 201203 (CN)
(74) Representative: Murgitroyd & Company
(86) International application number: PCT/CN2020/107221
(87) International publication number: WO 2021/023237

(57) **Abstract**

The present invention relates to the treatment of Alzheimer's disease. Provided is the use of an reagent for regulating the relative abundance of intestinal microorganisms in the preparation of a medicament for treating Alzheimer's disease in a subjet.

## Description

### TECHNICAL FIELD

The present invention relates to the treatment of Alzheimer's disease (AD). More specifically, the present invention relates to the use of brain-gut axis association to inhibit the progression of Alzheimer's disease.

### BACKGROUND OF THE INVENTION

Alzheimer's disease (AD) is a progressive neurodegenerative disease with insidious onset. In clinic, it is characterized by general dementia such as memory impairment, aphasia, apraxia, agnosia, impairment of visuospatial skills, executive dysfunction, and personality and behavior changes. The two pathological features of Alzheimer's disease are extracellular β-amyloid deposits (senile plaques) and intracellular neurofibrillary tangles (paired helical filament). β-amyloid deposits and neurofibrillary tangles result in the loss of synapses and neurons, which leads to severe atrophy in damaged areas of the brain, typically starting in the temporal lobe. The mechanism of this damage caused by β-amyloid peptides and neurofibrillary tangles has not been fully understood.

The mannuronic acid oligosaccharides developed by the research team led by Geng Meiyu, a researcher at the Institute of Pharmaceutical Innovation of the Chinese Academy of Sciences/Shanghai Institute of Materia Medica, is a new oral anti-Alzheimer's disease (AD) innovative drug with independent intellectual property rights. On July 17, 2018, the clinical Phase III unblind trial results showed that mannuronic acid oligosaccharides reached expectations in terms of the main efficacy indicators for cognitive function improvement, which has extremely significant statistical and clinical significance. In addition, the incidence of adverse events is comparable to that of the placebo group, and it has good safety and is suitable for long-term use. Mannouronic acid oligosaccharides have become the first drug in the global AD treatment field to succeed in a phase III clinical trial in 16 years.

Regarding mannuronic acid oligosaccharides, many related Chinese patents have been submitted. CN2017114675966 describes a composition of mannuronic diacid. CN2016100697039 describes a preparation method of oligomannuronic diacid. CN2018107134113 describes the use of a composition of mannuronic diacid in the treatment of Parkinson's disease. CN2015104243401 describes the use of mannuronic acid oligosaccharides with a carboxyl group at postion 1 from the reducing end and their derivatives in the treatment of Parkinson's disease. These patents are incorporated herein in their entirety by reference.

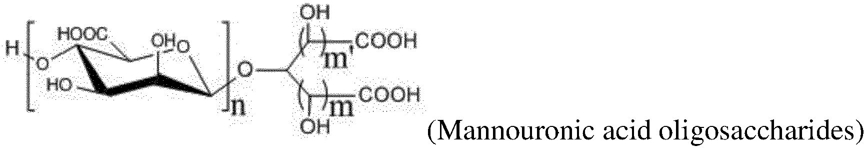

More drugs that can be used to treat Alzheimer's disease are needed in the art.

### SUMMARY OF THE INVENTION

In the present invention, the inventors provide a causal relationship between the gut microbiota dysbiosis and neuroinflammation in the progression of AD. Specifically, changes in the composition of the gut microbiota can lead to the peripheral accumulation of metabolites of the flora, such as phenylalanine and isoleucine. It promotes the proliferation and differentiation of peripheral pro-inflammatory-type 1 T helper (Th1) cells in the progression of AD. Peripheral immune cells infiltrate the brain and enhance neuroinflammation. Importantly, the elevation of such as phenylalanine and isoleucine in peripheral blood was confirmed in two independent cohorts of patients with mild cognitive impairment (MCI) caused by AD. The inventors also showed that the mannuronic acid oligosaccharides, which exhibited reliable and consistent cognitive improvement in phase III clinical trials in China, reshape the balance of gut flora, reduce the accumulation of amino acid metabolites of the flora in the blood, and inhibit neuroinflammation. In general, the inventor's findings highlight the role of neuroinflammation promoted by intestinal dysbiosis in the progression of AD, and propose a new strategy for AD treatment by intervening in the brain-gut axis.

In one aspect, the present invention provides the use of an agent for regulating the relative abundance of gut microbes in the manufacture of a medicament for treating Alzheimer's disease in a subject.

In another aspect, the present invention provides a pharmaceutical composition for treating Alzheimer's disease in a subject comprising an effective amount of an agent for regulating the relative abundance of gut microbes.

In still another aspect, the present invention provides a method for screening a drug candidate that can be used to treat Alzheimer's disease comprising:
a) administering a test agent to *in vivo* or *in vitro* models with gut microbes, and
b) selecting the test agent that regulates the relative abundance of gut microbes as the drug candidate that can be used to treat Alzheimer's disease.

In still another aspect, the present invention provides a method for establishing an animal model of Alzheimer's disease comprising administering an agent for regulating the relative abundance of gut microbes to an animal, so that the relative abundance of gut microbes of the animal is regulated by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; or close to or reach the relative abundance of the corresponding gut microbes of the corresponding animal model having Alzheimer's disease.

In still another aspect, the present invention provides a method for treating a patienthaving Alzheimer's disease comprising:
(a) detecting the relative abundance of gut microbes of the patient and comparing it with the relative abundance of the corresponding gut microbes of the corresponding normal population to select gut microorganism of which the relative abundance is different from that of the corresponding gut microorganism of the corresponding normal population;
(b) administering an agent for regulating the relative abundance of gut microbes to the patient to regulate the relative abundance of selected gut microorganism to make them close to or reach the relative abundance of the corresponding gut microorganism of the corresponding normal population.

### BRIEF DESCRIPTION OF THE DRAWINGS

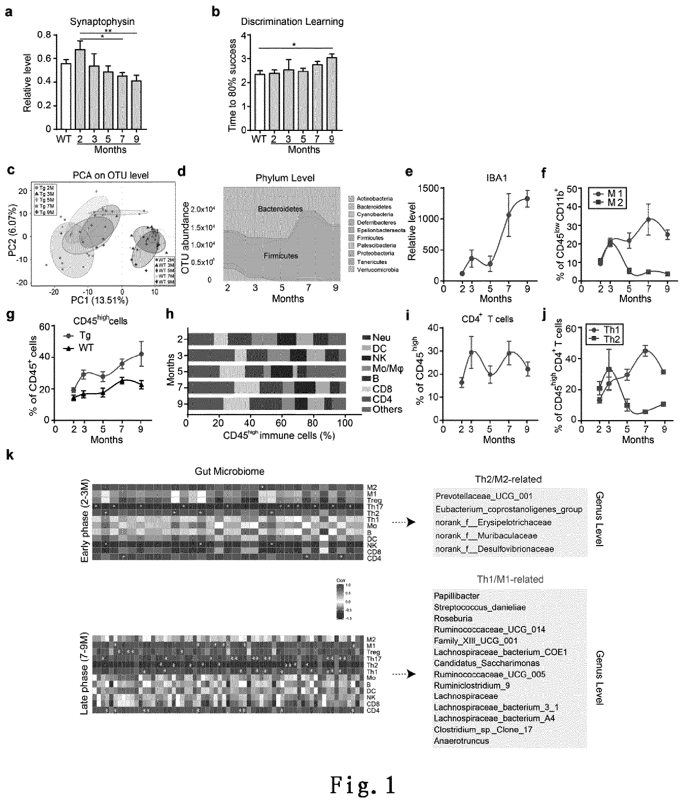

Figure 1 Gut dysbiosis and immune cell changes during disease progression in 5XFAD transgenic (Tg) mice.
   (a) Changes in the relative RNA expression levels of synaptophysin in the hippocampus of 5XFAD transgenic (Tg) mice at 2, 3, 5, 7 and 9 months and in wild-type (WT) mice at 2 months (n = 5-12). The data are presented as mean ± standard error of the mean (mean ± sem) relative to the expression level of actin. ^{∗}P < 0.05, ^{∗∗}P < 0.01 by one-way ANOVA (F (5, 43) = 2.952).
   (b) Changes in the time out of 10⁴ s taken to achieve 80% success (see Methods) in a test to evaluate the discrimination learning abilities of 5XFAD transgenic (Tg) mice at 2, 3, 5, 7, and 9 months and wild-type (WT) mice at 2 months (n = 4-8). Data are presented as mean ± standard error of the mean (mean ± sem). ^{∗}P < 0.05 by Student's t-test. s, seconds.
   (c) Principal component analysis (PCA) of the gut microbiome composition of WT and 5XFAD transgenic (Tg) mice on the operational taxonomic unit (OTU) level at different time points (n = 4-10). The shapes and colours of the points indicate samples from each individual from various months. The coloured ellipses indicate 0.95 confidence interval (CI) ranges within each tested group. M, months.
   (d) Relative abundance changes of operational taxonomic units (OTUs) in the overall population in the gut microbiome of 5XFAD transgenic (Tg) mice at various months, coloured at the phylum level on a stream graph (n = 4-10). The two most abundant phyla, *Bacteroidetes* and *Firmicutes,* are labelled on the graph. Colours indicate different phyla of the gut microbiota.
   (e) Changes in the positive densities of IBA1 immune-fluorescent staining, reflecting activation of microglial cells in the hippocampus of 5XFAD transgenic (Tg) mice at 2, 3, 5, 7, and 9 months relative to the values of 2-month-old wild-type (WT) mice (n = 2-7). The data are presented as mean ± standard error of the mean (mean ± sem); lines are fitted with a cubic spline. The value of IBA1 on the Y-axis is the relative value of the fluorescent staining expression of the transgenic animal at each time point relative to the fluorescent staining expression of the wild-type animal at the same time point.
   (f) Changes in activated M1 and M2 type microglia detected in the whole-brain homogenates of 5XFAD transgenic (Tg) mice at 2, 3, 5, 7 and 9 months (n = 4-8). M1-type microglia (CD45^{low}CD11b⁺CX3CR1⁺Siglec-H⁺F4/80⁺CD86⁺) and M2-type (CD45^{low}CD11b⁺CX3CR1⁺Siglec-H⁺F4/80⁺CD206⁺) microglia were detected by flow cytometry, and their cell counts are presented relative to the frequency of CD45^{low}CD11b⁺ cells. Red points and lines: M1 microglia. Green points and lines: M2 microglia. The data are presented as mean ± standard error of the mean (mean ± sem); lines are fitted with a cubic spline algorithm.
   (g) Changes in infiltrating cells (CD45^{high}) detected in the whole-brain homogenates of 5XFAD transgenic (Tg) mice (red points and lines) and WT mice (black points and lines) at different time points as detected by flow cytometry (Tg mice: 2 months old, n=5; 3 months old, n=4; 5 months old, n=4; 7 months old, n=7; 9 months old, n=3. WT mice: n=6.). Cell counts are presented relative to the frequency of CD45⁺ cells and formatted as mean ± standard error of the mean (mean ± sem). Lines are fitted with a cubic spline algorithm.
   (h) Changes in CD45^{high} cells in 5XFAD transgenic (Tg) mice at different time points (n = 4-8). On the barplot, cell counts are presented relative to the frequency of CD45^{high} cells. Colours indicate different subtypes of CD45^{high} cells: Neu, neutrophils; DC, dendritic cells; NK, natural killer cells; Mo/Mφ : monocytes and macrophages; B, B cells; Others, unclassified cells.
   (i) Changes in infiltrating CD4 T cells (CD45^{high}CD4⁺) detected in the whole-brain homogenates of 5XFAD transgenic (Tg) mice (red points and lines) at 2, 3, 5, 7 and 9 months as detected by flow cytometry (n = 4-8). Cell counts are presented relative to the frequency of CD45^{high} cells and formatted as mean ± standard error of the mean (mean ± sem). Lines are fitted with a cubic spline.
   (j) Changes in infiltrating peripheral Th1 and Th2 cells detected in the whole-brain homogenates of 5XFAD transgenic (Tg) mice at 2, 3, 5, 7 and 9 months (n = 4-8). Th1 cells (CD45^{high}CD4⁺CXCR3⁺CCR6⁻) and Th2 cells (CD45^{high}CD4⁺CXCR3⁺CCR6⁻CCR4⁺) were detected by flow cytometry, and presented relative to the frequency of CD45^{high}CD4⁺ T cells. Red points and lines: Th1 cells. Green points and lines: Th2 cells. The data are presented as mean ± standard error of the mean (mean ± sem). Lines are fitted with a cubic spline.
   (k) Correlation of brain lymphocytes and gut microbiota represented at genus level during the Th2/M2-related stage and Th1/M1-related stage in the early (2-3 months) and late phase (7-9 months), respectively (left panels, *n*= 4-8). All bacteria significantly correlated with brain lymphocyte counts in 5XFAD mice are listed in the right-hand panel. Squares in red (positive correlation) or blue (negative correlation) with a yellow asterisk (^{∗}) indicate significant correlations with P-values < 0.05 measured by the Pearson parametric correlation test.

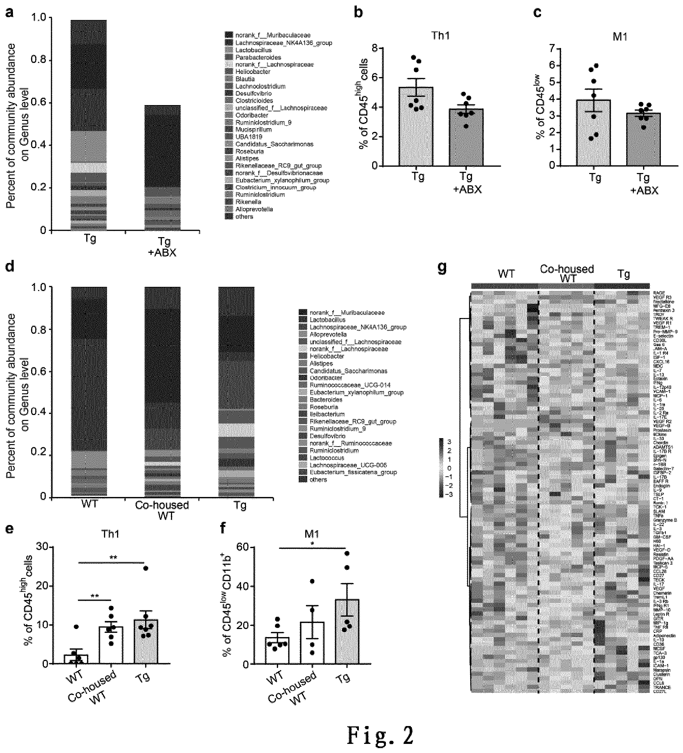

Figure 2. The gut microbiota is required for immune cell infiltration and microglial activation.
   (a) The effects of three-month oral gavage of antibiotics on the relative abundance of gut microbes in 7-month-old 5XFAD transgenic (Tg) mice (n = 6-7). ABX, a cocktail of mixed antibiotics composed of ampicillin (0.1 mg/mL), streptomycin (0.5 mg/mL) and colistin (0.1 mg/mL). Different genera of gut microbes are coloured differently, and their changes in relative abundance are presented on the barplot.
   (b-c) The effects of three-month oral gavage of antibiotics on the frequency of Th1 cells (b) and M1-type microglia (c) in the brain homogenate of 7-month-old 5XFAD transgenic (Tg) mice. Cell counts of Th1 cells (CD45^{high}CD4⁺CXCR3⁺CCR6⁻) are presented relative to the frequency of CD45^{high}CD4⁺cells (b), while those of M1-type microglia (CD45^{low}CD11b⁺CX3CR1⁺Siglec-H⁺F4/80⁺CD86⁺) are presented relative to the frequency of CD45^{low}CD11b⁺cells (c). Both are detected by flow cytometry. The data are presented as mean ± standard error of the mean (mean ± sem).
   (d) The relative abundance of gut microbes at the genus level in WT, co-housed WT and 5XFAD transgenic (Tg) mice (n = 6-7). All three groups of mice were at 7-month old. Different colours represent different genera. Co-housed WT: WT mice that were housed with Tg mice.
   (e-f) Changes in the frequency of Th1 cells (e) and M1 type microglia (f) in the brain homogenates of 7-month-old co-housed WT, WT and 5XFAD transgenic (Tg) mice (n = 6-7). Th1 cells (CD45^{high}CD4⁺CXCR3⁺CCR6⁻) are presented relative to the frequency of CD45^{high}CD4⁺cells (e), while the frequency of M1-type microglia (CD45^{low}CD11b⁺CX3CR1⁺Siglec-H⁺F4/80⁺CD86⁺) are presented relative to the frequency of CD45^{low}CD11b⁺cells (f). Both are detected by flow cytometry. The data are presented as mean ± standard error of the mean (mean ± sem). ^{∗}P < 0.05, ^{∗∗}P < 0.01 by Student's t-test.
   (g) Levels of cytokine proteins in the brain homogenates of WT, co-housed WT and 5XFAD transgenic (Tg) mice at 7-month old as detected by a cytokine antibody array (n = 6-7). All three groups of mice were 7 months old. The colors in the heat map indicate the relative cytokine levels normalized by Row Z-Score; red indicates cytokines that are upregulated, and blue indicates cytokines that are downregulated.

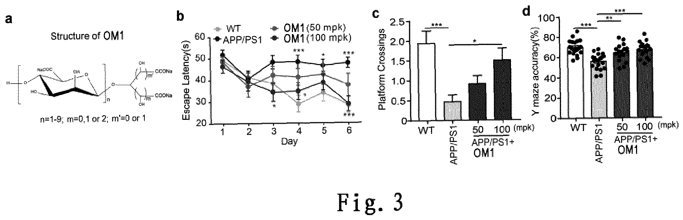

Figure 3. The effects of OM1 on behaviour changes in APP/PS1 mice models.
   (a) Structure of OM1. OM1 is a mixture of acidic linear oligosaccharides with degrees of polymerization ranging from dimers to decamers with an average molecular weight of approximately 1 kDa.
   (b) The escape latency time results of the Morris Water Maze (MWM) test as a measurement of spatial learning and memory in APP/PS1 mice. Nine-month-old APP/PS1 mice were treated with 50 mpk and 100 mpk of OM1 for 3 months until 12-month old. Then, the MWM test for spatial learning and memory abilities were conducted for 6 additional days. During the test, OM1 was continuously administrated. The escape latency time starting (seconds) was measured as one of the final readouts of the test (see Methods). Higher escape latency time shows that these mice will spend more time to reach the target, which indicates a more severely impaired spatial learning and memory ability (n = 11-14). The data are presented as mean ± standard error of the mean (mean ± sem). Black asterisk indicates the comparison between WT and APP/PS1 group. Blue asterisk indicates the comparison between OM1 (100 mpk) treatment and APP/PS1 group. ^{∗}P<0.05, ^{∗∗∗}P<0.001 by two-way ANOVA.
   (c) The number of platform-site crossovers in MWM test as a measurement of spatial learning and memory in APP/PS1 mice. Nine-month-old APP/PS1 mice were treated with 50 mpk and 100 mpk of OM1 for 3 months until 12-month old. Then, the MWM test for spatial learning and memory abilities were conducted for 6 additional days. During the test, OM1 was continuously administrated. The number of platform-site crossovers was measured as the other readout of the test (see Methods). Larger numbers of platform-site crossovers indicate less severely impaired spatial learning and memory ability (n = 11-17). ^{∗}P < 0.05, ^{∗∗∗}P < 0.001 by one-way ANOVA (F (3, 55) = 6.542).
   (d) The accuracy of spatial working memory as tested using the Y maze in APP/PS1 mice. Nine-month-old APP/PS1 mice were treated with 50 mpk and 100 mpk of OM1 for 3 months until 12-month old. Then the Y maze test was conducted. During the test, OM1 was continuously administrated. The accuracy of the Y maze was the ratio between the correct alternation and the total alternation (see "Materials and methods"). Higher accuracy indicates less severely inpaired working memory abilities. (n = 17-20). The data are presented as mean ± standard error of the mean (mean ± sem). ^{∗∗}P < 0.01, ^{∗∗∗}P <0.001 by one-way ANOVA (F (3, 71) = 12.39)

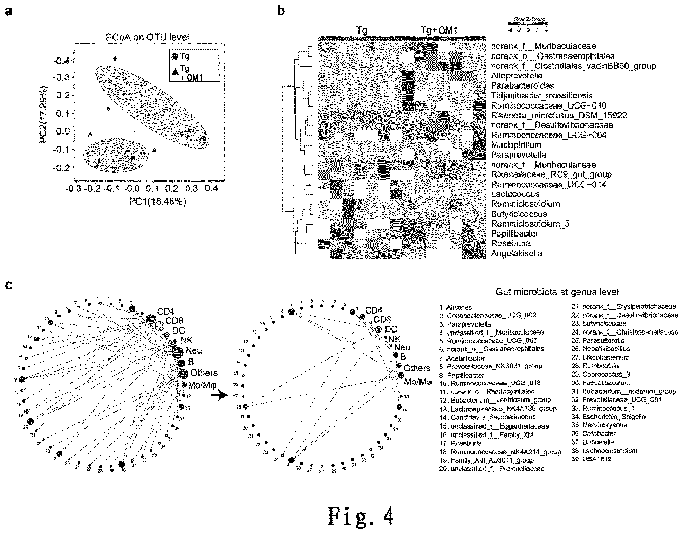

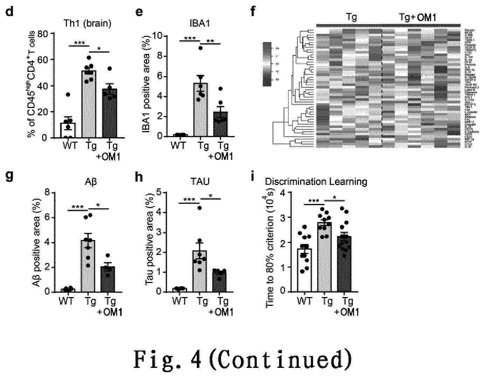

Figure 4. OM1 alleviates neuroinflammation by reconditioning the gut microbiota.
   (a) Principal coordinate analysis (PCoA) of the gut microbiome composition on the operational taxonomic unit (OTU) level based on the Bray-Curtis distance for 5XFAD (Tg) mice and OM1-treated Tg mice at 7-month old (n = 7). The shapes and colours of the points indicate samples from each individual. Coloured ellipses indicate 0.95 confidence interval (CI) ranges within each tested group. PC principal component.
   (b) Heatmap of significant gut microbiota changes represented at the genus level between 5XFAD (Tg) mice and OM1-treated Tg mice at 7-month old (n =17). Colours on the heatmap indicate the relative abundance of gut microbiota; red indicates bacteria that are upregulated, and blue indicates bacteria that are downregulated.
   (c) Changes in correlational links between the gut microbiome (designated with numbers near the blue circles) and brain lymphocytes (coloured circles) before (left) and after (right) oral gavage of OM1 in 7-month-old 5XFAD (Tg) mice. The right side lists the name of each gut microbiome.
   (d) Effect of OM1 treatment on the frequency of brain Th1 cells in 5XFAD (Tg) mice at 7 months old (n = 5-7). Th1 cell counts (CD45^{high}CD4⁺CXCR3⁺CCR6⁻) are presented relative to CD45^{high}CD4⁺ T cell counts detected by flow cytometry. The data are presented as mean ± standard error of the mean (mean ± sem). ^{∗}*P* < 0.05, *^{∗∗∗}P* < 0.001, by Student's t-test.
   (e) Effect of OM1 treatment on the positive signal density of IBA1 immunofluorescent staining detected in hippocampal slices from 5XFAD (Tg) mice at 7-month old, reflecting activation of microglial cells (*n* = 4-6). The data are presented as mean ± standard error of the mean (mean ± sem). ^{∗}*P* < 0.05, by one-way ANOVA (F (2, 15) = 21.94).
   (f) Effect of OM1 treatment on levels of cytokine proteins in the brain homogenates of 5XFAD (Tg) mice at 7-month old as detected by a cytokine antibody array (*n* = 5-6). The color on the heat map indicates the relative cytokine level normalized by Row Z-Score; red indicates cytokines that are upregulated, and blue indicates cytokines that are downregulated.
   (g-h) Effect of OM1 on Aβ-positive area (g) and tau-positive area (h) in the hippocampus of 5XFAD (Tg) mice at 7-month old, evaluated in brain slices (n = 4-7). The data are presented as mean ± standard error of the mean (mean ± sem). For Aβ analysis: ^{∗}P < 0.05, ^{∗∗}P < 0.01 (F (2, 14) = 22.78). For tau analysis: ^{∗}P < 0.05, ^{∗∗∗}P < 0.001 (F (2, 15) = 13.06) by one-way ANOVA.
   (i) Effects of OM1 on the time out of 10⁴ sec (s) taken to achieve 80% success (see Methods) in a test to evaluate the discrimination learning abilities of 5XFAD (Tg) mice at 7-month old (*n* = 10-13). Time means the time to reach the 80% performance level (seconds); the longer it takes, the severer the cognitive impairment is (see methods). ^{∗}*P* < 0.05, ^{∗∗∗}*P* < 0.001 by One-way ANOVA (F(2,31) = 9.751).

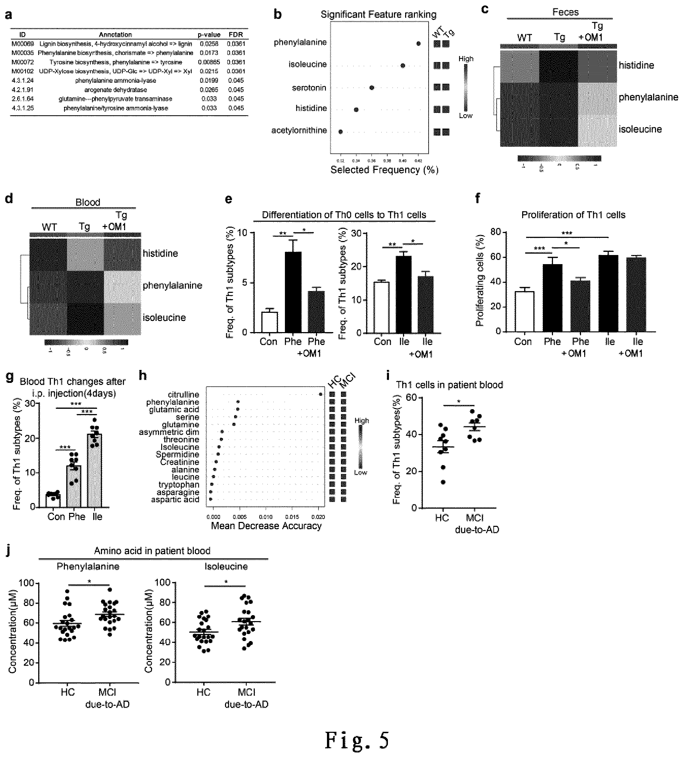

Figure 5. OM1 inhibits neuroinflammation by harnessing amino acid metabolism.
   (a) Pathway enrichment analysis of 31 kinds of faecal metabolites in 7-month-old 5XFAD (Tg) mice with or without OM1 treatment using MBROLE (*n* = 6-8). A list of the enrichment results is presented with KEGG modules and KEGG enzyme interactions which have been screened using a criterion of *P*-value <0.05.
   (b) Lists of blood amino acids between WT (n=30) and Tg (n=26) group could be distinguished in the disease progression period using the random forest model (Number of trees: 500; Number of predictors: 7; Randomness: On; See method).
   (c) Changes in histidine, phenylalanine and isoleucine levels in the feces of WT, Tg, and OM1-treated Tg mice (n = 6-11). Red, upregulated; Blue, downregulated.
   (d) Changes in histidine, phenylalanine and isoleucine levels in the blood of WT, Tg, and OM1-treated Tg mice (n = 6-7). Red, upregulated; Blue, downregulated.
   (e) The effects of OM1 on the differentiation of naive CD4⁺ T cells (Th0 cells) to Th1 cells induced by phenylalanine and isoleucine. Naive CD4⁺ T cells were cultured for 3 days with/without OM1 in the presence of phenylalanine (1 mM) or isoleucine (1 mM). The frequency of Th1 (CD4⁺IFN-γ⁺) cells was tested by flow cytometry. The data are presented as mean ± standard error of the mean (mean ± sem); *n*= 3 replicates per group. Left, ^{∗}*P* < 0.05, ^{∗∗}*P* < 0.01 by one-way ANOVA (F (2, 6) = 15.64). Right, ^{∗}*P* < 0.05, ^{∗∗}*P* < 0.01 by one-way ANOVA (F (2, 6) = 10.35).
   (f) The effects of OM1 on the proliferation of Th1 cells induced by phenylalanine and isoleucine. The naive CD4⁺ T cells were stained with CellTrace and cultured for 3 days with/without OM1 in the presence of phenylalanine (1 mM) and isoleucine (1 mM). The density of CellTrace fluorescence in Th1 (CD4⁺IFN-γ⁺) cells was tested by flow cytometry (see Methods). The data are presented as mean ± standard error of the mean (mean ± sem), *n = 3.* ^{∗}*P*< 0.05, ^{∗∗∗}*P* < 0.001 by one-way ANOVA (F (4, 9) = 28.34).
   (g) Blood Th1 cell changes after 4-day intraperitoneal (i.p.) injection of phenylalanine and isoleucine (*n* = 6). ^{∗∗∗}*P* < 0.001 by one-way ANOVA (F (2, 21) = 101.8).
   (h) Random forest classification of amino acid changes in healthy controls (HC) and mild cognitive impairment (MCI) due to AD patients. (first cohort, *n* = 9 for MCI due to AD, *n* = 18 for HC).
   (i) Frequency of Th1 cells in the blood of healthy controls (HC) and mild cognitive impairment (MCI) due to AD patients (first cohort, *n*= 8 for MCI due to AD, *n*= 9 for HC). ^{∗}*P* < 0.05 by Student's t-test. The vertical axis is the percentage of Th1 cells/CD4⁺ T cells.
   (j) Levels of phenylalanine and isoleucine in the blood of healthy controls (HC) and mild cognitive impairment (MCI) due to AD patients (second cohort, *n*= 22 for both groups). ^{∗}*P* < 0.05 by Student's t-test.

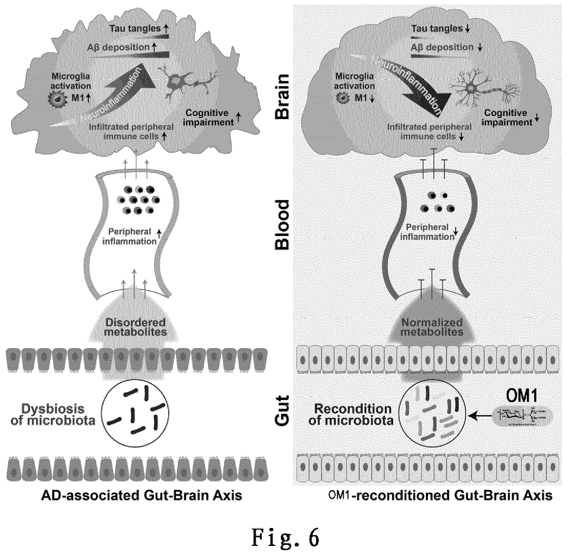

Figure 6 Schematic diagram of neuroinflammation in AD progression and the intervention strategy
   The alteration of the gut microbiota during AD progression causes disordered amino acid metabolism. It promotes the differentiation of naive CD4 T cells into Th1 cells in the blood. Meanwhile, amino acids and Th1 cells can infiltrate into the brain through blood circulation. Peripheral immune cell infiltration and microglia activation lead to pathological neuroinflammation in the brain, leading to cognitive impairment. Oral administration of OM1 can repair the gut microbiota, inhibit the abnormal production of amino acids, and reduce the infiltration of peripheral immune cells into the brain, and ultimately resolve neuroinflammation.
Figure 7
   (a-b) Changes in Aβ positive area (a) and phosphorylated-tau (p-TAU) positive area (b) in hippocampal slices from 5XFAD transgenic (Tg) mice (2-, 3-, 5-, 7-, and 9-month-old) versus 2-month-old wild-type (WT) mice (n = 2-7). Representative fluorescent images are presented; the scale bar represents 100 µm. Line charts summarize the results from all individual points relative to WT mice and are presented as the mean ± standard error of the mean (mean ± sem). Lines are fitted with a cubic spline algorithm. M, months.
   (c) Principal component (PC) 1 from principal component analysis (PCA) of the gut microbiome composition at the operational taxonomic unit (OTU) level in WT and 5XFAD transgenic (Tg) mice (2-, 3-, 5-, 7-, and 9-month-old) (n=4-10). Red points and lines, Tg mice; blue points and lines, WT mice. Naturally connect into a line without fitting. Use two-tailed Wilcoxon rank sum (ranked-sum) test; ^{∗} Indicates a significant difference compared with 2 months of age in the same group. ^{∗}P<0.05, ^{∗∗}P<0.01. # Indicates a significant difference compared to age-matched WT mice. #P<0.05; ##P<0.01; ###P<0.001.
   (d) Changes in the relative abundance of gut microbes at the family level in 5XFAD transgenic (Tg) mice (2-, 3-, 5-, 7-, and 9-month-old) (n=4-10). Different colours represent different families.
   (e) Cladogram of linear discriminant analysis effect size (LEfSe) analysis of the gut microbiome composition of 5XFAD transgenic (Tg) mice (2-, 3-, 5-, 7-, and 9-month-old) (n=4-10). The bacteria with the highest discriminatory power are labelled on the graph at each month. Colours indicate bacteria taxa that are enriched in each month. The inner to outer circles indicate different taxonomic levels (inner to outer: phylum, class, order, family and genus). M, months. *Verrucomicrobia; AlphaProteobacteria; Bacteroidetes; Prevotellaceae;* Erysipelotrichia; *Firmicutes; Lachnoclostridium.*
   (f) PCA analysis of the gut microbiome at the OTU level in APP/PS1 transgenic mice at 3-, 6-, 8-, 9-, 12- and 14-month-old (n=4-12). Colours and shapes indicate data from different months. Coloured ellipses indicate 0.95 confidence interval (CI) ranges within each tested group. PC, principal component.
   (g) Changes in the relative abundance of gut microbes at the phylum levels in APP/PS1 transgenic mice at 3-, 6-, 8-, 9-, 12- and 14-month-old (n=4-12). Colours represent different phyla. M, months.
   (h) Representative fluorescent images of changes in IBA1 positive are in hippocampal slices from 5XFAD transgenic (Tg) mice (2-, 3-, 5-, 7-, and 9-month-old) versus 2-month-old wild-type (WT) mice (n=2-7). The scale bar represents 500 µm in the small bracket (upper), 250 µm in the large bracket (down). M, months.
   (i) Changes in the frequency of infiltrating cells (CD45^{high}) detected in the whole-brain homogenates of APP/PS1 mice at 3-, 6-, 9- and 14-month-old as detected by flow cytometry (n=5-8). Cell counts are presented relative to the frequency of CD45⁺ cells and formatted as the mean ± standard error of the mean (mean ± sem). Lines are fitted with a cubic spline algorithm.
   (j) Changes in the frequency of infiltrating peripheral Th1 cells in the whole-brain homogenates of APP/PS1 mice at 3-, 6-, 9- and 14-month-old as detected by flow cytometry (n = 3-8). Th1 cells (CD45^{high}CD4⁺CXCR3⁺CCR6⁻) are presented relative to the frequency of CD45^{high}CD4⁺ T cells. The data are presented as the mean ± standard error of the mean (mean ± sem). Lines are fitted with a cubic spline algorithm.

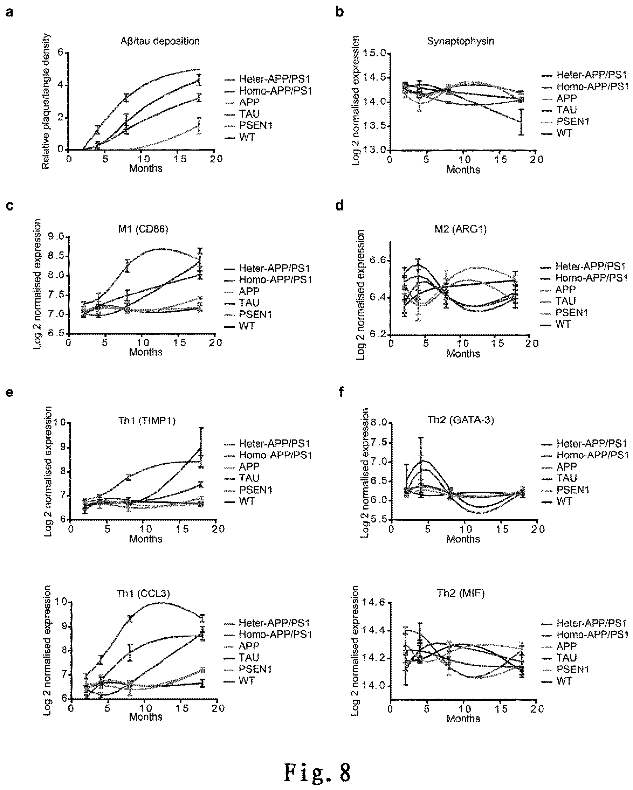

Figure 8 Changes in the hallmarks of AD in five animal models and wild-type (WT) mice at 2-, 4-, 8- and 18-month-old from the Mouseac database.
   (a) Changes in relative densities of Aβ plaque or tau neurofibrillary tangle (n = 4 for each group). The data are presented as the mean ± standard error of the mean (mean ± sem). Colours indicate different models; lines are fitted using a polynomial algorithm.
   (b) Changes in log₂ normalized expression levels of synaptophysin (n = 4 for each group). The data are presented as the mean ± standard error of the mean (mean ± sem). Colours indicate different models; lines are fitted using a polynomial algorithm.
   (c-d) Changes in log₂ normalized expression levels of CD86 and ARG1, representing changes in M1 and M2 cell counts (n = 4 for each group). The data are presented as the mean ± standard error of the mean (mean ± sem). Colours indicate different models; lines are fitted using a polynomial algorithm.
   (e-h) Changes in log₂ normalized expression levels of TIPM, CCL3, GATA-3 and MIF, representing changes in Th1 and Th2 counts (n = 4 for each group). The data are presented as the mean ± standard error of the mean (mean ± sem). Colours indicate different models; lines are fitted using a polynomial algorithm.
Figure 9
   (a) Effects of co-housing on the time out of 10⁴ seconds taken to achieve 80% success in a test to evaluate the discrimination learning abilities of WT, co-housed WT and 5XFAD transgenic (Tg) mice at 7-month-old (n = 5-8). Time spent means the time to reach the 80% performance level (seconds); the higher the time spent, the more severe the cognitive impairment is (see Methods).
   (b) Effects of faecal microbiota transplantation (FMT) on the counts of brain immune cells (Th1 and Th2) in WT recipient mice injected with Aβ (see Methods) using feces of either WT or 5XFAD transgenic (Tg) mice. Th1 cells (CD45^{high}CD4⁺CXCR3⁺CCR6⁻) and Th2 cells (CD45^{high}CD4⁺CXCR3⁻CCR6⁻CCR4⁺) are presented relative to CD45^{high}CD4⁺ T cells (n = 6-8); the data are presented as the mean ± standard error of the mean (mean ± sem). ^{∗}P<0.05, Student's t test.
   (c) Effects of FMT from 2-month old WT mice on the brain Th1 cells in 5XFAD transgenic (Tg) mice. (n = 6-7). The data are presented as the mean ± standard error of the mean (mean ± sem). ^{∗}P<0.05, Student's t test.
Figure 10
   (a) Cladogram of linear discriminant analysis effect size (LEfSe) analysis of the gut microbiome composition of 7-month-old 5XFAD transgenic (Tg) mice treated orally with OM1 (n=5-7). The phylum level of bacteria with the highest discriminatory power are labelled on the graph. Blue, bacteria enriched in 7-month-old Tg mice. Red, bacteria enriched in 7-month-old Tg mice that received OM1. The inner to outer circles indicate different taxonomic levels (inner to outer: phylum, class, order, family and genus). M, month
   (b-c) Correlation of between the microbiota having upregulated and downregulated microorganisms caused by OM1 and the frequency of brain immune cell subtypes in 5XFAD transgenic (Tg) mice at 7-month-old. Squares in red (positive correlation) or blue (negative correlation) with a yellow asterisk (^{∗}) has a P < 0.05 measured by the Pearson parametric correlation test, the numbers in each square are correlation coefficient.
   (d) Representative images of IBA1 staining, Aβ deposition and Tau phosphorylation in the brain hippocampus of WT, Tg and OM1-treated Tg. Scale bar represent 250 µm. Positive signal was visualized using the substrate 3,3' diaminobenzidine (DAB) shown as dark brown.
   (e) Effects of FMT from WT, Tg and OM1-treated Tg mice on the brain Th1 cell in the recipient C57 mice with Aβ hippocampus injection (n = 4-5). The data are presented as the mean ± standard error of the mean (mean ± sem).
   (f) Effects of antibiotic treatment (ampicillin (0.1 mg/mL), streptomycin (0.5 mg/mL), and colistin (0.1 mg/mL) on the relative abundance of gut microbiota on the genus level in 6-month-old APP/PS1 transgenic model mice treated orally with OM1 (n = 6-8). Colours indicate different genera.
   (g) Effects of OM1 on the brain Th1 cell frequency of antibiotic-treated 6-month-old APP/PS1 mice (see Methods). Th1 cells (CD45^{high}CD4⁺CXCR3⁺CCR6⁻) are presented relative to CD45^{high}CD4⁺T cells (n = 6-8), and the data are presented as the mean ± standard error of the mean (mean ± sem). From left to right: ^{∗}P<0.05, Student's t test. NS, no significance.
   (h) Effects of OM1 on the relative density of IBA1-positive immune-fluorescent staining detected in hippocampal slices from antibiotic-treated 6-month-old APP/PS1 mice (n = 4-6, see Methods). The IBA1-positive area reflects the activation of microglial cells. The data are presented as the mean ± standard error of the mean (mean ± sem). ^{∗∗∗}P < 0.0001 by Student's t-test. NS, no significance.
   (i) Representative immunofluorescence staining of IBA1 in the brain of APP/PS1 and OM1 treated APP/PS1 mice with or without antibiotics. IBA1 was visualized using FITC conjugated secondary antibody shown as green. Nucleus were stained with DAPI shown as blue. Scale bar represent 250 µm.
   (j) Effects of OM1 on cytokine levels in the brain homogenates of 7-month-old APP/PS1 mice as detected by a cytokine antibody array (n = 5-6). The colors in the heat map indicate the relative cytokine levels normalized by Row Z-Score; red indicates cytokines that are upregulated, and blue indicates cytokines that are downregulated.
Figure 11
   (a) The effect of OM1 on the differentiation of naive CD4⁺ T cells treated bacteria supernatant to Th1 and Th2 cells. Naive CD4 T cells were cultured and supernatant from microbiota of 5XFAD mice was added in the presence/absence of OM1 for 3 days. Th1 (CD4⁺IFN-γ⁺) cells and Th2 (CD4⁺IL-4⁺) cells were gated by flow cytometry. The data are presented as the mean ± standard error of the mean (mean ± sem); n = 3 replicates per group.
   (b-c) The volcano plot depicts the distribution of metabolites from the raw data of gut feces metabolomics of 7-month-old WT and 5XFAD (Tg) mice (n=6-8) (b) and 7-month-old Tg mice treated or not treated with OM1 (n=6) (c). Red points indicate significant changing metabolites. Significance is defined as P-value < 0.05 of Student's t-test and a fold change (FC) of < 0.83 or > 1.2 between the fold change (FC) of (T) and wild type (WT). The x-axis shows log₂FC, and the y-axis shows -log₁₀P
   (d-e) Heatmap of seven hundred eighty-six metabolites that were differentially regulated between Tg and WT mice (d), as well as 149 metabolites between OM1-treated and untreated Tg mice (e) (n=6-8). These metabolites were identified and annotated by aligning the molecular mass data (m/z) of the significant peaks with online METLIN database.
   (f) The Venn diagram shows the commonly deregulated gut feces metabolites between Tg and WT mice (T_W) and between OM1-treated and untreated Tg mice (Ttreat_T) (n=6-8). One hundred twenty-four metabolites had reversed patterns across the two comparisons, i.e., metabolites that are either both high in T_W and low in Ttreat_T, or both low in T_W and high in Ttreat_T.
   (g) The heatmap shows 31 identified metabolites that were differentially regulated among the WT, Tg and OM1-treated Tg groups (n=6-8) that could be matched to all three databases (Human Metabolites Database (HMDB), METLIN, Kyoto Encyclopedia of Genes and Genomes (KEGG)). Red, upregulated; blue, downregulated.
   (h) The receiver operating characteristic (ROC) curve and the area under the curve (AUC) value of all amino acids during disease progression are calculated using the random forest algorithm.
   (i) Effects of faecal microbiome transplantation (FMT) on blood phenylalanine and isoleucine levels. Feces of 2-month old WT mice were transplanted into 7-month old Tg mice (n = 6-7). For phenylalanine, ^{∗∗∗}P < 0.001((F (2, 17) = 26.59)). For isoleucine, ^{∗∗}P <0.01 (Tg versus WT), ^{∗∗}P <0.01 (Tg + FMT versus Tg) by one-way ANOVA (F (2, 17) = 8.181).
   (j) Levels of amino acid in the blood samples of WT, co-housed WT and Tg at various months (M) of ages. Red, upregulated; blue, downregulated.
   (k) The uptake of phenylalanine by the naive CD4 T cells. The naive CD4 T cells were cultured with/without ¹³C-labelled phenylalanine for 0.5 h. Mass Spectrometry of phenylalanine-related compounds were tested. ¹³C-Phenylalanine is administered at a concentration of 5 mmol/L, and is taken up by an L-type amino acid transporter.
Figure 12. JPH203 50 mg/kg effectively inhibits the proportion of Th1 cells in the brain.
Figure 13. The change trend of OTU levels of enterobacteria after administration of OM1 compared with the control - PCoA analysis of the effect of phenylalanine-degrading bacteria on the phenylalanine content in feces 1 week after transplantation.
Figure 14. The change trend of the relative abundance of enterobacteria after administration of OM1 compared with the control. The effect of phenylalanine degrading bacteria on the proportion of Th1 cells in the blood one week after transplantation.
Figure 15. The change trend of brain cytokine content compared with the control after administration of OM1.
Figure 16. Changes in the distribution of gut microbes before and after the application of agents used to regulate the relative abundance of gut microbes: OM1 or fecal bacteria (gut microbes complex).
Figure 17. The difference between the phylum level and the genus level of AD and HC, partly listed in detail. AD: AD patients; HC: Healthy control healthy controls.
Figure 18. Some of AD and HC significantly changed flora (genus and species level). AD: AD patients; HC: Healthy control healthy controls.
Figure 19. A list of amino acids related to AD. Multivariate ROC curve based exploratory analysis (Explorer) was used to analyze blood amino acids of wild-type mice and 5XFAD mice of different months of age, and look for potential amino acid combinations as markers to distinguish wild-type mice from 5XFAD mice. The following is a list of the top 15 amino acids sorted by selection frequency and all sorts.
Figure 20. 6.5-month-old 5XFAD mice, after receiving 100mpk OM1 for 1 month, have blood amino acids that tend to recover to that of wild mice.
Figure 21. 6.5-month-old 5XFAD mice after receiving 100mpk OM1 for 1 month, have the fecal amino acids that tend to recover to that of wild mice.
Figure 22. List of cytokines reversed after OM1 administration. 6.5-month-old 5XFAD mice received 100mpk OM1 treatment for 1 month, and had the brain cytokines that tended to recovers to that of wild mice.
Figure 23. The change trend of brain M1 cells in APP/PS1 mice with different months of age.

### DETAILED DESCRIPTION OF THE INVENTION

Certain exemplary embodiments will now be described to provide a comprehensive understanding of the principles of the structure, function, preparation, and application of the products, methods, and uses disclosed herein. One or more Examples of these implementations will be exemplified later. Those skilled in the art will understand that the products, methods, and uses specifically described herein and specifically illustrated in the enclosed drawings are non-limiting exemplary embodiments, and the scope of the present invention is limited only by the claims. Features illustrated or described together with one exemplary embodiment may be combined with features of other embodiments. Such modifications and changes are intended to be included in the scope of the present invention. All publications, patents and patent applications cited herein are incorporated herein by reference in their entirety.

In the context of describing the present invention (especially in the context of the following claims) unless otherwise stated herein or obviously contradicted by context, the terms "a/an" and "the" and the use of similar expressions shall be interpreted as covering both the singular and the plural. Unless otherwise stated, the terms "comprising", "having", "including" and "containing" shall be interpreted as open-ended terms (i.e. "including, but not limited to"), but they also include the partially closed or closed terms of "substantially consisting of" and "consisting of". Unless otherwise stated herein, the description of the numerical range herein is only intended to be used as a shorthand method for independently referring to each individual value falling within the range, and each individual value is incorporated into this specification as if it were independently recited herein. Unless otherwise stated herein or obviously contradicted by context, all methods described herein can be performed in any suitable order. Unless otherwise stated, the use of any and all examples or exemplary language (e.g. "such as") provided herein is only intended to better illustrate the present invention, and does not limit the scope of the present invention. No language in this specification should be construed as indicating that any unclaimed element is necessary for the practice of the present invention. All percentages are weight percentages, and all weight percentages are based on the total weight of the composition (without any optional concentration and/or dilution). The expression "one or more" includes "two or more" and "three or more" and the like.

The preferred embodiments of the present invention are described herein. After reading the foregoing description, changes to those preferred embodiments may become obvious to those of ordinary skill in the art. The inventor expects those skilled in the art to appropriately adopt such changes, and the inventor expects the present invention to be implemented in a manner different from that specifically described herein. Therefore, the present invention includes all modifications and equivalents of the subject matter described in the appended claims permitted by applicable laws. Moreover, unless otherwise stated herein or it is obviously contradicted by context, the present invention encompasses any combination of all possible variations of the above-mentioned elements.

Where a series of values recited in this application, it should be understood that any recited value can be the upper or lower limit of the numerical range. It should also be understood that the present invention encompasses all such numerical ranges, that is, a range having a combination of an upper limit and a lower limit, wherein the respective values of the upper limit and the lower limit can be any of the numerical values listed in the present invention. The range provided by the present invention should be understood to include all values within the range. For example, 1-10 should be understood to include all of the values 1, 2, 3, 4, 5, 6, 7, 8, 9, and 10, and include fractional values as appropriate. A range expressed as "up to" a certain value (for example, up to 5) should be understood as all values (including the upper limit of the range), such as 0, 1, 2, 3, 4, and 5, and include fractional values as appropriate. At most one week or within a week should be understood to include 0.5, 1, 2, 3, 4, 5, 6 or 7 days. Similarly, the range defined by "at least" should be understood to include the lower values provided and all higher values.

Unless otherwise stated, all percentages are weight/weight.

As used in the present invention, "about/approximately" should be understood to be included within three standard deviations of the average value or within the standard tolerance range in a specific field. In certain embodiments, about should be understood as a variation of no more than 0.5. "about/approximately" modifies all enumerated values thereafter. For example, "about 1, 2, 3" means "about 1", "about 2", "about 3".

Unless the context clearly stated otherwise, the term "or" is used inclusively in the present invention to refer to the term "and/or" and can be used interchangeably therewith.

The term "such as/for example/e.g." is used in the present invention to refer to the phrase "such as/for example/e.g. but not limited to" and can be used interchangeably therewith.

Those skilled in the art should understand that the technical features described in the various embodiments above can be used alone or in combination with the technical solutions of the various aspects of the present invention.

The inventors used the 5XFAD transgenic (Tg) mouse model widely used in AD research due to its severe and accelerated cognitive impairment. By analyzing the enterotype of Tg mice and WT mice in different stages of AD progression, it was found that the gut microbiota of Tg mice is highly dynamic (Figure Id). At the age of 2-3 months, *Bacteroidetes, Firmicutes* and *Verrucomicrobia* are the three most abundant bacterial phyla (46.8%, 32.3% and 12.6%, respectively), while at the age of 7-9 months, *Firmicutes* became the dominant bacteria, while the abundance of *Bacteroidetes* and *Verrucomicrobia* decreased significantly. This is in sharp contrast to the gut microbiota of WT mice.

The inventors also analyzed peripheral immune cells Th1 and Th1 and microglia M1 and M2, and found that the two main subtypes of CD4+ cells (infiltrating Th1 and Th2 cells) showed similar dynamics to the two main subtypes of microglia (M1 and M2 microglia) (Figure If And Figure 1j). In the early stage, it is mainly Th2 cells and neuroprotective M2 microglia, and in the late stage, it is mainly Th1 cells and pro-inflammatory M1 microglia. The inventors believe that as the gut microbiota pattern changes, the immune cell population tends to reach a status where dominated by Th1 and M1.

The inventors also analyzed the correlation between the abundance of gut microbiota and brain immune cells in Tg mice, and also noted that the bacterial composition in the early stage (2-3 months) is highly correlated with the counts of M2 and Th2 cells in the brain (Figure 1k, up), while in the late stage (7-9 months), changes in the bacterial pattern are highly correlated with M1 and Th1 cells (Figure 1k, bottom). Overall, these results indicate that during the progression of AD, intestinal bacteria are associated with peripheral immune cell infiltration and neuroinflammation.

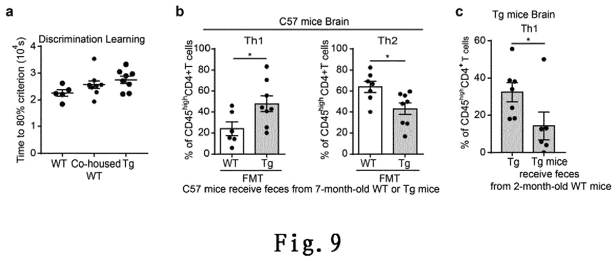

Furthermore, the inventors revealed that the gut microbiota dysbiosis is required for the infiltration of various peripheral immune cells (including CD4+ and CD8+ T cells, B cells, natural killer (NK) cells, neutrophils, dendritic cells (DC) and monocytes) into the brain. Among them, Th1 cells are particularly noteworthy because they are closely related to the activation of M1 microglia during the progression of AD. In view of the recognized functional crosstalk between Th1 and M1 microglia in the brain, the inventors propose that the intestinal dysbiosis promotes Th1 cell infiltration to allow local crosstalk with M1 microglia, thereby triggering the differentiation of microglia into a pro-inflammatory state. Through a series of discoveries obtained in this research, this mechanism insight has been strengthened. First, the dynamic changes of the composition of the gut microbiota during the progression of AD are significantly related to the increase of Th1 cell infiltration. Second, ablation of the gut microbiota by antibiotic treatment blocked Th1 cell infiltration and subsequent activation of M1 microglia in AD mice (Figure 2a-c). Third, long-term fecal bacterial exposure (cohabitation experiment) and fecal microbiota transplantation of fecal bacteria from AD mice both significantly enhanced Th1 cell infiltration (Figure 2e) and M1 microglia activation in WT mice, while the transplantation of WT mouse fecal microbiota into Tg mice reduced the Th1 cells of recipient Tg mice (Figure 9c). The inventor's research results as a whole highlight the gut microbiota as a driving factor to promote Th1/M1 microglia-led neuroinflammation in the progression of AD.

The causal relationship between the gut microbiota dysbiosis and neuroinflammation in AD is still unclear. In this study, the inventors detected that more than 100 metabolites were significantly changed in AD mice compared with WT mice. Among them, the most significant changes occurred in amino acids, especially those in the phenylalanine-related pathway. The inventors were able to confirm that the abundances of phenylalanine and isoleucine were increased in feces and blood of AD mice relative to WT mice. Both *in vitro* and *in vivo* functional evaluation revealed the role of phenylalanine and isoleucine in promoting the differentiation and proliferation of peripheral inflammatory Th1 cells. The use of antibiotics to ablate the gut microbiota resulted in a simultaneous decrease in blood phenylalanine and isoleucine, Th1 cell infiltration, and M1 cell activation. These findings emphasize the role of abnormal production of phenylalanine and isoleucine in the gut microbiota in stimulating neuroinflammation dominated by Th1 cells. Consistent with this point of view, the inventors have detected that the phenylalanine/isoleucine concentration and Th1 cell count in the blood of MCI patients caused by AD are higher than those of the healthy control group.

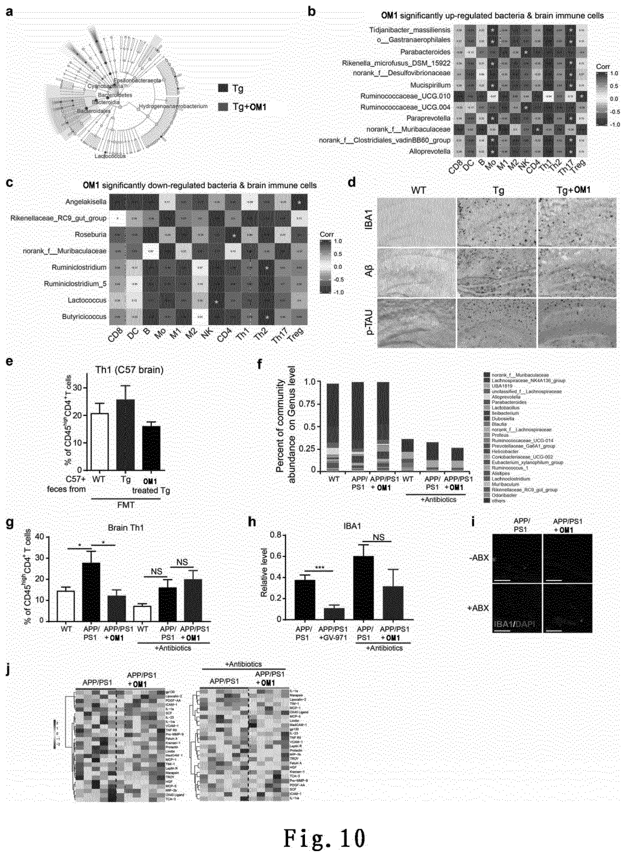

Newly emerging data show that polysaccharides or oligosaccharides have the advantage of regulating the gut microbiota. Mannouronic acid oligosaccharides are carbohydrate-based anti-AD drugs. In a phase III clinical trial recently completed in China, it has been proven to improve cognitive impairment in patients with mild to moderate AD. Mannouronic acid oligosaccharides are well tolerated and have safety similar to placebo controls. In this study, the inventors found that OM1 effectively reconditions the gut microbiota (Figure 4a-b; Figure 10a), reduces the amount of phenylalanine and isoleucine in feces and blood (Figure 5c-d), and reduces Th1-related neuroinflammation (Figure 4g-i). It is worth noting that Tg feces treated with OM1 can largely mimic the therapeutic effect of OM1 treatment itself, and antibiotic treatment eliminates its therapeutic effect. These findings provide important evidence that the therapeutic effect of OM1 is mainly through the restoration of the gut microbiome. Therefore, OM1 can provide an attractive approach to AD treatment strategies centered on the microbiota, which is worthy of further study.

All these findings allow the inventors to propose conceptual advances in understanding the pathogenesis of AD. AD is not just a local Aβ-driven brain disease, but its development also requires systemic interactions between the intestine, brain, and intermediate inflammatory factors (Figure 6). In the case of Aβ deposition, the altered gut microbiota composition during AD progression causes an abnormal increase in amino acids (especially phenylalanine and isoleucine). These amino acids promote the infiltration of peripheral Th1 cells into the brain through blood circulation. Infiltrating peripheral Th1 cells can locally cross-talk with M1 microglia in the brain, leading to pathological neuroinflammation and cognitive impairment. These insights into the pathogenesis of AD can be used to recondition the gut microbiota to facilitate the anti-neuro-inflammatory response and provide new therapeutic solutions.

The inventor's research results can have transformative significance for the diagnosis and treatment of AD. The composition of specific bacteria (for example, Th1/M1 related bacteria), amino acids (for example, phenylalanine and isoleucine) and brain infiltrating immune cells (for example, Th1 cells dominate) or a combination of one or more of them can be used as early diagnostic biomarkers for MCI patients caused by AD, and it is worthy of further verification in a large AD patient cohort.

More importantly, the established anti-AD effect of OM1 centered on the microbiota will open up new therapeutic approaches for AD treatment by reshaping the gut microbiota, and guide the development of effective therapies in the future by exploring the highly undiscovered sugar chemistry.

The numerous characteristic gut microbes, amino acids, immune cells and cytokines identified by the inventors related to the brain-gut axis each constitute the gut microbial profile, amino acid profile, immune cell profile and cytokine profile. One or more of these profiles (such as the gut microbial profile) show differences between normal and diseased individuals. The present invention aims to detect or regulate the state of an individual by detecting or regulating one or more of these profiles (such as the gut microbial profile). In some embodiments, for diagnostic purposes, the profile (e.g. gut microbial profile) of the individual can be detected to compare with the profile (e.g. gut microbial profile) with normal characteristics of the corresponding normal individual and/or the profile (e.g. gut microbial profile) with disease characteristics of the corresponding diseased individual, so as to determine whether the individual is in a normal state or in a diseased state, thereby diagnosing the individual. In some embodiments, for therapeutic purposes, the profile of an individual with disease characteristics can be regulated to that of a corresponding normal individual, so that the individual's disease state can be regulated to a normal state, thereby treating the individual.

Therefore, in one aspect, the present invention provides the use of an agent for regulating the relative abundance of gut microbes in the manufacure of a medicament for treating Alzheimer's disease in a subject.

In another aspect, the present invention provides a pharmaceutical composition for treating Alzheimer's disease in a subject, the pharmaceutical composition comprises an effective amount of an agent for regulating the relative abundance of gut microbes.

In some embodiments, the gut microbes are selected from one or more of *Firmicutes, Bacteroidetes, Proteobacteria, Actinomycetes, Fusobacteria, Cyanobacteria, Verrucomicrobia,* or a combination thereof.

In some embodiments, the agent is selected from carbohydrate drugs, gut microbes complexes, or a combination thereof; wherein the carbohydrate drug is selected from monosaccharides, disaccharides, oligosaccharides, polysaccharides, or derivatives thereof, or a combination of them and/or derivatives thereof; preferably oligosaccharides and polysaccharides; more preferably mannuronic acid oligosaccharides or a composition comprising mannuronic acid oligosaccharides; wherein the gut microbes complex comprises one or more selected from *Firmicutes, Bacteroidetes, Proteobacteria, Actinomycetes, Fusobacteria, Cyanobacteria, Verrucomicrobia,* or a combination thereof.

In some embodiments, the agent regulates the relative abundance of gut microbes of the subject by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; and/or make the relative abundance of gut microbes of the subject close to or reach the relative abundance of the corresponding gut microbes of the corresponding normal subject.

In some embodiments, the regulating the relative abundance of gut microbes is to increase the relative abundance of one or more gut microbes and/or reduce the relative abundance of one or more gut microbes.

In still another aspect, the present invention provides a method for screening drug candidates that can be used to treat Alzheimer's disease, the method comprises:
a) administering a test agent to *in vivo* or *in vitro* models with gut microbes, and
b) selecting the test agent that regulates the relative abundance of gut microbes as the drug candidate that can be used to treat Alzheimer's disease.

In some embodiments, the method also comprises administering OM1 as a positive control to an *in vivo* or *in vitro* model with gut microbes, preferably selecting a test agent that regulates the relative abundance of gut microbes substantially consistently with the OM1.

In some embodiments, the method further comprises administering a selected test agent to an *in vivo* or *in vitro* model with gut microbes for verification, wherein the selected test agent regulates the relative abundance of gut microbes by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; and/or make the relative abundance of gut microbes close to or reach the relative abundance of the corresponding gut microbes in the corresponding normal *in vivo* or *in vitro* model.

In some embodiments, the gut microbes are selected from one or more of *Firmicutes, Bacteroidetes, Proteobacteria, Actinomycetes, Fusobacteria, Cyanobacteria, Verrucomicrobia,* or a combination thereof.

In still another aspect, the present invention provides a method for establishing an animal model of Alzheimer's disease, the method comprises administering an agent for regulating the relative abundance of gut microbes to an animal, so that the relative abundance of gut microbes of the animal is regulated by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; or close to or reach the relative abundance of the corresponding gut microbes of the corresponding animal model having Alzheimer's disease.

In some embodiments, the gut microbes are selected from one or more of *Firmicutes, Bacteroidetes, Proteobacteria, Actinomycetes, Fusobacteria, Cyanobacteria, Verrucomicrobia,* or a combination thereof.

In some embodiments, the method further comprises administering the aggregated Aβ protein to the animal, preferably via hippocampal injection.

In still another aspect, the present invention provides a method for treating patients with Alzheimer's disease, the method comprises:
(a) detecting the relative abundance of gut microbes of the patient and comparing it with the relative abundance of the corresponding gut microbes of the corresponding normal population to select gut microorganism of which the relative abundance is different from that of the corresponding gut microorganism of the corresponding normal population
(b) administering an agent for regulating the relative abundance of gut microbes to the patient to regulate the relative abundance of the selected gut microorganism to make them close to or reach the relative abundance of the corresponding gut microorganism of the corresponding normal population.

In some embodiments, the carbohydrate drug is OM1.

In some embodiments, the carbohydrate drug is not OM1.

In some embodiments, the mannuronic acid oligosaccharide is OM1.

In some embodiments, the mannuronic acid oligosaccharide is not OM1.

The structure and preparation method of mannuronic acid oligosaccharides have been described in many prior art documents. The prior art patent CN2016100697039 discloses a preparation method of oligomannuronic acid, the prior art patent CN2017107964853 discloses a method for determining the weight average molecular weight and amount of mannuronic acids, the prior art patent CN2017114675966 discloses a composition of mannuronic acid and its preparation, all of them are incorporated herein by reference in their entirety. OM1 used herein is the composition A according to CN2018107213276 ("alginic oligosaccharic acid composition"), which is incorporated herein by reference.

### Microbiota

Disclosed herein are methods and compositions that include altering the microbiota in the intestinal tract of a subject. The term "microbiota" is used to refer to one or more bacterial communities that can be found in or exist (colonize) in the intestinal tract of an organism. It can be used interchangeably with "microbes/microorganism" or "gut microbes/microorganism" herein. When referring to more than one microbiota, the microbiota can be of the same type (strain) or can be a mixture of groups, such as *Bacteroidetes, Proteobacteria,* and/or *Firmicutes,* or its sub-groups (class, order, family, genus, species). The microbiota can be a mixture of microorganisms at the same level, such as *Bacteroidetes, Proteobacteria* and/or *Firmicutes;* it can also be a mixture of different levels of microorganisms, such as *Bacteroidetes* and *Proteobacteria.*

In some embodiments, the gut microbes are selected from one or more selected from the phylum, class, order, family, genus, or species in Table 1, or a combination thereof.

In some embodiments, the gut microbes are selected from one or more selected from the phylum, class, order, family, genus, or species in Table 2, or a combination thereof.

In some embodiments, the gut microbes are selected from one or more selected from *Firmicutes, Bacteroidetes, Proteobacteria, Actinobacteria, Fusobacteria, Cyanobacteria, Verrucomicrobia,* or a combination thereof.

In some embodiments, the gut microbes are one or more selected from the phylum in the following table or a combination thereof:

| | |
|---|---|
| phylum | *Firmicutes* |
| phylum | *Bacteroidetes* |
| phylum | *Proteobacteria* |
| phylum | Actinobacteria |
| phylum | *Fusobacteria* |
| phylum | *Cyanobacteria* |
| phylum | *Verrucomicrobia* |

In some embodiments, the gut microbes are one or more selected from the classes or a combination thereof in the following table:

| | |
|---|---|
| class | B acteroidia |
| class | Clostridia |
| class | *GammaProteobacteria* |
| class | Bacilli |
| class | Negativicutes |
| class | Actinobacteria |
| class | Fusobacteriia |
| class | *BetaProteobacteria* |
| class | *AlphaProteobacteria* |

In some embodiments, the gut microbes are one or more selected from the orders in the following table or a combination thereof:

| | |
|---|---|
| order | B acteroidales |
| order | Clostridiales |
| order | Enterobacteriales |
| order | Selenomonadales |
| order | Lacto bacillales |
| order | Bifidobacteriales |
| order | *Fusobacteriales* |
| order | B urkholderiales |
| order | Bacillales |

In some embodiments, the gut microorganism is one or more selected from the families or a combination thereof in the following table:

| | |
|---|---|
| family | Rikenellaceae |
| family | Ktedonobacteraceae |
| family | Nannocystaceae |

In some embodiments, the gut microbes are one or more selected from the genera or a combination thereof in the following table:

| | |
|---|---|
| genus | Lachnospiraceae_NK4A136_group |
| genus | Alistipes |
| genus | Ruminococcus_1 |
| genus | Ruminococcaceae_UCG-002 |
| genus | Ruminococcaceae_UCG-005 |
| genus | Coprococcus_2 |
| genus | Tyzzerella_4 |
| genus | Lachnospiraceae_UCG-001 |
| genus | Anaerotruncus |
| genus | Cloacibacterium |
| genus | norank_f__Ktedonobacteraceae |
| genus | Nannocystis |
| genus | norank_f__Hydrogenophilaceae |

In some embodiments, the gut microbes are one or more selected from the following species or a combination thereof:

| | |
|---|---|
| species | unclassified_g__Subdoligranulum |
| species | unclassified_g__Alistipes |
| species | uncultured_organism_g__Parasutterella |
| species | unclassified_g__Tyzzerella_4 |
| species | uncultured_organism_g__Ruminococcaceae_UCG-005 |
| species | uncultured_organism_g__Anaerotruncus |
| species | uncultured_Alistipes_sp._g__Alistipes |
| species | Lachnospiraceae_bacterium_TF01-11 |
| species | unclassified_g__Anaerotruncus |
| species | unclassified_g__norank_o__Mollicutes_RF9 |
| species | uncultured_bacterium_g__Family_XIII_AD3011_group |
| species | uncultured_bacterium_g__norank_f__Christensenellaceae |
| species | uncultured_bacterium_g__Cloacibacterium |
| species | uncultured_organism_g__Peptococcus |
| species | Mycobacterium_celatum_g__Mycobacterium |
| species | unclassified_g__Oceanobacillus |
| species | Alistipes_putredinis_DSM_17216 |
| species | Prevotella_loescheii |
| species | uncultured_bacterium_g__norank_o__MBA03 |
| species | Eubacterium_brachy |
| species | uncultured_bacterium_adhufec 108 |
| species | uncultured_Clostridiales_bacterium_g__norank_f__Ktedon obacteraceae |
| species | Nannocystis_pusilla |
| species | bacterium_2013Ark19i |
| species | Auxenochlorella_protothecoides_g__norank |
| species | *uncultured_Bacteroidetes_bacterium_g__Dinghuibacter* |

The inventors discovered that a variety of gut microbes are involved in the brain-gut axis according to the present invention. As shown in the Examples, between WT mice and TG mice, the levels of some gut microbes changed, and after administration of, for example, OM1, the levels of these gut microbes recovered towards the WT mice. Such gut microbes constitute the profile of gut microbes. As mentioned earlier, such a profile can be used for diagnostic and/or therapeutic purposes.

In some embodiments, the change (e.g. increasing or decreasing) in the level of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66 or 67) gut microbes selected from the phylum, class, order, family, genus, and species from the above table in the subject relative to the level of the corresponding gut microbes of the corresponding normal subject indicates that the subject is at risk of having AD or has AD. In one embodiment, the change (e.g. increasing or decreasing) in the level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% of the gut microbes in the intestinal microbial profile consisting of the gut microbes of the phylum, class, order, family, genus, and species in the above table in the subject relative to the level of the corresponding gut microbes of the corresponding normal subject indicates that the subject is at risk of having AD or has AD.

In some embodiments, the change (e.g. increasing or decreasing) in the level of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66 or 67) gut microbes selected from the phylum, class, order, family, genus, and species from the above table in the subject with AD towards the level of the corresponding gut microbes in the corresponding normal subject relative to the level of the corresponding gut microbes of the corresponding normal subject indicates that the subject receives appropriate treatment. In one embodiment, the change (e.g. increasing or decreasing) in the level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% of the gut microbes in the intestinal microbial profile consisting of the gut microbes of the phylum, class, order, family, genus, and species in the above table in the subject with AD towards the level of the corresponding gut microbes in the corresponding normal subject relative to the level of the corresponding gut microbes of the corresponding normal subject indicates that the subject receives appropriate treatment.

The relative abundance of gut microbes can be changed by using the composition or method of the present invention in the following ways: administrating a composition comprising the relevant microbiota, or administrating a composition comprising one or more compounds that significantly increase and/or decrease the relative abundance of relevant gut microbes.

The *Bacteroidetes* comprises three major classes of bacteria: *Bacteroidia, Flavobacteria* and *Sphingobacteria.* They are distributed in the environment, including soil, sediment, sea water, and animal intestines and skin.

*Proteobacteria* is the largest bacterial phylum. These organisms display extremely high metabolic diversity and represent the medical, industrial, and agricultural importance of most known bacteria. This is evolutionarily, geologically and environmentally important. All *Proteobacteria* bacteria are Gram-negative and their outer wall is composed of lipopolysaccharide. Many have gas vesicles, flagella, or can move by sliding; they can have stalks, other appendages, or have the ability to form multicellular fruit bodies. Most are facultative or obligate anaerobic, autotrophic and heterotrophic, but there are exceptions. Some species are capable of photosynthesis, others store sulfur inside or outside the cell.

*Firmicutes* is a phylum of mainly Gram-positive bacteria. However, a few have porous pseudo-outer membranes that cause them to be Gram-negative. Scientists once classified *Firmicutes* as including all Gram-positive bacteria, but have recently defined them as a core group with a related form called low-G+C. They are mainly round cells, called cocci (singular cocci), or rod-shaped (bacilli). Many *Firmicutes* produce endospores that are resistant to desiccation and can survive extreme conditions, allowing them to survive in various environments.

The method of altering the microbiota may also include measuring the relative abundance of one or more gut microbes in a sample from the subject. In next-generation sequencing, many sequences are measured for each sample. After preprocessing, sequence clustering algorithms are used to put together sequences with a similarity of more than 97% to form an OTU. Then OTU_table can be obtained, this is a matrix that gives how many reads each OTU contains in each sample, i.e., each sample corresponds to the number of sequence reads in each OTU. The relative abundance is 100% for each sample (each row), and the percentage of the number of reads in each OTU to the number of all reads in a sample is calculated. Therefore, the relative abundance refers to the relative percentage of the sequence number of each microorganism in the sample. The relative abundance can be detected by the method described in the present invention or a method known in the art that can be used to detect it, such as the detection of 16S rRNA gene.

The relative abundance of gut microbes can be measured by obtaining samples from subjects. The sample can be saliva, feces and stomach, intestine and/or rectal contents; tissue samples from digestive tract tissues (such as oral tissue, esophagus, stomach, small intestine, ileum, cecum, colon and/or rectum); ascites in gastrointestinal tissues; and any other samples that may be used by persons familiar with microbiota assays.

The relative abundance of one or more gut microbes can be compared with the normal relative abundance of the corresponding gut microbes. The normal relative abundance can be from one or more subjects of similar age, gender, race, and the like. The normal relative abundance may be from healthy subjects of similar age, gender, race, and the like, who responded to or showed beneficial results of the treatment or therapeutic intervention. In a specific embodiment, the normal relative abundance is the relative abundance of gut microbes in healthy subjects.

The methods and compositions of the invention may involve altering the relative abundance of one or more gut microbes. Exemplary embodiments may involve methods or compositions for changing the relative abundance of gut microbes by administering gut microbes to a subject. Depending on the desired outcome (e.g., decreased metabolites, decreased lymphocyte infiltration into the brain, decreased activation of microglia, decreased neuroinflammation, improved cognition, relief of Alzheimer's disease symptoms) and individual subjects, the relative abundance of gut microbes in the subject can be regulated 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 to 100% or more, or the range consistuted by the endpoints of the aforementioned value or any value therein, such as about 7% to about 28%, and the like. or about 7%, 14%, 21%, 28%, and the like.; and/or make the relative abundance of gut microbes of the subject close to or reach the relative abundance of the corresponding gut microbes of the corresponding normal subject.

As described throughout this invention, the present invention aims to regulate the indicator that is different from the corresponding indicator of the corresponding normal subject towards the corresponding indicator of the corresponding normal subject, so that the regulated indicator is close to or reaches the corresponding indicator of the corresponding normal subject. This regulation is applicable to the various indicators to be regulated or regulated as described herein. Obviously, it is ideal to reach the corresponding indicator of the corresponding normal subject, but it is also desirable to be close to the corresponding indicator of the corresponding normal subject. Therefore, the present invention aims to make the one or more indicatores of the individual whose one or more indicatores are to be regulated to be close to or reach the corresponding indicatores of the corresponding normal subjects. As used herein, the term "close to or reach" means that the difference between the indicator before regulation and the corresponding indicator of the corresponding normal subject is reduced by greater than or equal to about 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 100% or the range consistuted by the endpoints of the aforementioned value or any value therein, such as about 7% to about 28%, and the like. or about 7%, 14%, 21%, 28%, and the like. relative to the difference after regulation. When the difference between the indicator before regulation and the corresponding indicator of the corresponding normal subject is reduced by about 100% relative to the difference after regulation, the regulated indicator reaches the corresponding indicator of the corresponding normal subject. Those skilled in the art understand that the specific difference value would depend on, for example, the regulation object, indicator type, measurement method, and the like.

An agent capable of making one or more indicators of an individual whose one or more indicators are to be regulated to be closed or reach the corresponding indicators of a corresponding normal subject is therapeutically desirable. The selection of such agents can be, for example, based on comparison with agents (such as OM1) serving as a positive control. Preferably, a test agent that regulates the relevant indicator substantially consistent with the agent used as a positive control (for example, OM1) is selected.

On the other hand, one or more indicators of the individual whose one or more indicators need to be regulated are substantially consistent with the reference indicator (for example, a reference indicator for diagnosing Alzheimer's disease or for identifying carbohydrate drug-sensitive patients in Alzheimer's disease patients), which can indicate that the individual is at risk of having Alzheimer's disease or has Alzheimer's disease.

As used herein, the term "substantially the same" refers to the standardization of the test indicator to the reference indicator (that is, the reference indicator is taken as 100%), the difference between the test indicator and the reference indicator is less than or equal to about 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100%, or the range constituted by endpoints of any aforementioned values or any value therein, for example, about 7% to about 28%, and the like, or about 7%, 14%, 21%, 28%, and the like. Those skilled in the art understand that the specific difference value will depend on, for example, the object to be regulated, the type of the indicator, the measurement method, and the like.

In some embodiments, the agent for regulating the relative abundance of gut microbes comprises gut microbes complex. In some embodiments, the gut microbes complex comprises microorganisms derived from fecal material or bacterial library. In some embodiments, the fecal material is derived from a normal subject or a patient with Alzheimer's disease.

Definition of the bacterial library: In order to obtain the species classification information corresponding to each OTU, the RDP classifier Bayesian algorithm is used to perform taxonomic analysis on the 97% similar level of OTU representative sequences, and the community composition of each sample is counted at each classification level (domain, kingdom, phylum, class, order, family, genus, species). The species taxonomy database comparison database used for the 16S analysis of bacterial flora is silva132/16S:silva132/16S (http://www.arb-silva.de).

### Metabolites involved

The inventor found that some metabolites of gut microbes, such as amino acids, are involved in the AD brain-gut axis studied in the present invention. These amino acids can be, for example, one or more selected from the following table; preferably one or more selected from phenylalanine, isoleucine, serotonin, histidine and acetylornithine; more preferably phenylalanine and/or isoleucine; most preferably phenylalanine.

| | |
|---|---|
| | 4-OH Proline |
| | Acetylornithine |
| | Alanine |
| | Alpha-Aminoadipic Acid |
| | Asparagine |
| | Aspartic Acid |
| | Asymmetric |
| Dimethylarginine | |
| | Beta-Alanine |
| | Carnosine |
| | Citrulline |
| | Creatinine |
| | Gaba |
| | Glutamic Acid |
| | Glutamine |
| | Glycine |
| | Histidine |
| | Hypotaurine |
| | Isoleucine |
| | Kynurenine |
| | Leucine |
| | Lysine |
| | Methionine |
| | Methionine Sulfoxide |
| | Ornithine |
| | Phenylalanine |
| | Pipecolic Acid |
| | Proline |
| | Putrescine |
| | Pyroglutamic Acid |
| | Serine |
| | Serotonin |
| | Taurine |
| | Threonine |
| | Tryptophan |
| | Tyrosine |
| | Valine |

The inventors discovered that a variety of amino acids are involved in the brain-gut axis according to the present invention. As shown in the examples, between WT mice and TG mice, the levels of some amino acids changed, and after administration of, for example, OM1, the levels of these amino acids recovered to the direction of WT mice. Such amino acids constitute the amino acid profile. As mentioned earlier, such a profile can be used for diagnostic and/or therapeutic purposes. In some embodiments, the change (e.g. increasing or decreasing) in the level of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36) amino acids selected from the above table in the subject relative to the level of the corresponding amino acid in the corresponding normal subject indicates that the subject is at risk of having AD or has AD. In one embodiment, the change (e.g. increasing or decreasing) in the level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% of the amino acids in the amino acid profile composed of the amino acids in the above table in the subject relative to the level of the corresponding amino acid in the corresponding normal subject indicates that the subject Are at risk of having AD or have AD.

In some embodiments, the change (e.g. increasing or decreasing) in the level of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36) amino acids selected from the above table in a subject having AD relative to the level of the corresponding amino acid of the corresponding normal subject toward the level of the corresponding amino acid of the corresponding normal subject indicates that the subject receives appropriate treatment. In one embodiment, the change (e.g. increasing or decreasing) in the level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% of the amino acid in the amino acid profile composed of the amino acids in the above table of the subject having AD relative to the level of the corresponding amino acid of the corresponding normal subject toward the level of the corresponding amino acid of the corresponding normal subject indicates the subject receives appropriate treatment.

The inventors found that the level of one or more amino acids in the above table in the subject is not consistent with the corresponding amino acid level of the corresponding normal subject, which can be attributed to the disease state of the subject. When the amino acid level of the subject is lower than the corresponding amino acid level of the corresponding normal subject, the amino acid level is intended to be upregulated. When the amino acid level of the subject is higher than the corresponding amino acid level of the corresponding normal subject, the amino acid level is intended to be downregulated.

In some embodiments, one or more amino acids in the above table are lower than the corresponding amino acid level of the corresponding normal subject. In some embodiments, one or more amino acids in the above table are higher than the corresponding amino acid level of the corresponding normal subject. In some embodiments, one or more amino acids in the above table are lower than the corresponding amino acid level of the corresponding normal subject, while the other one or more amino acids are higher than the corresponding amino acid level of the corresponding normal subject. The upregulation or downregulation may depend on, for example, the object to be regulated, the type of the indicator, the measurement method, and the like.

In some embodiments, the glutamine level is lower than the corresponding amino acid level of the corresponding normal subject. In some embodiments, the methionine level is lower than the corresponding amino acid level of the corresponding normal subject. In some embodiments, the levels of both glutamine and methionine are lower than the corresponding amino acid levels of the corresponding normal subject.

In some embodiments, the glutamine level is higher than the corresponding amino acid level of the corresponding normal subject. In some embodiments, the methionine level is higher than the corresponding amino acid level of the corresponding normal subject. In some embodiments, the levels of both glutamine and methionine are higher than the corresponding amino acid levels of the corresponding normal subject.

In some embodiments, the level of one, two, three, four or five of phenylalanine, isoleucine, serotonin, histidine, and acetylornithine is higher than the corresponding amino acid level of the corresponding normal subject. In some embodiments, the level of phenylalanine and/or isoleucine is higher than the corresponding amino acid level of the corresponding normal subject. In some embodiments, the level of phenylalanine is higher than the corresponding amino acid level of the corresponding normal subject.

In some embodiments, the levels of both glutamine and methionine are lower than the corresponding amino acid levels of the corresponding normal subject, whereas the level of one or more of 4-OH proline, acetylornithine, alanine, alpha-aminoadipate, asparagine, aspartic acid, asymmetric dimethylarginine, beta-alanine, carnosine, citrulline, creatinine, Gaba, glutamic acid, glycine, histidine, hypotaurine, isoleucine, kynurenine, leucine, lysine, methionine sulfoxide, ornithine, phenylalanine, pipecolic acid, proline, putrescine, pyroglutamic acid, serine, serotonin, taurine, threonine, tryptophan, tyrosine and valine is higher than the corresponding amino acid level of the corresponding normal subject.

In some embodiments, the level of one or more of 4-OH proline, acetylornithine, alanine, alpha-aminoadipate, asparagine, aspartic acid, asymmetric dimethylarginine, beta-alanine, carnosine, citrulline, creatinine, Gaba, glutamic acid, glutamine, glycine, histidine, hypotaurine, isoleucine, kynurenine, leucine, lysine, methionine, methionine sulfoxide, ornithine, phenylalanine, pipecolic acid, proline, putrescine, pyroglutamic acid, serine, serotonin, taurine, threonine, tryptophan, tyrosine and valine is higher than the corresponding amino acid level of the corresponding normal subject.

In some embodiments, one or more of the amino acids in the above table are upregulated. In some embodiments, one or more of the amino acids in the above table are downregulated. In some embodiments, one or more amino acids in the above table are upregulated while the other one or more amino acids are downregulated. In some cases, the upregulation or downregulation may depend on, for example, the object to be regulated, the type of the indicator, the measurement method, and the like.

In some embodiments, glutamine is upregulated. In some embodiments, methionine is upregulated. In some embodiments, both glutamine and methionine are upregulated.

In some embodiments, glutamine is downregulated. In some embodiments, methionine is downregulated. In some embodiments, both glutamine and methionine are downregulated.

In some embodiments, one, two, three, four, or five of phenylalanine, isoleucine, serotonin, histidine, and acetylornithine are downregulated. In some embodiments, phenylalanine and/or isoleucine are downregulated. In some embodiments, phenylalanine is downregulated.

In some embodiments, both glutamine and methionine are upregulated, whereas one or more of 4-OH proline, acetylornithine, alanine, alpha-aminoadipate, asparagine, aspartic acid, asymmetric dimethylarginine, beta-alanine, carnosine, citrulline, creatinine, Gaba, glutamic acid, glycine, histidine, hypotaurine, isoleucine, kynurenine, leucine, lysine, methionine sulfoxide, ornithine, phenylalanine, pipecolic acid, proline, putrescine, pyroglutamic acid, serine, serotonin, taurine, threonine, tryptophan, tyrosine and valine is downregulated.

In some embodiments, one or more of 4-OH proline, acetylornithine, alanine, alpha-aminoadipate, asparagine, aspartic acid, asymmetric dimethylarginine, beta-alanine, carnosine, citrulline, creatinine, Gaba, glutamic acid, glutamine, glycine, histidine, hypotaurine, isoleucine, kynurenine, leucine, lysine, methionine, methionine sulfoxide, ornithine, phenylalanine, pipecolic acid, proline, putrescine, pyroglutamic acid, serine, serotonin, taurine, threonine, tryptophan, tyrosine and valine is downregulated. The inventor found that immune cells such as naive T cells or undifferentiated T cells take up amino acids (as exemplified phenylalanine and isoleucine) in some cases, and for example differentiate into specific types of T cells, such as Th1 cells, by certain transporters, such as SLC7A5 as exemplified. The inventors found that preventing differentiation into Th1 cells, which leads to a Th1 dominance state, through various means can treat Th1 dominance related diseases. Such measures include, but are not limited to, reducing the level of related amino acids, preventing immune cells from taking up related amino acids, and the like.

SLC7A5 is called L-type amino acid transporter 1 (LAT1) and belongs to the APC superfamily. It forms a heterodimeric amino acid transporter that interacts with the glycoprotein CD98 (SLC3A2) through conservative disulfide bonds. CD98 (4F2hc, SLC3A2) is a type II glycoprotein, which acts as a chaperone protein of LAT1, stabilizing and promoting its translocation to the plasma membrane. This complex is responsible for the uptake of essential amino acids in key body areas such as the placenta and blood-brain barrier. The substrate includes a series of large neutral amino acids such as tyrosine, leucine, isoleucine, valine and phenylalanine, as well as drugs including L-DOPA and gabapentin.

Various agents (such as enzymes) can be used to degrade amino acids to reduce the level of related amino acids, thereby reducing the differentiation of naive T cells into Th1 cells. The enzymes shown in the table below can be used to degrade related amino acids, such as phenylalanine and isoleucine. The enzymes shown can be delivered to relevant parts of the subject by various means, for example the intestine or peripheral circulation (such as peripheral blood), to degrade amino acids. For example, the enzyme can be delivered to the relevant site by delivering a microorganism (for example, Escherichia coli) expressing the enzyme to the relevant site to express the enzyme at the relevant site. The microorganism can express one or more of the enzymes shown. Those skilled in the art understand that any microorganism that can be delivered to the relevant site through various delivery routes (for example, oral) without losing the ability to express the enzyme at the relevant site can be used to deliver the enzyme. For example, Escherichia coli engineered to express an enzyme for degrading phenylalanine was used as a phenylalanine-degrading bacterium, which was administered orally to mice to reduce the phenylalanine content in mice (as shown by detecting the content of phenylalanine in feces) and reduced the proportion of Th1 cells (as shown by detecting the proportion of Th1 cells in peripheral blood).

**Enzymes that can be used to degrade phenylalanine**

| | Converted Products | Metabolic Enzymes |
|---|---|---|
| 1 | Tyrosine | Phenylalanine-4-hydroxylase, PAH , Preferred |
| 2 | 2-Phenyl-acetamide | Phenylalanine 2-monooxygenase, PAO |
| | | Catalase-peroxidase, katG |
| 3 | Phenyl-acetaldehyde | Phenylacetaldehyde synthase, PAAS |
| 4 | Phenyl-ethyl amine | Aromatic-L-amino-acid/L-tryptophan decarboxylase, DDC, Preferred |
| | | Phenylalanine decarboxylase, AADC |
| 5 | N-Acetyl-L-phenylalanine | Phenylalanine N-acetyltransferase |
| 6 | Phenylpyruvate | Aspartate aminotransferase, AST, Preferred |
| | | Tyrosine aminotransferase, TAT, Preferred |
| | | L-Amino-acid oxidase, IL4I1, Preferred |
| | | Phenylalanine dehydrogenase |
| | | Aromatic-amino-acid transaminase |
| | | Histidinol-phosphate aminotransferase |
| | | Aromatic amino acid aminotransferase II, AR09 |
| 7 | D-Phenylalanine | Phenylalanine racemase |
| 8 | Trans-Cinnamate | Phenylalanine ammonia-lyase, PAL |
| | | Phenylalanine/tyrosine ammonia-lyase, PTAL |

**Enzymes that can be used to degrade isoleucine**

| | Converted Products | Metabolic Enzymes |
|---|---|---|
| 1 | (S)-3-Methyl-2-oxopentan oate | Branched-chain amino acid aminotransferase |
| | | L-Amino-acid oxidase, IL4I1 |

Transporter inhibitors can be used to prevent immune cells from taking up the relevant amino acids by inhibiting the naive T cells from taking up the relevant amino acid by the inhibiting the transporter (for example, the common transporter SLC7A5 for phenylalanine and isoleucine) to, thereby reducing the differentiation of the naive T cells into Th1 cells. Inhibitors that can be used to inhibit the transporter SLC7A5 include, but are not limited to, JPH 203, BCH, and KMH-233 known in the art. For example, as shown in Example 4, administration of the SLC7A5 inhibitor JPH 203 to mice significantly reduces the proportion of Th1 cells in the mouse brain.

JPH203 is a chemically synthesized low molecular weight compound available from J-Pharma of Japan (http://www.j-pharma.com/b3_e.html), which selectively inhibits LAT1, with CAS number 1037592-40-7 (https: //www.medkoo.com/products/9544).

BCH is a known LAT1 inhibitor with CAS number 20448-79-7 (https://www.tocris.com/cn/products/bch_5027).

KMH-233 is a known LAT1 inhibitor with CAS number 1941174-13-5 (https://www.medkoo.com/products/9545).

Those skilled in the art understand that the means that can be used to prevent the differentiation of naive T cells into Th1 cells are not limited to this. Any means that can be used to prevent naive T cells from being affected by gut microbes and/or their metabolites to differentiate into Th1 cells can be used to reduce the proportion of Th1, and alleviate or reverse the Th1-dominant state as described herein.

The inventors discovered that a variety of cytokines are involved in the brain-gut axis according to the present invention. As shown in the examples, between WT mice and TG mice, the levels of some cytokines changed, and after administration of, for example, OM1, the levels of these cytokines recovered towards the WT mice. Such cytokines constitute a profile of cytokines. As mentioned earlier, such a profile can be used for diagnostic and/or therapeutic purposes.

In some embodiments, the change (e.g. increasing or decreasing) in the level of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31) cytokines selected from the followings: OPG, Resistin, TARC, VEGF, Chemerin, IL-9, MMP-2, VEGF-D, TCA-3, gp130, MMP-10, 6Ckine, VEGF-B, IL-22, IL-la, IFNg R1, Granzyme B, LIX, CT-1, CD27L, Endoglin, TRANCE, MCSF, 4-1BB, Leptin R, CD36, TremL 1, VEGF R2, TGFbl, IL-3 Rb, H60 in the subject relative to the level of the corresponding cytokine in the corresponding normal subject indicates that the subject is at risk of having AD or has AD. In one embodiment, the change (e.g. increasing or decreasing) in the level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% of the cytokines in the cytokine profile composed of the cytokines selected from the followings: OPG, Resistin, TARC, VEGF, Chemerin, IL-9, MMP-2, VEGF-D, TCA-3, gp130, MMP-10, 6Ckine, VEGF-B, IL-22, IL-la, IFNg R1, Granzyme B, LIX, CT-1, CD27L, Endoglin, TRANCE, MCSF, 4-1BB, Leptin R, CD36, TremL 1, VEGF R2, TGFbl, IL-3 Rb, H60 in the subject relative to the level of the corresponding cytokine in the corresponding normal subject indicates that the subject is at risk of having AD or has AD.

In some embodiments, the change (e.g. increasing or decreasing) in the level of one or more (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31) cytokines selected from the followings: OPG, Resistin, TARC, VEGF, Chemerin, IL-9, MMP-2, VEGF-D, TCA-3, gp130, MMP-10, 6Ckine, VEGF-B, IL-22, IL-la, IFNg R1, Granzyme B, LIX, CT-1, CD27L, Endoglin, TRANCE, MCSF, 4-1BB, Leptin R, CD36, TremL 1, VEGF R2, TGFbl, IL-3 Rb, H60 in the subject with AD towards the level of the corresponding cytokines in the corresponding normal subject relative to the level of the corresponding cytokines of the corresponding normal subject indicates that the subject receives appropriate treatment. In one embodiment, the change (e.g. increasing or decreasing) in the level of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% of the cytokines in the intestinal microbial profile consisting of the cytokines selected from the followings: OPG, Resistin, TARC, VEGF, Chemerin, IL-9, MMP-2, VEGF-D, TCA-3, gp130, MMP-10, 6Ckine, VEGF-B, IL-22, IL-la, IFNg R1, Granzyme B, LIX, CT-1, CD27L, Endoglin, TRANCE, MCSF, 4-1BB, Leptin R, CD36, TremL 1, VEGF R2, TGFbl, IL-3 Rb, H60 in the subject with AD towards the level of the corresponding gut microbes in the corresponding normal subject relative to the level of the corresponding cytokines of the corresponding normal subject indicates that the subject receives appropriate treatment.

The present invention provides a composition that can directly or indirectly change the relative abundance of gut microbes to a predetermined level (such as a therapeutic level) for a predetermined amount of time (such as until the next dose is used). The predetermined level may be obtained from the measured relative abundance of the microbiota that led to the therapeutic response (for example, decreased metabolites, decreased lymphocyte infiltration into the brain, decreased activation of microglia, decreased neuroinflammation, improved cognition, relief of Alzheimer's disease symptoms).

In some embodiments, the method, agent or composition of the present invention may comprise sufficiently purified or enriched gut microbes such that the agent or composition may comprise at least about 5 wt%, 10 wt%, 20 wt%, 30 wt%, 40 wt%, 50 wt%, 60 wt%, 70 wt%, 80 wt%, 85 wt%, 90 wt%, 95 wt%, 99 wt%, or more of desired gut microbes based on the weight of the agent or composition, and/or less than about 40 wt%, 30 wt%, 20 wt%, 15 wt%, 14 wt%, 13 wt%, 12 wt%, 11 wt%, 10 wt%, 9 wt%, 8 wt%, 7 wt%, 6 wt%, 5 wt%, 4 wt%, 3 wt%, 2 wt%, 1 wt% or less of undesired gut microbes based on the weight of the agent or composition.

The agents and compositions that regulate the relative abundance of gut microbes according to the present invention can lead to altered metabolic functions. For example, the altered metabolic function may include the regulation of the amino acid level of microbial metabolites.

In some embodiments, by detecting a peripheral blood sample from a subject, the methods, agents, and compositions of the present invention can regulate the amino acid level by about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300 µM or more, or the range consistuted by the endpoints of the aforementioned value or any value therein; and/or make the amino acid level close to or reach the corresponding amino acid level of the corresponding normal subject.

In some embodiments, by detecting a faecal sample from a subject, the methods, agents, and compositions of the present invention can regulate the amino acid level by about 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000 µmol/g or more, or the range consistuted by the endpoints of the aforementioned value or any value therein; and/or make the amino acid level close to or reach the corresponding amino acid level of the corresponding normal subject.

In some embodiments, the methods, agents, and compositions of the present invention can cause altered immune cell infiltration into the brain. For example, the proportion of pro-inflammatory Th1 cells in CD4+ T cells is reduced. In some embodiments, the agents and compositions of the present invention can reduce the ratio of pro-inflammatory Th1 cells to CD4+ T cells in a sample from a subject by about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; and/or make the proportion of pro-inflammatory Th1 cells to CD4+ T cells close to or reach the proportion of corresponding pro-inflammatory Th1 cells to CD4+ T cells in a corresponding normal subject.

In some embodiments, the methods, agents, and compositions of the present invention can reduce the relative uptake of amino acids by naive T cells. In some embodiments, the agents and compositions of the present invention can reduce the relative uptake of amino acids by naive T cells by about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300 or more; and/or make the relative uptake level of amino acids by the naive T cells close to or reach the relative uptake level of amino acids by the corresponding naive T cells of the corresponding normal subject.

In some embodiments, the methods, agents, and compositions of the present invention can result in altered activation of microglia in the brain. For example, altered activation of microglia in the brain can include an increase or decrease in activation of microglia in the brain. The activation of microglia in the brain can be increased or decreased by about 1 to 100%, for example, about 1%, 2%, 3%, 4%, 5%, 10%, 15%, 20%, 25%, 30%, 35%, 40%, 45%, 50%, 55%, 60%, 65%, 70% , 75%, 80%, 85%, 90%, 95% or 99% or 100%, or the range consistuted by the endpoints of the aforementioned value or any value therein, for example, about 7% to about 28%, and the like., or about 7%, 14%, 21%, 28%, and the like.

In some embodiments, the methods, agents, and compositions of the invention can result in altered IBA1 levels. For example, changing the level of IBA1 can include increasing or decreasing the level of IBA1. IBA1 level can be increased or decreased by about 0 to about 3000 relative levels, for example, a relative level of about 0, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2150, 2200, 2250, 2300, 2350, 2400, 2450, 2500, 2550, 2600, 2650, 2700, 2750, 2800, 2850, 2900, 2950, 3000 relative levels, or the range consistuted by the endpoints of the aforementioned value or any value therein, for example, about 500 to about 3000, or about 500, 1000, 1500, and the like.

### Formulations

The agent or pharmaceutical composition of the present invention may contain gut microbes that can regulate the relative abundance of gut microbes in a subject. Gut microbes can be viable (live), dormant, inactive or dead bacteria. The agent or pharmaceutical composition of the present invention may contain a compound or agent that can regulate the relative abundance of gut microbes in a subject. One or more compounds or agents can be used to alter the microbiota in the subject. Such compounds or agents may include, but are not limited to, antibiotic treatments and/or antibacterial agents, prebiotics, such as bacterial cell wall components, bacterial nucleic acids (such as DNA and RNA), bacterial membrane components, and bacterial structural components (such as proteins, carbohydrates, lipids and their combinations, such as lipoproteins, sugar esters and glycoproteins), organic acids, inorganic acids, alkalis, proteins and peptides, enzymes and coenzymes, amino acids and nucleic acids, sugars, lipids, glycoproteins, lipoproteins, sugar esters, vitamins, biologically active compounds, metabolites comprising inorganic components, small molecules such as nitrogen-containing molecules or sulfurous acid-containing molecules, resistant starch, potato starch or high amylose starch, modified starch (including carboxylated starch, acetylated starch, propionated starch and butylated starch), non-digestible oligosaccharides, such as fructo-oligosaccharide, oligodextrose, xylo-oligosaccharide, galacto-oligosaccharide, arabinoxylan, arabinogalactan, galactomannan polysaccharides, polydextrose, oligofructose, inulin and their derivatives, but other oligosaccharides, other soluble fibers and a combination thereof that can play a prebiotic role are not excluded. The sugar can be selected from monosaccharides, disaccharides, oligosaccharides, polysaccharides, or derivatives thereof, or a combination of them and/or derivatives thereof; preferably oligosaccharides and polysaccharides; more preferably mannuronic acid oligosaccharides.

The agent or pharmaceutical composition of the present invention may also include, for example, amino acids, amino sugars, sugar alcohols, proteins, carbohydrates, monosaccharides, disaccharides, oligosaccharides, polysaccharides, nucleic acids, buffers, surfactants, lipids, liposomes, other excipients, and mixtures thereof. Other useful ingredients may include steroids, anti-inflammatory agents, non-steroidal anti-inflammatory agents, analgesics, cells, anti-inflammatory agents, growth factors, growth factor fragments, small molecule wound healing stimulators, hormones, cytokines, peptides, antibodies, enzymes, isolated cells, platelets, immunosuppressants, nucleic acids, cell types, viruses, viral particles, essential nutrients, minerals, metals, or vitamins, and a combination thereof. In addition, the agents and compositions of the present invention may comprise diluents such as water, saline or buffer.

The agent or pharmaceutical composition of the present invention can be formulated as a pharmaceutical composition including a pharmaceutically acceptable carrier. As used herein, "pharmaceutically acceptable carrier" includes any and all physiologically compatible solvents, dispersion media, coatings, antibacterial and antifungal agents, isotonic and absorption delaying agents, and the like. In one embodiment, the agent or pharmaceutical composition of the invention may be incorporated into a pharmaceutical composition suitable for delivery to a subject. The pharmaceutical composition may also include a pharmaceutically acceptable carrier. As used herein, "pharmaceutically acceptable carrier" includes any and all physiologically compatible solvents, dispersion media, coatings, antibacterial and antifungal agents, isotonic and absorption delaying agents, and the like. Examples of pharmaceutically acceptable carriers include one or more of the following: water, saline, phosphate buffered saline, glucose, glycerol, ethanol, etc., and a combination thereof. In many cases, it is preferable to include isotonic agents such as sugars, polyalcohols such as mannitol, sorbitol or sodium chloride in the composition.

The pharmaceutical composition can be formulated in a variety of forms. These include, for example, liquid, semi-solid, and solid preparation forms such as liquid solutions (such as injectable and infusible solutions), dispersions or suspensions, tablets, pills, powders, liposomes, suppositories, and other formulations. The pharmaceutical composition can be formulated for high drug concentration. The pharmaceutical composition may further be sterile and stable under handling and storage conditions. Sterile injection solutions can be prepared by incorporating it with one of the ingredients listed above or a combination thereof (as required) in a suitable solvent in the required amount, followed by filtration and sterilization.

The exemplary form of the pharmaceutical composition may depend on the intended mode of delivery and therapeutic application. In one embodiment, the pharmaceutical composition is formulated for oral delivery. Some compositions may be in the form of pill-based delivery (as disclosed in U.S. Patent Application No. 12/976,648 entitled "pill catchers" filed on October 22, 2010) and extended release methods. In one embodiment, pill-based delivery may be part of a system that allows delivery to occur at a precise location within the intestinal tract. In another embodiment, the pharmaceutical composition can be formulated as an extended release formulation. In another embodiment, the pharmaceutical composition may be encapsulated in a coating, which does not begin to degrade until it leaves the patient's stomach. In another embodiment, the pharmaceutical composition can be prepared with a carrier that protects the composition from rapid release, such as a sustained or controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for preparing such formulations have been granted patent rights or are well known to those skilled in the art. See, for example, Sustained and Controlled Release Drug Delivery Systems, J.R. Robinson, ed., Marcel Dekker, Inc., New York, 1978. "Sustained release" refers to the release of the composition or its active compound over an extended period of time relative to the release achieved by the delivery of a conventional formulation of the composition.

Another type of pharmaceutical composition includes an activatable form, such as formulating the composition with a microbiota in a dormant or inactive state (eg, a lyophilized state). In the combined composition, the microbiota can be in a dormant or inactive state, or the compound or agent that cultivates the microbiota can be inactive. In an exemplary embodiment, the pharmaceutical composition may be formulated to include at least one dormant or inactive microbiota and an inactive compound or agent that cultivates the microbiota.

The disclosed pharmaceutical compositions and combined pharmaceutical compositions can also be formulated into foods, beverages, dietary supplements and/or additives. Such compositions are those suitable for human and/or animal consumption. Those skilled in the art can readily know the specific formulations of the microbiota that can be used in oral or ingestible formulations and are considered suitable for human and/or animal administration. Many compositions are used in the manufacture of food or food additives/supplements; therefore, another important aspect is to provide human or animal food or food additives/supplements including microbiota to regulate the gut microbes of the subject.

The consumable composition may be formulated to include sweeteners, stabilizers or binders, humectants, and/or natural and/or artificial flavors. The composition may also include natural and/or artificial colorants and preservatives. In one embodiment, the composition may include monosaccharides, disaccharides and polysaccharides, such as, but not limited to, sucrose (sugar), dextrose, maltose, dextrin, xylose, ribose, glucose, mannose, galactose, sucromalt, fructose (levose), invert sugar, corn syrup, maltodextrin, oligofructose syrup, partially hydrolyzed starch, corn syrup solids, polydextrose, soluble fiber, insoluble fiber, natural cane juice, gelatin, citric acid, lactic acid, natural colors, natural flavors, fractionated coconut oil, carnauba wax, or a combination thereof.

### Dosage

The agent or composition of the present invention may comprise a "therapeutically effective amount" or "effective amount" of ingredients. "Therapeutically effective amount" refers to an amount that is effective to achieve the desired therapeutic result at the required dose and within a period of time. The therapeutically effective amount of the agent or pharmaceutical composition can vary depending on various factors such as the individual's disease state, age, sex, and brain, and the ability of the composition to cause a desired response in the individual. A therapeutically effective amount is also an amount in which the therapeutically beneficial effects of the agent or pharmaceutical composition exceed the toxic or harmful effects of the agent or pharmaceutical composition. In an exemplary embodiment, the therapeutically effective amount of the agent or pharmaceutical composition is an amount in which the relative abundance of one or more gut microbes is increased. For example, a therapeutically effective amount of an agent for regulating the relative abundance of gut microbes increases the relative abundance of one or more gut microbes in the subject.

As used herein, the term "treatment" generally refers to obtaining a desired pharmacological and/or physiological effect. The effect may be prophylactic in terms of completely or partially preventing the disease or its symptoms; and/or may be therapeutic in terms of partially or completely stabilizing or curing the disease and/or side effects due to the disease. "Treatment" as used herein covers any treatment of a patient's disease, including: (a) prevention of diseases or symptoms that occur in patients who are susceptible to diseases or symptoms but have not yet been diagnosed with the disease; (b) inhibiting the symptoms of the disease, such as inhibiting the progression of the disease and preventing the development of the disease; or (c) alleviating the symptoms of the disease, that is, causing the disease or symptoms to degenerate.

### Indications

Through the research of the present invention, the inventors found that the abnormal intestinal microbial pattern caused the naive T cells to over-differentiate into Th1 cells, and the level of Th1 cells in the peripheral blood increased abnormally. The increased levels of peripheral Th1 cells cause more Th1 cells to infiltrate the brain, causing diseases such as Alzheimer's disease. By restoring the gut microbiota, the differentiation of naive T cells into Th1 cells can be reduced, and the abnormally elevated level of Th1 cells in the peripheral blood can be reduced, thereby treating diseases. Therefore, the present invention provides a method for treating diseases related to high peripheral blood Th1 cell levels in a subject, the method comprises regulating the relative abundance of gut microbes in a subject as described herein, reducing the level of amino acids in peripheral blood as described herein, or inhibiting the naive T cell from uptaking the amino acids in peripheral blood as described herein.

The terms "Th1 dominance" and "Th1 cell dominance" are used interchangeably as described herein. In some cases, Th1 dominance can be represented by high peripheral blood Th1 cell levels. High peripheral blood Th1 cell level may refer to the peripheral blood Th1 cell level in the subject being 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500%, 550%, 600%, 650%, 700%, 750%, 800%, 850%, 900%, 950%, 1000% or more higher than that of a suitable control (for example, a normal control, such as a normal human population), or the range consistuted by the endpoints of the aforementioned value or any value therein, such as about 7 % to about 28%, and the like, or about 7%, 14%, 21%, 28%, and the like. Such diseases include, but are not limited to, Multiple Sclerosis, Crohn's Disease, Type 1 Diabetes, Rheumatoid Arthritis, Hashimoto's Thyroiditis, Vitiligo, Sjögren's Syndrome, Polycystic ovarian syndrome (PCOS), Celiac Disease and Graves's disease.

As used herein, the term "subject" refers to any living organism, including, but not limited to, humans, non-human primates such as chimpanzees and other apes and monkeys, farm animals such as cows, sheep, pigs, goats and horses, domesticated mammals such as dogs and cats, laboratory animals, including rodents such as mice, rats, rabbits, guinea pigs and the like. The term does not indicate a specific age and gender. In one embodiment, the subject is a human. In the context of the present invention, the terms "subject", "individual" and "patient" are used interchangeably.

In one embodiment, the relative abundance of one or more of the gut microbes in the subject is higher than that of a normal subject. In one embodiment, the relative abundance of one or more of the gut microbes in the subject is lower than that of a normal subject. In one embodiment, the relative abundance of one or more of the gut microbes in the subject is higher than that of the normal subject, and the relative abundance of the other one or more is lower than that of the subject.

The dosage may depend on the type of microbiota present in the agent or pharmaceutical composition of the invention. The dosage can also be determined based on the relative abundance of one or more microbiota present in the subject.

In one embodiment, the agent or pharmaceutical composition of the present invention can effectively change the relative abundance of one or more microbiota. In another embodiment, the agent or pharmaceutical composition can effectively increase or decrease the relative abundance of the microbiota in the subject. In one embodiment, the agent or pharmaceutical composition can increase or decrease the relative abundance of specific microorganisms of the microbiota in the subject, and decrease the relative abundance of other specific microorganisms of the microbiota in the subject.

In another embodiment, the agent or pharmaceutical composition of the present invention can also effectively change the microbiota in the subject, so that after administration of the agent or pharmaceutical composition, the microbiota in the subject mimics the microbiota present in the subject responding to the Alzheimer's treatment process. The agent or pharmaceutical composition can effectively change the microbiota to simulate the microbiota of normal and healthy subjects with similar brains, age, sex, race and the like.

The dosage regimen can be adjusted to provide the most suitable desired response (such as a therapeutic response or a preventive response). For example, a single bolus can be delivered, multiple divided doses can be delivered over time or the dose can be reduced or increased proportionally as dictated by the urgency of the treatment situation. It is particularly advantageous to formulate parenteral compositions in unit dosage form to facilitate delivery and uniformity of dosage. Unit dosage form as used herein refers to a physically discrete unit suitable as a single dose for a mammalian subject to be treated; each unit contains a predetermined amount of active compound calculated to produce the desired therapeutic effect in combination with the required pharmaceutical carrier. The specifications of the unit dosage form used in the present invention are defined by or directly depend on the following: (a) the unique properties of the active compound and the specific therapeutic or preventive effect to be achieved; and (b) the limitations inherent in the field of combining this active compound for individual therapy.

When applied to the methods provided by the present invention, an exemplary dosage range of the agent or pharmaceutical composition of the present invention can be about 0.001 to about 100 mg/kg body weight per day, about 0.01 to about 50 mg/kg body weight per day, such as about 0.05 to about 10 mg/kg body weight per day, delivered in one or more doses, such as 1-3 doses. The exact dosage will depend on the frequency and mode of delivery, the sex, age, weight and general condition of the subject being treated, the nature and severity of the condition being treated, any comorbidities that will be treated and other factors known to those skilled in the art.

In one embodiment, the agent or pharmaceutical composition of the present invention comprises a microbiota, such as *Verrucomicrobia,* having a total concentration in the range of about 0.001 mg/kg to about 100 mg/kg. In another embodiment, the agent or pharmaceutical composition comprises a microbiota, such as *Verrucomicrobia,* having a total concentration in the range of about 0.1 mg/kg to about 50 mg/kg. In further another embodiment, the agent or pharmaceutical composition comprises a microbiota, such as *Verrucomicrobia,* having a total concentration in the range of about 1 mg/kg to about 10 mg/kg.

In some aspects, the agents, compositions, or kits described herein are administered in doses based on the weight of the subject. In some embodiments, the agents, compositions or kits described herein comprise one or more agents mentioned herein in an amount by the weight of the subject

In some embodiments, the amount of one or more of the agents mentioned herein in the agents, compositions or kits described herein is in the range of about 1.0 to about 50.0 mg/kg or more, preferably about 5.0 to 40.0 mg/kg, more preferably about 10.0 to 30.0 mg/kg, based on the weight of the subject. In some embodiments, the amount of one or more of the agents mentioned herein in the agents, compositions, or kits described herein is about 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5, 50.0 mg/kg or more or the range consistuted by the endpoints of the aforementioned value or any value therein, such as about 1.1 to 1.4 mg/kg and the like or about 1.1, 1.2, 1.3, 1.4 mg/kg, and the like, based on the weight of the subject.

In other aspects, the agents, compositions or kits described herein are administered in fixed doses. In some embodiments, the agents, compositions or kits described herein comprise a fixed amount of one or more agents mentioned herein.

In some embodiments, the amount of one or more of the agents mentioned herein in the agents, compositions or kits described herein is each independently about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200 mg or more, or the range consistuted by the endpoints of the aforementioned value or any value therein, for example, about 1.1 to 1.4 mg and the like or about 1.1, 1.2, 1.3, 1.4 and the like.

In still other aspects, some of the components of the agents, compositions, or kits described herein are administered at doses by weight of the subject as described above, while other components are administered at fixed doses as described above. In some embodiments, the amounts of some of the components in the agents, compositions, or kits described herein are the amounts based on the weight of the subject as described above, and the other components are fixed amounts as described above.

### Delivery

The agent or pharmaceutical composition of the present invention can be delivered or administered by various methods known in the art. The terms "delivery", "deliver to", "administer" and "apply to" are used interchangeably herein. As those skilled in the art realize, the route and/or mode of delivery will vary according to the desired result. In one embodiment, the agent or pharmaceutical composition of the invention is delivered orally. In another embodiment, the agent or pharmaceutical composition of the invention is delivered orally. In another embodiment, the agent or pharmaceutical composition of the invention is delivered nasally. Yet another mode of delivery may include methods and combinations of delivery to the intestine.

The agent or pharmaceutical composition of the present invention can be delivered to a target region and/or structure in a subject. The region that can be targeted in the intestine may include, but is not limited to, stomach, biliary pancreatic branch (limb), Roux branch, common branch, ileum, cecum, or colon. It can be targeted to a structure that constitutes a differentiated ecological location with a specific pH range, temperature, humidity, and metabolite content. Diseases or conditions associated with altered microbiota genealogy can show the presence of new microorganisms, the lack of normal microorganisms, or a change in the proportion of microorganisms.

The delivery of the agent or pharmaceutical composition of the present invention can be targeted to one or more regions in the subject. The area may include, but is not limited to, the region in the intestine. In an exemplary embodiment, the delivery is targeted to the oral cavity, stomach, biliary pancreatic branch, Roux branch, common branch, small intestine, ileum, cecum, large intestine, or colon in the intestine. The delivery can also be targeted to one or more tissues in the subject. The tissue may include any tissue in the intestine, such as stomach, biliary pancreatic branch, Roux branch, common branch, small intestine, ileum, cecum, large intestine, or colon.

The components of the agent or pharmaceutical composition of the present invention can be formulated separately, or part or all of them can be formulated together. In one embodiment, the agent or pharmaceutical composition of the present invention can be formulated into an agent or pharmaceutical composition suitable for single or multiple administrations.

The components of the agent or the pharmaceutical composition of the present invention may be administered separately, or part or all of them may be administered together. The agents or components of the pharmaceutical composition of the present invention may be administered at substantially different times, or some or all of them may be administered at substantially the same time.

The agents or components of the pharmaceutical composition of the present invention can each be independently administered by various suitable routes, including, but not limited to, oral or parenteral (by intravenous, intramuscular, topical or subcutaneous routes). In some embodiments, the components of the agent or the pharmaceutical composition of the present invention can each be independently administered orally or by injection, such as intravenous injection or intraperitoneal injection.

The components of the agent or pharmaceutical composition of the present invention may each independently be a suitable dosage form, including, but not limited to, dosage forms of tablets, troches, pills, capsules (for example hard capsules, soft capsules, enteric-coated capsules, microcapsules), elixirs, granules, syrups, injections (intramuscular, intravenous, intraperitoneal), granules, emulsions, suspensions, solutions, dispersions, and sustained-release preparations for oral or parenteral administration.

The agents or components of the pharmaceutical composition of the present invention may each independently comprise a pharmaceutically acceptable carrier and/or excipient.

The agents of the present invention or the components of the pharmaceutical composition can be administered independently every 1 day, every 2 days, every 3 days, every 4 days, every 5 days, every 6 days, every week, every 2 weeks, every 3 weeks or every month or at a lower frequency.

The agents of the present invention or the components in the pharmaceutical composition can be administered independently, 1 time, 2 times, 3 times, 4 times, 5 times, 6 times, 7 times, 8 times, 9 times, or 10 times or more per day.

The agents of the present invention or the components of the pharmaceutical composition can be administered independently for 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 consecutive days or more.

One of the components in the agent or pharmaceutical composition of the present invention can be administered 1, 2, 3, 4, 5, 6, 7, 8, 9 or 10 or more days before or after administration of the other component. For example, in one embodiment, the agent or component 1 of the pharmaceutical composition of the present invention is administered on day 1, and the agent or component 2 of the pharmaceutical composition of the present invention is administered 2 days later (i.e. day 3), and the agent or component 1 of the pharmaceutical composition of the present invention is administered another three days later (i.e. day 6).

The delivery of the agent or pharmaceutical composition of the present invention can also be repeated one or more times. The repeated delivery of the agent or pharmaceutical composition of the present invention can be one or more times before and/or after the treatment of the disease. Repeated delivery can be in a similar manner to the initial delivery.

The agent or pharmaceutical composition of the present invention can also be administered together with an agent that may include a therapeutic, preventive or diagnostic agent. The therapeutic, preventive or diagnostic agent is selected from small molecules, nucleic acids, proteins, such as polypeptide prebiotics, including bacterial components (such as bacterial cell wall components (such as peptidoglycan), bacterial nucleic acid (such as DNA and RNA), bacterial membrane components, and bacterial structural components (such as proteins, carbohydrates, lipids and combinations of these, such as lipoproteins, sugar esters and glycoprotein)), bacterial metabolites, organic acids, inorganic acids, bases, proteins and peptides, enzymes and coenzymes, amino acids and nucleic acids, carbohydrates, lipids, glycoproteins, lipoproteins, sugar esters, vitamins, biologically active compounds, metabolites comprising inorganic components, small molecules (for example, nitrogen-containing molecules or sulfurous acid-containing molecules), resistant starch, potato starch or high amylose starch, modified starch (including carboxylated starch, acetylated starch, propionated starch and butylated starch), non-digestible oligosaccharides such as fructooligosaccharides, dextrose, xylo-oligosaccharide, galacto-oligosaccharide, arabinoxylan, arabinogalactan, galactomannan, polydextran, oligofructose, inulin and their derivatives (but other oligosaccharides that can play a prebiotic role are not excluded) other soluble fibers and a combination thereof. In one embodiment, the delivered agent is a delivered small molecule that has low oral bioavailability and acts on the microbial location of the host intestine. Low oral bioavailability is generally undesirable in medicine, because intestinal absorption is the goal of most oral treatments.

The agent or pharmaceutical composition of the present invention may also be administered in the same composition as the above-mentioned agent, or may be administered separately from the agent administered before, at the same time, and/or after the agent or pharmaceutical composition of the present invention. The agent or pharmaceutical composition of the present invention can be administered at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 or more days before the administration of the agent. The agent or pharmaceutical composition of the present invention can also be administered at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 or more days after the administration of the agent.

The agent or pharmaceutical composition of the invention can also be delivered by an at least partially implantable system. The implantable system can be any system known and used in the art. The system may include programmable pumps (such as those commonly used to deliver insulin to diabetic patients). One or more of these components may be modular and connected to a transdermal delivery system that may include ports, needles, patches, and the like. In an exemplary embodiment, the implantable system includes a reservoir and a port. The reservoir may include a refillable or reloadable container for holding the composition. In another embodiment, the system may include a catheter. In another embodiment, the implantable system is a transluminal catheter. The system can also be configured to deliver the composition at a prescribed dose and/or at a prescribed interval. The prescribed dose and/or prescribed interval can be determined by those skilled in the art.

Some embodiments of the invention are illustrated by the following non-limiting examples.

### EXAMPLE

### Materials and Methods

**Animals.** Tg mice and co-housed WT mice (corresponding WT mice generated by mating Tg mice and C57 mice) were housed in the same cage after birth. WT mice (C57) were housed in separate cages to avoid cross-transfer of microbiota. All mice were kept in a 23°C room with a 12-hour (h) light-dark cycle. Mice were randomly assigned to different groups before treatment. For time course analysis of Tg mice, male and female Tg mice were sacrificed at 2, 3, 5, 7 and 9 months of age. The mouse brain was collected and stained for immune cell analysis and cytokine analysis. Before the mice were sacrificed, feces were collected for gut microbiota analysis. For OM1 treatment, at the age of 6.5 months, based on 450 mg b.i.d. (twice a day) in the phase III trial, Tg mice were treated with 100 mg/kg OM1 by oral administration once a day for one month. Behavioral tests were performed to monitor cognitive activity after the last treatment. The brains and feces of the mice were then used for different analyses. For behavioral testing, Tg mice and WT mice in different months and treated mice were tested by discrimination learning, as previously reported. All mouse movements that occur in PhenoTyper (HomeCage) are recorded by a computerized tracking system, which calculates the time and number of entrances required to reach 80% of the performance standard (Noldus, Ethovision). For the intraperitoneal injection of phenylalanine and isoleucine, C57 mice of 4 months old were treated with 50 mg/kg of phenylalanine or isoleucine for 4 days. For hippocampal injection of phenylalanine and isoleucine, C57 mice of 4 months old were injected with 3 µL of phenylalanine or isoleucine at 120 mmol/L. After 10 days, these mice were subjected to blood sample analysis and behavioral testing using FACS.

For APP/PS1 mouse time point analysis, mice and age-matched wild-type mice were sacrificed at 3, 9 and 14 months of age. Mouse brain and blood were collected for different analysis. Before sacrifice, mouse feces were collected for microbiota analysis. For APP/PS 1 mouse treatment, 6-month-old mice treated with or without antibiotics for 3 months were treated with 50 mg/kg of OM1 by oral gavage once a day. Behavioral tests were performed to monitor cognitive activity after the last treatment. The animal experiment was approved by the ethics committee of Shanghai SIPPR-Bk Lab Animal Co., Ltd (No.: 2016002).

**Faecal sample DNA extraction and PCR amplification.** All faecal samples were frozen at -80 °C before DNA extraction and analysis. Microbial DNA was extracted from faecal samples using the E.Z.N.A.^{®} Soil DNA Kit (Omega Bio-Tek, Norcross, GA, U.S.) according to the manufacturer's protocols. The final DNA concentration and purification were determined by a NanoDrop 2000 UV-vis spectrophotometer (Thermo Scientific, Wilmington, USA), and DNA quality was checked by 1% agarose gel electrophoresis. The V3-V4 hypervariable regions of the bacterial 16S rRNA gene were amplified with primers 338 F (5'-ACTCCTACGGGAGGCAGCAG-3') and 806 R (5'-GGACTACHVGGGTWTCTAAT-3') by a thermocycler PCR system (GeneAmp 9700, ABI, USA). PCR reactions were conducted using the following program: 3 min (min) of denaturation at 95 °C, 27 cycles of 30 sec (s) at 95 °C, 30 s for annealing at 55 °C, and 45 s for elongation at 72 °C, and a final extension at 72 °C for 10 min. PCR reactions were performed in triplicate in a 20 µL mixture containing 4 µL of 5 × FastPfu Buffer, 2 µL of 2.5 mmol/L dNTPs, 0.8 µL of each primer (5 µmol/L), 0.4 µL of FastPfu Polymerase and 10 ng of template DNA. The resulting PCR products were extracted from a 2% agarose gel and further purified using the AxyPrep DNA Gel Extraction Kit (Axygen Biosciences, Union City, CA, USA) and quantified using QuantiFluor^{™}-ST (Promega, USA) according to the manufacturer's protocol. A degenerate primer refers to a mixture of different sequences that represent all the different base possibilities for encoding a single amino acid. In order to increase specificity, one can refer to the codon usage table to reduce degeneracy according to the base usage preferences of different organisms. In addition to the normal four base symbols of A, T, G, and C, other letters such as R, Y, M, K and the like appear in the nucleotide chain, wherein R=A/G, Y=C/T, M=A/C, K=G/T, S=C/G, W= A/T, H= A/C/T, B= C/G /T, V=A/C/G, D=A/G /T, N=A/C/G/T.

**Illumina MiSeq sequencing**. According to the standard protocol of Majorbio Bio-Pharm Technology Co. Ltd. (Shanghai, China), purified amplicons were pooled in equimolar and paired-end sequenced (2 × 300) on an Illumina MiSeq platform (Illumina, San Diego, USA).

Processing of sequencing data. Raw fastq files were demultiplexed, quality filtered by Trimmomatic and merged by FLASH based on the following criteria: (i) The reads were truncated at any site that received an average quality score < 20 over a 50 bp sliding window. (ii) The primers were exactly matched, allowing a 2-nucleotide mismatch, and reads containing ambiguous bases were removed. (iii) Sequences with overlaps of longer than 10 bp were merged according to their overlap sequence. Operational taxonomic units (OTUs) were clustered with a 97% similarity cutoff using UPARSE (version7.1 http://drive5.com/uparse/), and chimeric sequences were identified and removed using UCHIME. The taxonomy of each 16S rRNA gene sequence was analyzed by the RDP Classifier algorithm (http://rdp.cme.msu.edu/) against the Silva (SSU123) 16S rRNA database using a confidence threshold of 70%.

**Metabolites sample preparation**. Samples stored at -80 °C were taken out and thawed at room temperature. In the experiment, 50 mg samples were used, and 400 µL methanol-water (4:1, v/v) were also added to homogenize the sample using a homogenizer for 10 seconds. The solution was ultrasonically extracted on ice for 10 min and stored at -20 °C for 30 min, then centrifuged for 15 min at 13000 rpm at 4 °C. For LC-MS analysis, 200 µL supernatant was used.

**LC/MS analysis parameters.** LC-MS was performed on AB Sciex TripleTOF 5600TM mass spectrometer system (AB SCIEX, USA). LC Conditions: Column: Acquity BEH C18 column (100 mm × 2.1 mm i.d., 1.7 µm; Waters, Milford, USA). Solvent: The column was maintained at 40 °C and separation was achieved using the following gradient: 5% B-30% B over 0-3 min, 30% B-95% B over 3-9 min, 95% B-95% B over 9-13.0 min; 95% B-5% B over 13.0-13.1 min, and 13.1-16 min holding at 5 % B; the flow rate is 0.40 mL/min, wherein B is acetonitrile: isopropanol 1:1 (0.1% (v/v) formic acid) and A is aqueous formic acid (0.1% (v/v) formic acid). Injection Volume was 20 µL. The mass spectrometric data was collected using an AB Sciex TripleTOF 5600TM mass spectrometer system equipped with an electrospray ionization (ESI) source operating in either positive or negative ion mode with a capillary voltage 1.0 kV, sample cone, 40 V, collision energy 6 eV. The source temperature was set at 120 °C, with a desolvation gas flow of 45 L/h. Centroid data was collected from 50 to 1000 m/z with a 30000 resolution.

**Metabolites QC sample preparation.** QC sample was prepared by mixing aliquots of all samples to be a pooled sample and then analyzed using the same method with the analytic samples. The QCs were injected at regular intervals (every 10 samples) throughout the analytical run to provide a set of data from which repeatability can be assessed.

***In vitro* differentiation of naive CD4 to Th1 and Th2 induced by the supernatant of mice feces.** Naive CD4⁺ T cells were seprated from the splenocytes of 8-month-old C57BL/6 female mice using the naive CD4⁺ T cell Isolation Kit (Stem Cell, #19765). As decribed above, supernatant of feces of 7-month-old mice were prepared. A total of 0.5 × 10⁶ cells/well in 0.5 mL of RPMI-1640 medium were plated in 48-well anti-CD3 and anti-CD28 pre-coated plates, and the culture medium was supplemented with cytokines and blocking antibodies. Th0: 20 ng/mL mIL-2; Th1: 20 ng/mL mIL-2, 10 µL supernatant, 5000 ng/mL 11B11; Th2: 20 ng/mL mIL-2, 10 µL supernatant, 5000 ng/mL XMG1.2. OM1 was added to the designated wells to a final concentration of 100 µg/mL. After incubation at 37 °C in 5% CO₂ for 5 days, cells were acquired on a BD Aria III cytometer, and data were analyzed using FlowJo software.

**Immunohistochemistry.** For 3,30-diaminobenzidine (DAB)-developed staining, sections were immunostained using a Leica BOND-RX Autostainer (Leica Microsystems) and Coverslipper CV5030 (Leica Microsystems) according to the manufacturer's IHC protocol. Briefly, sections were submitted to heat-induced epitope retrieval with Epitope Retrieval solution 2 (ER2, AR9640, Leica Biosystems) for 20 min. Endogenous peroxidase activity was blocked with 3%-4% (v/v) hydrogen peroxide (part of DS9800, Leica Biosystems) for 10 min. Then, sections were incubated with blocking buffer (10% NGS in PBS with 0.3% Triton x-100) for 60 min. Finally, staining was performed using the Bond Polymer Refine Detection System (DS9800, Leica Biosystems) according to the manufacturer's protocol. The primary antibody incubation time was 30 min. Sections were stained for activated microglia using rabbit anti-IBA1 antibody (1:1,000, cat# 019-19741, Wako), amyloid depositions using mouse anti-Aβ42 antibody (1:1,000; cat# 803003, Biolegend) and Tau phosphorylation using mouse anti-PHF-Tau & tangles-Thr231 antibody (1:300, cat# MN1040, Thermo Fisher). Stained slices were automatically scanned by a high-throughput bright field scanner (NanoZoomer 2.0HT, Hamamatsu), and images were obtained by NDP.scan 3.2 software (Hamamatsu). For fluorescent staining, slides were blocked by blocking buffer (10% NGS in PBS with 0.3% Triton x-100) at RT for 1 h, and then incubated in the primary antibody solution (Iba-1 1:1,000, Aβ42 1:1,000, Tau 1:300) overnight at 4 °C. After washing, slides were incubated with fluorescent anti-rabbit or anti-mouse secondary antibody (1:1000, Invitrogen) for 60 min at RT and further washed 3 times in PBS. Finally, slides were counterstained with DAPI (1:10000 in PBS, Sigma) for 5 min at RT, washed, sealed and stored at 4 °C for image acquisition. Representative fluorescence images were acquired by upright fluorescence microscope Zmager-m2 (Zeiss, Germany) under 10× objective using Zen software (Zeiss).

**Neurotoxicity test.** SH-SY5Y cells (ATCC, USA) were seeded into a white polystyrene 96-well plate (Corning Inc., USA) at a density of 5000 cells/well, and were cultivated at 37°C and 5% CO₂ humidified incubator in the Dulbecco modified Eagle medium (DMEM) (Corning Inc., USA) supplemented with 10% fetal bovine serum (FBS), 100 units/mL penicillin and 100 µg/mL streptomycin. After 24 hours, the cells were treated with L-phenylalanine or L-isoleucine (Sigma) at final concentrations of 10 mM, 1 mM, and 0.1 mM, respectively. CellTiter-Glo (Promega Inc., USA) was used to detect cell viability after 24 hours. Cytation5 instrument (BioTek) was used to record the luminescence signal.

**Amino acid detection**. A set of amino acid standard mixture solutions was prepared at a concentration range of 100-2000 µmol/L. A portion of 10 µL of each standard mixture solution or plasma sample was pipetted into the bottom of a tube, and then 70 µL of sodium borate buffer (200 mmol/L at pH 8.8) was added. After 20 µL of 6-aminoquinolyl-N-hydroxysuccinimidyl carbamate (AQC) (4 mg/mL in acetonitrile) was added, the tube was closed and heated for 10 min at 55 °C to form AQC-amino acid. The solution was then cooled down to room temperature and 2 µL portion of each solution was injected into the UPLC-ESI-MS system for amino acid analysis without further purification.

### Human subjects.

The blood from MCI due to AD patients and healthy control was collected from a pilot study. MCI due to AD is defined in the NIA-AA 2011 clinical and research diagnostic criteria for MCI due to AD. The patients with MCI due to AD in this study must meet the following criteria. First, concern regarding a change in cognition. Second, impairment in one or more cognitive domains. Third, preservation of independence in functional abilities. Forth, not demented (NIA-AA 2011 clinical diagnosis criteria for MCI due to AD). All participants underwent a screening process that included a review of their medical history, physical and neurological examinations, laboratory tests, and MRI scans. The clinical assessment of mild cognitive impairment or dementia included neuropsychological tests, as well as behavioral and psychiatric interviews conducted by attending psychiatrists. AD patients recorded scores of < 4 on the Hachinski Ischemia Scale and showed no history of significant systemic or psychiatric conditions, or traumatic brain injuries that could compromise brain function. The Clinical Dementia Rating (CDR) and Montreal Cognitive Assessment (MoCA) were assessed for all of the participants. Based on the assessment, the inventors retained MCI due to AD subjects and others were excluded such as those who had impairment in a single non-memory domain (single, non-memory domain MCI subtype) and those who had impairment in two or more domains (multiple domains, slightly impaired MCI subtype). Normal control subjects had no history of cognitive decline, neurological disorders, or uncontrolled systemic medical disorders. All subjects included in this study had no more than two lacuna ischemia (of diameter < 1 cm) as revealed by MRI fluid-attenuated inversion recovery (FLAIR) sequence.

The sample size for the first cohort (Figure 5h, i) is 9 MCI due to AD patients and 18 healthy subjects. The sample size for the second cohort (Figure 5j) is 22 MCI due to AD patients and 22 healthy subjects. A diagnosis of MCI was based on the following criteria, which were adapted from the MCI diagnostic criteria of Petersen: (1) memory complaints, preferably corroborated by a spouse or relative; (2) objective memory impairment; (3) normal general cognitive function; (4) intact activities of daily living; and (5) absence of dementia. The Ethics Committee of the Shanghai Mental Health Centre Institutional Review Board approved the study (Number: 2016-22R1). Informed consents were obtained from the subjects and the guardian of the subjects. Information about gender and age etc. are provided below.

**First cohort:**

| Type | Sample ID | Age | Gender |
|---|---|---|---|
| Healthy | HC-01 | 75 | F |
| Healthy | HC-02 | 71 | F |
| Healthy | HC-04 | 69 | F |
| Healthy | HC-05 | 73 | F |
| Healthy | HC-08 | 63 | M |
| Healthy | HC-09 | 62 | M |
| Healthy | HC-10 | 68 | M |
| Healthy | HC-11 | 61 | M |
| Healthy | HC-12 | 68 | M |
| Healthy | HC-13 | 75 | F |
| Healthy | HC-14 | 70 | F |
| Healthy | HC-15 | 68 | M |
| Healthy | HC-16 | 62 | M |
| Healthy | HC-17 | 75 | M |
| Healthy | HC-18 | 65 | F |
| Healthy | HC-19 | 64 | F |
| Healthy | HC-20 | 64 | F |
| Healthy | HC-22 | 63 | F |
| MCI due to AD | MCI-02 | 61 | F |
| MCI due to AD | MCI-03 | 79 | M |
| MCI due to AD | MCI-04 | 71 | F |
| MCI due to AD | MCI-05 | 72 | F |
| MCI due to AD | MCI-06 | 80 | F |
| MCI due to AD | MCI-07 | 65 | M |
| MCI due to AD | MCI-09 | 61 | M |
| MCI due to AD | MCI-10 | 69 | M |
| MCI due to AD | MCI-14 | 77 | M |

**Second cohort**

| Type | Sample ID | Age | Gender |
|---|---|---|---|
| Healthy | HC-0149 | 73 | M |
| Healthy | HC-0150 | 70 | F |
| Healthy | HC-0153 | 63 | F |
| Healthy | HC-0156 | 67 | M |
| Healthy | HC-0158 | 71 | F |
| Healthy | HC-0165 | 61 | F |
| Healthy | HC-0168 | 62 | F |
| Healthy | HC-0172 | 70 | F |
| Healthy | HC-0176 | 71 | F |
| Healthy | HC-0187 | 73 | F |
| Healthy | HC-0191 | 65 | M |
| Healthy | HC-0013 | 70 | F |
| Healthy | HC-0043 | 81 | M |
| Healthy | HC-0050 | 64 | F |
| Healthy | HC-0051 | 66 | M |
| Healthy | HC-0056 | 75 | M |
| Healthy | HC-0068 | 67 | F |
| Healthy | HC-0074 | 57 | F |
| Healthy | HC-0076 | 60 | F |
| Healthy | HC-0081 | 71 | F |
| Healthy | HC-0089 | 57 | F |
| Healthy | HC-0090 | 76 | F |
| MCI due to AD | MCI-0169 | 65 | F |
| MCI due to AD | MCI-0175 | 61 | F |
| MCI due to AD | MCI-0211 | 69 | M |
| MCI due to AD | MCI-0229 | 65 | M |
| MCI due to AD | MCI-0240 | 59 | F |
| MCI due to AD | MCI-0244 | 64 | F |
| MCI due to AD | MCI-0257 | 61 | F |
| MCI due to AD | MCI-0260 | 60 | F |
| MCI due to AD | MCI-0282 | 66 | F |
| MCI due to AD | MCI-0284 | 64 | F |
| MCI due to AD | MCI-0287 | 75 | F |
| MCI due to AD | MCI-0303 | 62 | F |
| MCI due to AD | MCI-0319 | 63 | F |
| MCI due to AD | MCI-0332 | 67 | F |
| MCI due to AD | MCI-0374 | 61 | M |
| MCI due to AD | MCI-0385 | 68 | F |
| MCI due to AD | MCI-0401 | 57 | F |
| MCI due to AD | MCI-0414 | 64 | F |
| MCI due to AD | MCI-0001 | 75 | M |
| MCI due to AD | MCI-0043 | 55 | F |
| MCI due to AD | MCI-0061 | 63 | F |
| MCI due to AD | MCI-0091 | 78 | F |

***In** Vitro* differentiation and proliferation induced by phenylalanine and isoleucine. Naive CD4⁺ T cells were separated from the splenocytes of 8-month-old C57BL/6 female mice using the Naive CD4⁺ T cell Isolation Kit (Stem Cell, #19765), and the cells were stained with CellTrace (Thermo Fisher, #C34557). A total of 1 × 10⁵ cells/well in 0.2 mL of RPMI-1640 medium were plated in 96-well plates, and the culture medium was supplemented with cytokines or blocking antibodies. Th0: 10 ng/mL mIL-2; Th1:10 ng/mL mIL-2, 5000 ng/mL 11B11. Phenylalanine (final concentration, 2 mmol/L), isoleucine (final concentration, 2 mmol/L) and OM1 (final concentration, 100 µg/mL) were added into the indicated wells, respectively. After incubation at 37 °C in 5% CO₂ for 5 days, cells were acquired on a BD Fortessa cytometer, and data were analyzed using FlowJo software.

**Uptake of phenylalanine.** Naive CD4⁺ T cells were separated from the splenocytes of 8-month-old C57BL/6 female mice using the Naive CD4⁺ T cell Isolation Kit (Stem Cell, Cat No. 19765) and were induced to Th1 differentiation by 20 ng/mL IL-12. After 3 days, a total of 5 × 10⁵ cells/well Th1 cells and Th0 cells in 0.5 mL of RPMI-1640 medium were plated into 48-well plates. ¹³C-labelled phenylalanine and 5 mM amino transporter inhibitor BCH were added into indicated wells. After 0.5 h, cells were collected and washed twice with ice-cold D-PBS. 50 µL deionized water was added and cells were lysed through freezing and thawing twice at -80 °C. The cell lysate was centrifuged at 12000 × g for 10 min and ¹³C-labelled phenylalanine in the supernatant was analyzed by Mass spectrometry.

**Antibiotic treatments**. Mice were treated by adding an antibiotic solution (ATB) containing ampicillin (0.1 mg/mL, final concentration in drinking water), streptomycin (0.5 mg/mL, final concentration in drinking water), and colistin (0.1 mg/mL, final concentration in drinking water) (Sigma-Aldrich) to sterile drinking water. Solutions and bottles were changed 3 times and once weekly, respectively. The antibiotic activity was confirmed by 16S rRNA gene sequencing. Types of bacteria with a relative abundance of less than 0.01 are deleted in the Tg group. The duration of ABX treatment was slightly different based on the experimental settings. In the context of faecal microbia transplantation experiments, mice received 3 days of ATB before undergoing fecal microbiota transplantation the next day by oral gavage using animal feeding needles.

**Fecal microbiota transplantation (FMT) experiments.** FMT was performed by thawing faecal material. Then, 200 µL of the suspension was transferred by oral gavage into each ATB-pretreated recipient. FMT was performed 3 times in 5 days. Twelve-month-old C57 mice were first treated with an antibiotic cocktail in drinking water for 3 days, and then 40 mg of the mixed stool suspended in PBS was inserted by gavage into each mouse 3 times with a 2-day break in between. After 3 days, 4.2 µg Aβ 1-40 oligomer was injected into both sides of the hippocampus. The mice were sacrificed 3 days later and used for different analyses.

**Bioinformatics analysis.** Pathway analysis and biological function enrichment analysis were performed using the Kyoto Encyclopedia of Gene Genotype (KEGG). Data were enriched using the R package "DOSE", "GO.db", "GSEABase" and "ClusterProfiler". Only pathways with a false discovery rate (FDR) corrected p-value of < 0.05 were represented. The R package "ggcorrplot" was used for correlation analysis. The R package "igraph" was used to generate correlation circus graphs. The R packages "ggalluvial" and "networkD3" were used to perform bacterial flow diagrams and Sankey diagrams. The R package "Mfuzz" was used for trend cluster analysis. Other bioinformatics analysis was conducted using the online platform of the Majorbio I-Sanger Cloud (www.i-sanger.com). The ROC biomarker analysis and random forest classification were performed with MetaboAnalyst 4.0 (https://www.metaboanalyst.ca/).

**Flow cytometry sample preparation and analysis.** Mice were anesthetized, blood samples were collected into EDTA-containing tubes, and red blood cells were removed using 1 × red blood lysis buffer. Before tissue collection, the brains were perfused with ice-cold PBS to avoid sampling the circulating blood immune cells, and the brains were removed, chopped into pieces and dissected according to the introduction of the Adult Brain Dissociation Kit (Miltenyi, Cat No. 130-107-677) using the gentleMACS dissociator (Miltenyi Biotec). The brain homogenate was filtered through a 70-µm cell strainer and centrifuged at 300 × g for 5 min at 4 °C. The cell pellet was resuspended in cold PBS buffer and centrifuged again at 300 × g for 5 min at 4 °C. All samples were counted and adjusted to a density of 2-3 × 10⁶/100 µL, labeled with a Live/Dead kit for 30 min, and then centrifuged at 500 × g for 3 min at 4 °C. The cells were resuspended in 100 µL PBS buffer, blocked with anti-CD16/32 (Biolegend, Cat No. 101320) for 10 min, and incubated with the antibody according to the manufacturers' protocols at 4 °C for 30 min. The following antibodies were used in the FACS analysis: CD45 ( 30-F11 ) -APC-Cy7( 103116, Biolegend), CD11b(M1/70)-FITC(101205, Biolegend), CX3CR1 (SA011F11) -PE-Dazzle 594 (149014, Biolegend), F4/80 (BM8) -BV421 (123132, Biolegend), CD86 (IT2.2) -PE (305438, Biolegend), CD206 (15-2) -APC (321110, Biolegend), CD206 (15-2) -BV785 (321142, Biolegend), CD11c (N418) -PE-Cy7 (117318, Biolegend), CD8 (53-6.7) -Percp-Cy5.5(100734, Biolegend), Ly-6C(HK1.4)-PE-Dazzle 594(128044, Biolegend), Gr-1 (RB6-8C5) -Percp-Cy5.5 (108428, Biolegend), B220 (RA3-6B2) -BV421 (103240, Biolegend), CD19 (6D5) -PE (115508, Biolegend), CD49b (DX5) -PE-Cy7 (108922, Biolegend), CD4 (GK1.5) -PE-Cy7 (100422, Biolegend), CD4 (GK1.5) -FITC (100406, Biolegend), CXCR3 (CXCR3-173) -BV421 (126522, Biolegend), CCR4 (L291H4) -PE-Cy7( 359410, Biolegend), CCR6(29-2L17)-APC(129814, Biolegend), CD127 (A019D5) -PE (351304, Biolegend), CD25 (3C7) -Percp-Cy5.5 (101912, Biolegend), Live/Dead (423104, Biolegend) . Cells were added to 500 µL PBS buffer, centrifuged at 500 × g for 3 min at 4 °C and resuspended in 200 µL running buffer. Relevant negative control, Fluorescence Minus One (FMO) control and each fluorescence compensation sample were used to adjust fluorescence compensation and identify the populations of interest. Cells were acquired on a BD Aria III cytometer, and data were analyzed using FlowJo software.

**Antibody array.** The brain homogenates (from 20 mg tissue) were analyzed with a glass slide-based antibody cytokine array including 200 proteins (RayBiotech, GSM-CAA-4000-1). A 100 µL sample diluent was added to each well and incubated at room temperature for 30 min. Then, the buffer was replaced with another 100 µL of sample and incubated at room temperature for 2 h. The samples were discarded and washed 5 times (5 min each) with 150 µL of 1 × Wash Buffer I and 2 times (5 min each) with 150 µL of 1 × Wash Buffer II at room temperature with gentle shaking. 80 µL of the detection antibody cocktail were added to each well and incubated at room temperature for 2 h. The slide was washed 5 times (5 min each) with 150 µL of 1 × Wash Buffer I and then 2 times (5 min each) with 150 µL of 1 × Wash Buffer II at room temperature with gentle shaking. Eighty microliters of Cy3 equivalent dye-conjugated streptavidin was added to each well and incubated at room temperature for 1 h. After 5 washes (5 min each), the signal was visualized through a laser scanner. The data were then visualized by a heatmap diagram (www.metaboanalyst.ca).

**Brain section preparation**. Mice were transcardially perfused with 0.9% NaCl after deep anesthesia with pentobarbital (100 mg/kg, i.p.). Brain tissues were quickly removed, frozen and stored at -70 °C. Serial coronal brain sections (12 µm thickness) were created using a sliding, freezing microtome (Leica Microsystems), mounted on slides and dried overnight in the air at room temperature. Tissue sections were stored at -70 °C or used immediately.

**Laser microdissection and Q-PCR analysis.** Frozen mouse brain samples were sectioned and collected on PEN membrane slides (Leica, 11600288). The hippocampus was isolated by laser microdissection microscopy (Leica Microsystems, LMD6). RNA was extracted with a RNeasy Micro Kit (Qiagen, 74004) and reverse transcribed into cDNA (Takara, PrimeScript RT Master Mix, RR036A). Q-PCR was performed using the ABI 7500 system via the SYBR method (Takara, SYBR Premix Ex Taq II). Following primers were used: Synaptophysin-forward: CAGTTCCGGGTGGTCAAGG; Synaptophysin-reverse: ACTCTCCGTCTTGTTGGCAC; actin-forward: GCTCTTTTCCAGCCTTCCTT; actin-reverse: AGGTCTTTACGGAT GTCAACG.

**Statistical analysis.** In the behavior test, animals were randomly distributed into different groups. For two group comparisons, an unpaired two-tailed Student's t-test was applied. For more than two group comparisons, one-way ANOVA or two-way ANOVA followed by Dunnett's test was performed. All data with error bar are represented as mean ± SEM. P < 0.05 was considered statistically significant. Most of the data were analyzed in GraphPad Prism. For image quantification, Iba-1-positive, Aβ 42-positive and phosphorylated Tau-positive cells were analyzed by ImageJ v1.8.0 with 'area' readout.

**Behavioral tests.** The following behavioral tests were conducted: Morris water maze (Morris water maze, MWM), Y maze and elevated plus maze (EPM). All traces were recorded by using the camera, and the software (Jiliang) was used to calculate the relevant parameters.

The MWM test is used to measure spatial learning and memory according to a protocol published previously. Briefly, the mice underwent 6-day acquisition experiments, and each mouse performed 3 trials each day. The animals were released into the water at the desired start position, and the latency to find the platform was timed. On the 7th day, the platform was removed, and the mice were allowed to swim for 60 seconds. The trace and the number of platform-site crossovers were recorded using a video camera.

Working memory was assessed by the Y maze according to the literature with some modifications. The Y maze was composed of three identical arms (A, B, C) with different cues. Mice were placed in the start arm (A) and the sequence of explored arms was recorded (such as ABCBA). The total number of arm entries and alternation behavior were recorded using a video camera. The accuracy of the Y maze was the ratio between the correct alternation and the total alternation.

The EPM test is widely used to assess animal anxiety and consists of two open arms (30 cm × 6 cm), two closed arms (30 cm × 6 cm × 22 cm) and a central area (6 cm×6 cm). Each mouse was placed in the center of the maze facing the closed arm and allowed to explore the maze for 5 minutes. The trace was recorded and the frequency of visiting the open arm was calculated, which is negatively correlated with the anxiety of the mice.

### Examples

### Example 1: AD progression is associated with the alteration of gut microbiota and immune cell infiltration

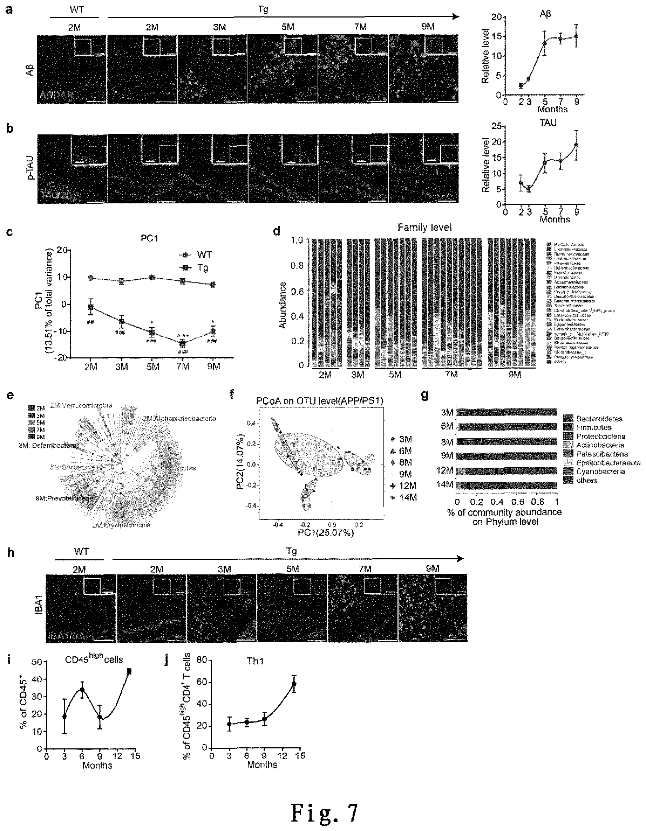

To assess the role of gut microbiota alteration in AD pathogenesis, the inventors used the 5XFAD transgenic (Tg) mouse model, which is widely used in AD study for its rapid onset and faithfully recapitulating multiple AD-associated pathological features and clinical phenotypes. It exhibits memory impairment in the 4th postnatal month, behavioural changes in the 7th month and loss of neurons in the 9th month. Age-matched wild-type (WT) mice from the same strain raised under the same conditions were used as controls. Consistent with previous reports, the inventors observed changes in Aβ plaque deposition, Tau phosphorylation, synaptophysin expression, behavior and the like. Specifically, Aβ plaque deposition in the cortex and hippocampus are rapidly accumulated beginning from the 3rd postnatal month (Figure 7a). Tau phosphorylation in the brain of Tg mice was first found in the 5th month and increased gradually with age (Figure 7b); The synaptophysin expression level in the hippocampus significantly decreased from the 7th to the 9th months, indicative of synaptic degeneration (Figure 1a). The behavioural test in Tg mice showed a significant decline in discrimination learning at 9 months of age (Figure 1b). Note that the early (2-3 months) and late (7-9 months) stages of Tg mice in the present invention are not the same concept as the early and late stages of human clinical AD. The late stage of Tg mice is symptomatically comparable to mild cognitive impairment (MCI) due to AD in humans. Therefore, in the present invention, patients with MCI due to AD are used as human research subjects.

A remarkable shift in the gut microbiota composition during AD progression in Tg mice was revealed with further principal component analysis (PCA) upon a series of time points, while hardly any changes were observed in WT mice over time (Figure 1c; Figure 7c).

Using OTUs to track the dynamics of the abundance of different bacterial phyla in Tg mice, the inventors found two distinct changes of gut microbiota profiles in Tg mice during disease progression. At 2-3 months of age, *Bacteroidetes, Firmicutes* and *Verrucomicrobia* were the three most abundant bacteria phylum (46.8%, 32.3% and 12.6%, respectively). At 7-9 months of age, *Firmicutes* became the predominant bacteria, while the abundance of *Bacteroidetes* and *Verrucomicrobia* markedly decreased, indicating an alteration of the types of bacteria (Figure 1d). The inventors further explored representative bacteria from Tg mice at each time point (Figure 7d, Figure 7e). These results indicated that the gut microbiota of Tg mice was highly dynamic, in great contrast to that of the WT mice. Similar results were also obtained in the APP/PS1 double transgenic mouse model, another widely used model for AD study (Figure 7f, Figure 7g).

The inventors hypothesized that the observed gut microbiota alteration during AD progression might be associated with neuroinflammation. To test this hypothesis, the inventors evaluated the inflammatory status of Tg mice. Immunostaining of IBA1, a hallmark of microglial activation, in AD mouse brain sections revealed two evident stages of microglial activation, at the 3rd month and the 7-9th months, respectively (Figure 1e). Given that microglial activation can be categorized into two opposite types, the pro-inflammatory M1 and the neuroprotective M2 subtype, the inventors also carefully characterized M1 and M2 phenotypes. At early stage of 2-3-month old, both M1 and M2 microglia increased. In the following months, M1 subtype continued to increase and peaked at 7-9 months, whereas M2 type microglia declined from 3 to 5 month and maintained a low level thereafter (Figure 1f). It can be seen that as the AD of Tg mice progresses over time, the pro-inflammatory M1 microglia subtype in the brain has changed from being comparable to the neuroprotective M2 microglia subtype (Figure If, 2-3 months) to being dominant in microglia (Figure If, 5-9 months) and leading to an increase in the overall activation of microglia (Figure 1e, 5-9 months).

In addition to microglia activation, AD-associated neuroinflammation is known to involve the infiltration of peripheral immune cells. The inventors therefore also analyzed the infiltrating peripheral immune cells in the brain during AD progression. It was observed that CD45^{high} cells in the brain was significantly higher in Tg mice than that in WT mice, similar to the result of IBA1 staining (Figure 1g). CD45^{high} cell subtypes at a series of time points were further analyzed during AD progression, revealing the alteration of CD4⁺ T cells, as the major proportion of CD45^{high} cells, closely has a similar alteration trend with IBA1 (Figure 1h-i). Over the time period the inventors explored, infiltrating Th1 and Th2 cells, two major subtypes of CD4⁺ cells, exhibited similar dynamics to that of M1 and M2 microglial cells (Figure 1j). It can be seen that in the progression of AD in Tg mice over time, Th1 has changed from being comparable to Th2 (Figure 1j, 2-3 months) to being dominant in CD4⁺ T cells (Figure 1j, 5-9 months), and leading to an overall increase in CD4⁺ T (Figure 1i, 5-9 months).

Therefore, it appeared to the inventors that, as the pattern of the gut microbiota shifted, the immune cell population tended to change to a Th1- and M1-dominated state, especially the relevance of these changes in time is striking (Figure 1d-j). Similar results were also found in the APP/PS1 mouse model (Figure 8, Figure 7i, Figure 7j, Figure 23). It should be pointed out that the APP/PS1 mouse model and the 5XFAD transgenic (Tg) mouse model are different in the progression of AD over time. Tg mice have obvious Aβ deposition at 5 months of age (Figure 7a), while APP/PS1 mice have no obvious Aβ deposition at 5 months of age (Figure 8a). The changes of the gut microbes of the two over time (Figure Id, Figure 7g) are not completely consistent, reflecting the intra-species and inter-individual AD progress and the differences in the gut microbes, but they all show a correlation between changes in gut microbial patterns and changes in immune cells over time.

The inventors also analyzed the correlation between gut microbiota abundance and brain immune cell frequency, and noted that early (2-3 months) bacterial composition is highly correlated with M2 and Th2 cell counts in the brain (Fig. 1k, top), but bacterial pattern changes were highly correlated with M1 and Th1 cells (Fig. 1k, bottom). The bacteria that were significantly interrelated with immune cells were listed in the right panel of Figure 1k, again verifying the correlation between the gut microbiota pattern and immune cells, especially Th1 and M1.

Overall, these results indicated that gut bacteria were associated with the infiltration of peripheral immune cells and neuroinflammation occurrence during AD progression, which will be further studied below.

### Example 2: Changes in the gut microbiota lead to excessive infiltration of Th1 cells and excessive activation of M1 microglia in the brain

To determine whether the gut microbiota change is required for driving peripheral immune cell infiltration and in turn neuroinflammation in AD progression, the inventors used an antibiotic cocktail containing ampicillin (0.1 mg/mL), streptomycin (0.5 mg/mL), and colistin (0.1 mg/mL) in Tg mice at late stage (7 months of age) to ablate gut microbiota. Antibiotic treatment in Tg mice at late stage (7 months of age) resulted in a marked reduction in both microbial diversity and abundance in the gut (Figure 2a).

Along with this change, the inventors observed a reduction in both infiltrating pro-inflammatory Th1 cells (Figure 2b) and M1 cells (Figure 2c) in the brain. This preliminarily proves that by interfering with the distribution of gut microbes in AD mice, the dominant state of Th1 and M1 in late-stage AD mice can be changed, indicating a causal relationship between changes in gut microbes and changes in immune cells.

Further, these findings were confirmed by a co-housing experiment. WT mice were co-housed with Tg mice of the same age for 7 months since birth. The co-housed WT mice displayed the decline in discrimination learning, comparable to that of Tg mice (Figure 9a). To find out why, analysis of the gut microbiota change in these 7-month-old mice indicated that the composition of the gut microbiota was quite similar between co-housed WT mice and Tg mice at the late stage (7-month-old), but significantly different from that of WT mice of the same age under separate housing (Figure 2d), indicating that the long-term exposure of WT mice to the Tg bacteria (i.e. the AD gut microbial model of Tg mice) caused the composition of the gut microbiota of WT mice to shift towards the direction of Tg mice, and at the same time caused cognitive impairment. This proposes a strategy to construct an AD model that is different from the conventional scheme, i.e., by regulating the gut microbiota pattern of the WT model to change to that of the AD model.

Moreover, infiltrating Th1 cells between co-housed WT and Tg mice were comparable as well, which were both significantly higher than that of separately housed WT mice (Figure 2e). Meanwhile, M1 cells were increased in the co-housed WT mice (Figure 2f). In line with the immune cell change, cytokine expression in the brain showed a marked similarity between co-housed WT and Tg mice, but distinct from those of WT mice (Figure 2g).

Therefore, the above description confirmed that by changing the gut microbiota of WT mice to that of Tg mice, the Th1 and M1 cells of WT mice were transformed into a state similar to that of Tg mice, resulting in similar cognitive impairments and once again verifying the causal relationship between changes in gut microbiota and changes in immune cells as well as cognition.

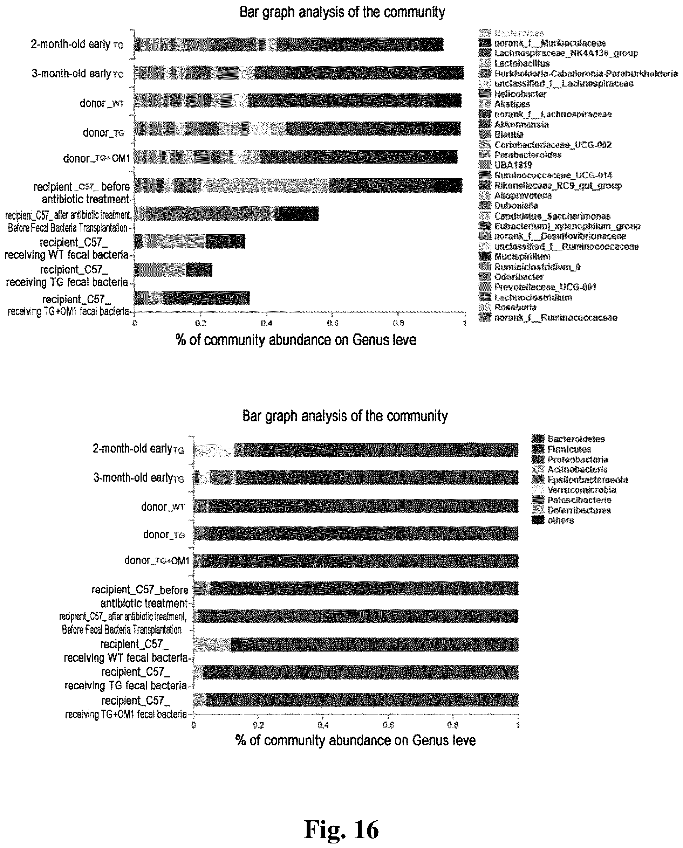

Furthermore, the inventors used C57BL/6WT mice to construct a model to demonstrate the therapeutic value of the above method. The inventors performed fecal microbiota transplantation (FMT) experiments using C57BL/6 WT mice, and the results are shown in columns 6 to 10 of Figure 16.

C57BL/6WT mice were treated with antibiotics to provide an initial environment for subsequent fecal microbiota transplantation. Aggregated Aβ was injected into its hippocampus, and then fecal bacteria from Tg mice aged 7 to 9 months ("Receptor_C57_ Receiving TG Fecal Bacteria") or fecal bacteria from WT mice ("Receptor_C57_Receive WT Fecal Bacteria") as controls were orally administered. This resulted in a significant increase in Th1 cells and a significant decrease in Th2 cells in the brain compared with the control (Figure 9b), which is consistent with the changes in Tg mice above.This proved that transplantation of Tg feces can lead to a change in the gut microbial pattern of WT mice, which also leads to a change in the immune cell pattern.

On the other hand, reversely, transplantation of feces from WT mice with normal gut microbial distribution pattern decreased Th1 cells in the brain of the recipient Tg mice (Figure 9c). This proved that the gut microbial pattern of Tg mice is changed to that of WT mice, and the immune cell pattern is also changed.

These results once again verified the causal relationship between changes in gut microbiota and changes in brain immune cells (especially Th1/M1) and cognition in the progress of AD. The strategy of using the brain-gut axis to treat AD was proposed and verified.

Together, these findings suggest that the gut microbiota alteration drives peripheral immune cell infiltration and neuroinflammatory activation in AD progression. By administering agents (for example, the WT feces with normal gut microbial distribution patterns or the OM1 exemplified below) for regulating the relative abundance of gut microbes to Tg mice of which gut microbial distribution shows a disease pattern, the gut microbial distribution of Tg mice is reconditioned toward the early and normal gut microbial distribution. This effectively reversed the immune cell pattern dominated by Th1/M1 in Tg mice, thereby improving cognition and treating AD. This provides a solid foundation and conclusive evidence for the further improvement of cognition and treatment of AD by reconditioning the distribution of gut microbes in human AD patients (for example, the strategy of use of agents to regulate the relative abundance of gut microbes) to reverse the immune cell pattern; as well as for the use of gut microbial distribution and/or immune cell status (for example, Th1 status (for example, proportion in the population), M1 status (for example, proportion in the population)) as markers of AD progression.

### Example 3 OM1 alleviates neuroinflammation by regulating the gut microbiota

The essential role of gut microbiota in AD progression revealed herein may suggest the therapeutic implications by the intervention of gut microbiota. In order to verify again, the inventors used OM1, which is a clinically proven anti-AD drug extracted from brown algae. OM1 is a mixture of acidic linear oligosaccharides with a degree of polymerization ranging from dimer to decamer, with an average molecular weight of about 1 kDa (Figure 3a).

In the late-stage AD model APP/PS1 mice from 9 months to 12 months of age treated with OM1 for 3 months, OM1 exhibited significant ameliorative effect on the cognitive impairment, as shown by the enhanced spatial learning and memory performance of APP/PS 1 mice in both training trial (Figure 3b) and probe trial (Figure 3c) in the Morris Water Maze (MWM) task. OM1 also significantly improved the mice performance in Y maze (Figure 3d). Recently, OM1 has shown the therapeutic effect on reversing cognition impairment in AD patients in a 36-week multi-center, randomized, double-blind, placebo-controlled Phase 3 clinical trial (Clinical trial code: CTR20140274) in China. The inventors are interested in understanding whether OM1 exerts a cognitive improvement effect by affecting the gut microbiota of AD patients.

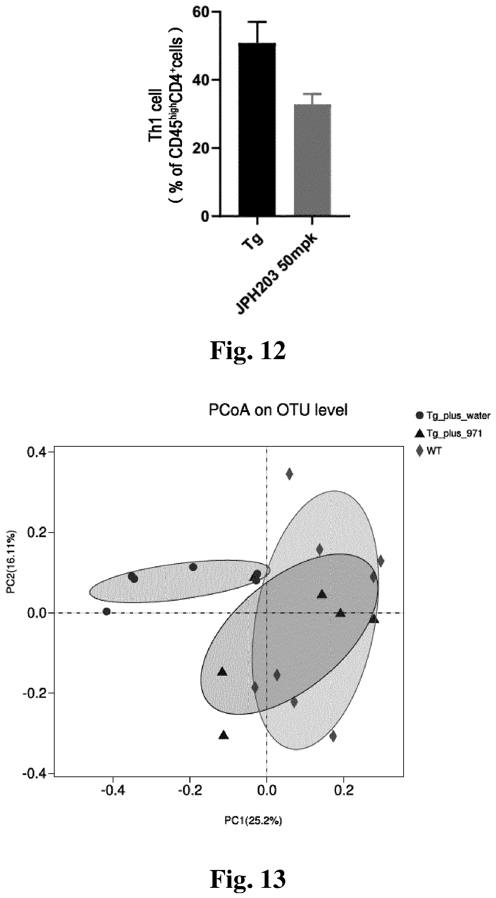

Intriguingly, one-month oral administration of OM1 in late-stage Tg mice beginning from 7-month-old age markedly altered the composition of gut microbiota (Figure 4a-b; Figure 10a), recovered to move closer to the WT pattern (Figure 13-14; Figure 16).

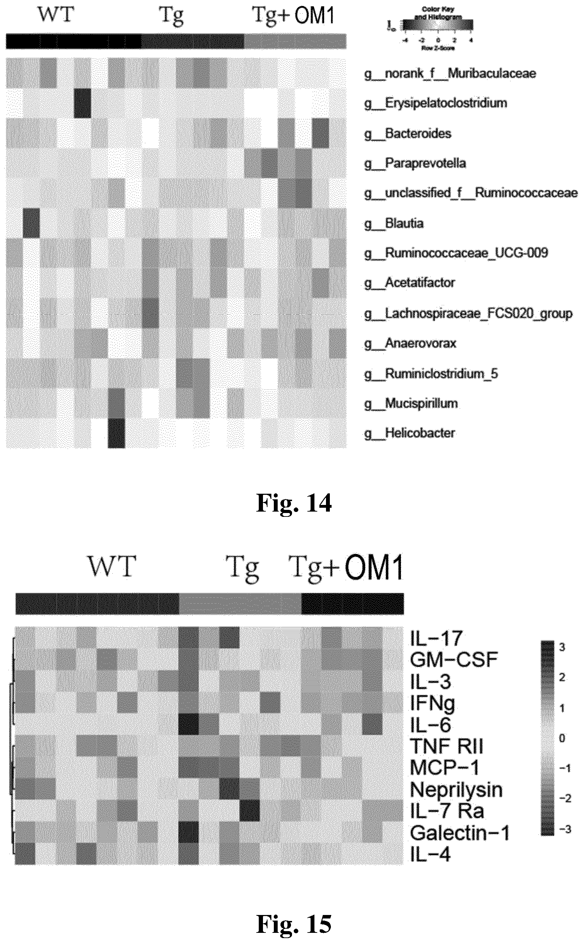

In line with the gut microbiota alteration, OM1 treatment in Tg mice disrupted the correlation between brain lymphocytes counts and gut bacterial change (Figure 4c; Figure 10b and c), decreased Th1 cells in the brain (Figure 4d), significantly reduced microglial activation to levels similar to WT mice (Figure 4e), and decreased brain cytokines levels (Figure 4f), recovered to move closer to the WT pattern (Figure 15). In parallel, OM1 treatment significantly reduced the Aβ plaque deposition, tau phosphorylation, and the decline in discrimination learning seen in Tg mice (Figure 4g-i). It has again verified the strategy of reconditioning the distribution of gut microbes, such as using agents for regulating the relative abundance of gut microbes, to reverse the immune cell pattern, thereby improving cognition and treating AD.

Further, the inventors did fecal microflora transplantation (FMT), transplanting feces of OM1-treated Tg mice to the recipient C57BL/6 WT mice which were intraventricularly injected with aggregated Aβ. Feces of OM1-treated Tg mice resulted in decreased Th1 cells in the brain of recipient mice as compared to that of Tg mice without OM1 treatment (Figure 10e). It can be seen that the feces from Tg mice treated with OM1 and the feces from WT mice verified in Example 2 have a similar effect on restoring the distribution of gut microbes and reducing Th1, which verifies the restoration effect of OM1 on the gut microbes of Tg mice. Consistently, antibiotic treatment impaired the effect of OM1 on Th1 cells, IBA1 levels and cytokine expression in the APP/PS1 mouse model (Figure 10f-j). All these data suggested that OM1 could alleviate neuroinflammation and cognition decline via modulating gut dysbiosis.

In general, the above *in vivo* experiments in mice have fully confirmed the changes in gut microbes and their regulation-immune cell Th1/M1 dominant trend and its reversal--cognitive disorders and their improvement, such as AD brain-gut axis regulation pathways and their intervention treatments. And such regulatory pathways and interventions are consistently demonstrated in a variety of different mouse models (5XFAD transgenic (Tg) mouse model, APP/PS 1 mouse model, C57BL/6 mouse model), although there are certain intra-species and inter-individual differences in the initial state and process transition of gut microbes among these mice. Therefore, the commonality of such regulatory pathways and interventions in different individuals is fully confirmed. Therefore, a strategy is proposed to improve cognition and treat AD by regulating the gut microbes to recondition the gut microbes to a normal and healthy mode to reverse the immune cell disease mode.

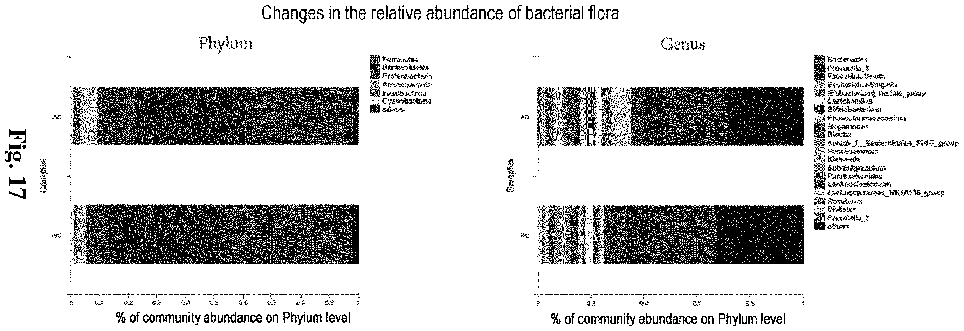

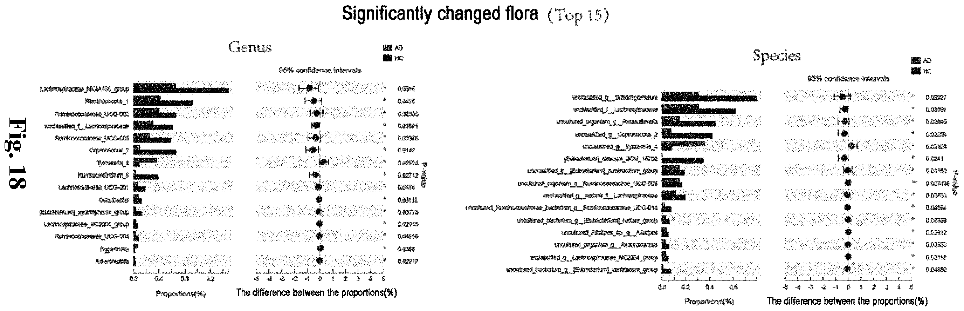

The inventors used the data of human AD patients and healthy controls to compare and analyze AD brain-gut axis-related gut microbes that are different between the two, as listed in tables 1-2 and as exemplified in Figures 17-18, as the target of regulation. When applying the aforementioned AD brain-gut axis treatment strategy to humans, in addition to the interspecies intestinal microbial differences between mice tested as rodents and humans as primates tested in the present invention, the intra-species and inter-individual gut microbial differences between AD patients should also be fully considered. Therefore, the types of mouse gut microbes specifically shown in the above experiments are more instructive, and should not be rigidly and mechanically applied to human AD patients.

### Example 4: OM1 inhibits neuroinflammation by regulating amino acid metabolism

The present invention also explores the intermediate link between the change of the gut microbiota and the change of immune cells and its intervention.

### The causality between the metabolites of gut microbes and the differentiation of naive T cells to Th1/Th2, and its intervention

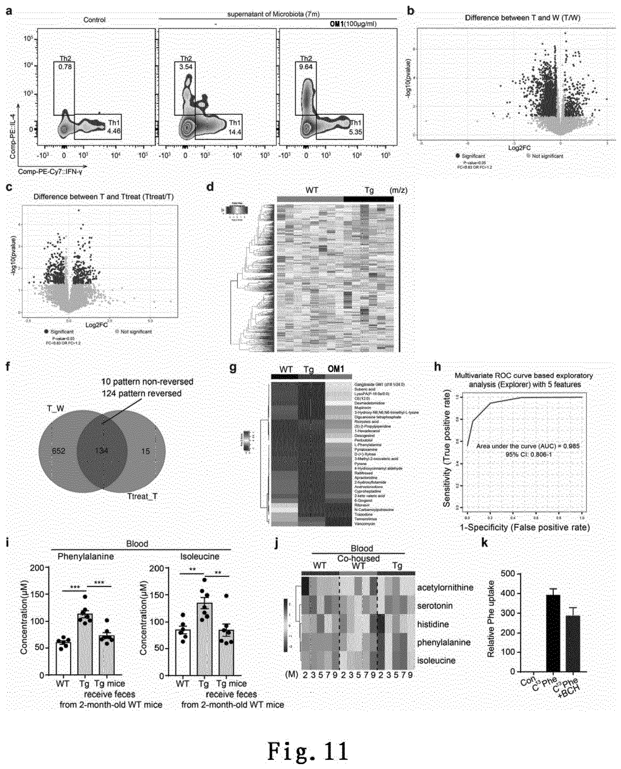

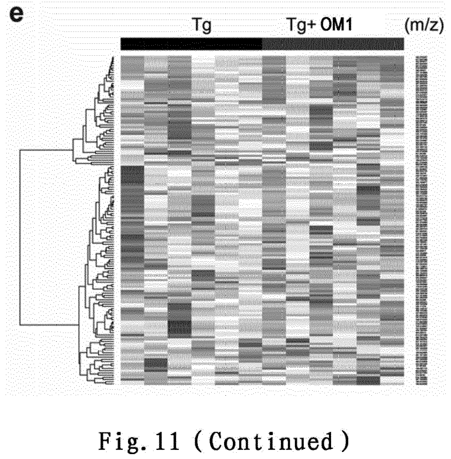

To test the possible involvement of metabolites in immune modulation, the supernatant of *in vitro*-cultured feces from 7-month-old Tg mice was added to naive CD4⁺ T cell culture, which stimulated the differentiation of naive CD4⁺ T cells to Th1 and Th2 cells. In contrast, fecal supernatant of Tg mice treated with OM1 promoted Th2 differentiation and inhibited Th1 differentiation (Figure 11a), indicating that the gut microbial metabolites affect the differentiation of naive T cells into Th1 and Th2 cells.

### The causality between the amino acids in the metabolites of gut microbes and the differentiation of naive T cells to Th1/Th2, and its intervention

The inventors next employed a non-targeted metabolomics technique to characterize the fecal metabolome. A total of 11289 metabolites were identified in fecal samples from WT, Tg and OM1-treated Tg mice (Figure 11b-c). Among those metabolites, the abundance of 124 metabolites, as annotated by METLIN database, was significantly changed, downregulated or upregulated in Tg mice relative to the WT mice, indicating it related to AD. Strikingly, all these metabolite changes can be reversed by OM1 treatment to a large extent (Figure 11d-f), indicating that the treatment of AD repaired the abnormal state of these AD-related metabolites. These altered metabolites were further annotated with the Human Metabolome Database (HMDB) and the Kyoto Encyclopaedia of Genes and Genomes (KEGG), yielding a total of 31 metabolites that were differentially regulated among WT, Tg and OM1-treated Tg mice, which could be matched to all three databases (HMDB, METLIN, KEGG). Pathway enrichment analysis of these metabolites using MBROLE or MetaboAnalyst further revealed significant changes in amino acid-related metabolic pathways and enzymes, especially phenylalanine-related pathways (Figure 5a).

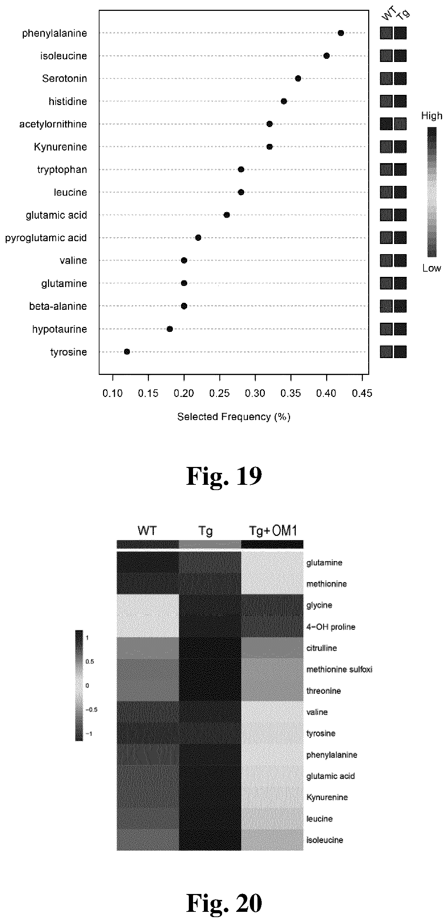

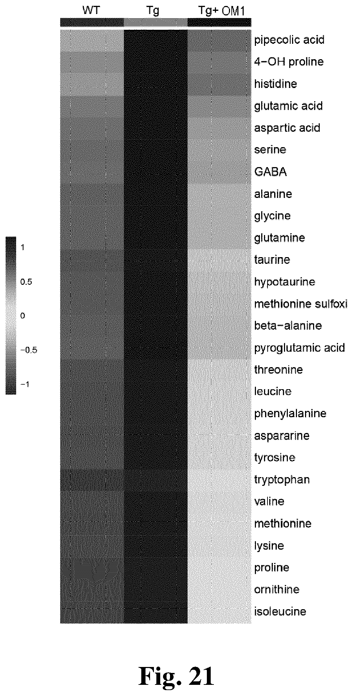

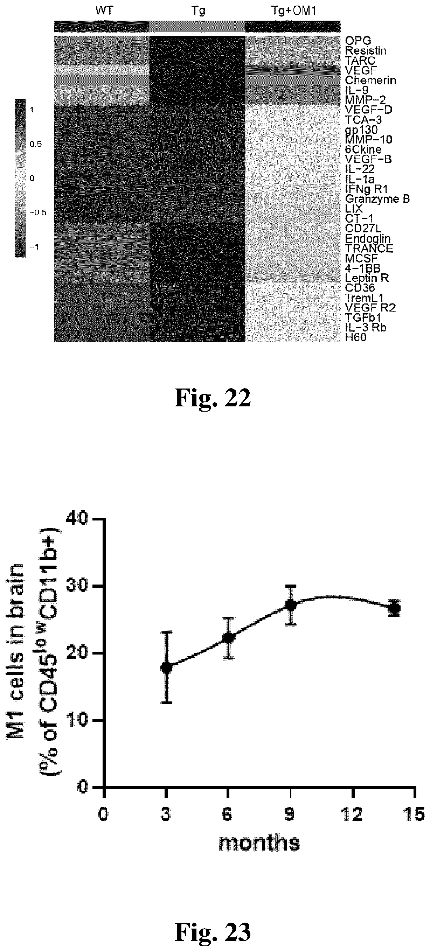

The inventors therefore chose to focus on amino acids for further study. Plasma concentrations of a total of 36 amino acids (Table 3) were screened in both WT and Tg mice. The random forest algorithm is used to classify these amino acids, and some of them are sorted as shown in Figure 19 and listed in Table 4. Phenylalanine was rated as the highest hit, followed by isoleucine, serotonin, histidine, and acetylornithine. The multivariate exploratory analysis of these five amino acids revealed significant differences between WT and Tg mice according to the receiver operating characteristic (ROC) curve (Figure 5b; Figure 11h), indicating that they are closely related to disease progression. The inventors then examined the concentration of the selected amino acids in the fecal and blood samples in OM1-treated or untreated Tg mice, and compared it with that of WT mice. Referring to Figure 20 and Figure 21, the inventors found that a variety of amino acids were affected by OM1 and recovered from the Tg pattern to the wild-type pattern. Espectially, the concentrations of phenylalanine and isoleucine were significantly higher in the feces of Tg mice than those of WT mice, and OM1 treatment significantly reduced their concentrations to a level comparable to that of WT mice (Figure 5c). A similar change mode in the abundance of phenylalanine and isoleucine was detected in blood (Figure 5d). Note that phenylalanine and isoleucine are the representatives of gut microbial metabolites that change with disease progression and are reversed by OM1. In addition, the inventors also found that after receiving OM1, the cytokines of mice have a tendency to recover to the wild-type pattern (Figure 22). This corresponds to the cytokine status of WT mice tending to change to the Tg mouse pattern as shown in Figure 2g when co-housed with Tg mice.

To test whether the elevation of these amino acids resulted from gut microbiota change thereby allowing the intervention of amino acids by interfering with the gut microbiota, the inventors examined the concentration of amino acids in FMT study. Feces from 2-month-old WT mice could significantly reduce the level of phenylalanine and isoleucine in Tg mice (Figure 11i). Likewise, in co-housed WT mice across described in Figure 2, phenylalanine and isoleucine concentrations in blood were also elevated, comparable to that of Tg mice (Figure 11j). Therefore, it can be determined that when WT mice co-housed with Tg mice to transform their own WT gut microbial pattern to Tg pattern, the abnormal production of phenylalanine and isoleucine occurred and the level of phenylalanine and isoleucine increased. On the contrary, transplanting WT mouse feces containing normal WT gut microbiota into Tg mice caused the Tg mouse gut microbiota to shift to WT pattern, resulting in the restoration of abnormal production of phenylalanine and isoleucine and the level of phenylalanine and isoleucine decreased. This confirmed that the levels of gut microbial metabolites phenylalanine and isoleucine can be reconditioned by restoring the gut microbiota of Tg mice.

### Verifying that phenylalanine and isoleucine affect the differentiation of naive T cells to Th1/Th2 and their intervention

To assess the effects of phenylalanine and isoleucine on cells, these two compounds were added directly to the naive CD4⁺ T cell culture. Both Th0 cell differentiation into Th1 cells (Figure 5e) and Th1 cell proliferation (Figure 5f) increased. Conversely, OM1 treatment can inhibit the differentiation of Th1 induced by phenylalanine and isoleucine and the proliferation of Th1 cells stimulated, indicating that OM1 not only affects gut microbes, but also directly affects its metabolite amino acid *per se.* In addition, the inventors treated WT mice with intraperitoneal injection of phenylalanine and isoleucine and found that the Th1 cell count in the blood increased significantly (Figure 5g), verifying that amino acids promote the differentiation of Th0 into Th1 *in vivo.* These results indicate that the accumulation of phenylalanine and isoleucine can increase the Th1 cell count in the blood.

### Intervention in the differentiation of naive T cells by interfering with the uptake of amino acids by naive T cells

Amino acids could be taken by immune cells through specific transporters. The inventors further examined the expression levels of SLC7A5, the transporter of phenylalanine and isoleucine, in naive CD4⁺ T cells and found that SLC7A5 was expressed in CD4⁺ T cells. Incubation of CD4⁺ T cells with ¹³C-labelled phenylalanine revealed the uptake of phenylalanine by CD4⁺ T cells, which could be blocked by a pharmacological inhibitor of SLC7A5, JPH 203 (Figure 11k), suggesting being able to inhibit the uptake of amino acids by immune cells by inhibiting amino acid transporters. The inventors further tested the resulting change in the proportion of Th1 cells. 6-month-old 5XFAD mice were given 50 mg/kg of JPH 203 or a vehicle control by intraperitoneal injection every day. One month after the administration, the mice were euthanized, and the proportion of pro-inflammatory Th1 cells in the brains of the mice in the control group and the JPH 203 treatment group was analyzed by flow cytometry. This was a representative, reflecting the neuroinflammation in the brain. The results are shown in Figure 13. Compared with the vehicle control group, JPH203 treatment for one month significantly downregulated the proportion of pro-inflammatory Th1 cells in the mouse brain, demonstrating that by inhibiting the amino acid transporter, the uptake of amino acids by immune cells is inhibited, thereby preventing the promotion of the differentiation of immune cells by amino acids and reducing neuroinflammation in the brain.

### Verifying abnormal levels of phenylalanine and isoleucine in human AD subjects

Finally, the inventor verifies this in humans, exploring whether the above findings could be recapitulated in MCI due to AD (see the method used in this study for definition of MCI used in this study) patients. Indeed, phenylalanine and isoleucine concentrations as well as Th1 cell counts in the blood of MCI subjects due to AD (n=9) were significantly higher than those in the age-matched healthy counterparts (n=18) (Figure 5h, i). The increased levels of both phenylalanine and isoleucine in the blood were also confirmed in another small MCI cohort due to AD (Figure 5j), thus allowing human diagnosis and treatment strategies to be designed based on this.

In summary, the inventors discovered that several metabolites in the gut microbial metabolites involved in the subsequent transition of immune cells to a Th1 dominant state. The inventors especially investigated the amino acids in the metabolites, especially phenylalanine and isoleucine, and confirmed the causal relationship between changes in the composition of the gut microbiota, abnormal production of phenylalanine and isoleucine, and excessive Th1 differentiation of naive T cells. By administering the WT fecal bacteria and OM1, which were confirmed above to recondition the abnormal gut microbiota of Tg mice to the normal WT gut microbiota, the abnormally high levels of phenylalanine and isoleucine were reduced and recovered to the normal level. Furthermore, the differentiation of Th0 cells into Th1 cells were inhibited, reversing the disease state dominated by Th1, confirming that gut microbial metabolites are the bridge between abnormal gut microbiota composition and abnormal immune cell state, and are part of the relationships between AD brain-gut axis. The inventors also tested other means of interfering with metabolites, such as reducing the level of metabolites or preventing the uptake of metabolites by immune cells, to intervene in the downstream abnormal immune cell state caused by gut microbial metabolites, and thus the cognitive state.

Although the principle of the present invention has been described above in conjunction with the preferred embodiments, it should be clearly understood that the description is only made by way of example and not as a limitation on the scope of the present invention.

**Table 1. List of altered flora in AD patients or mice**

| **Classification** | **Name in English** | **matched_name** |
|---|---|---|
| Phylum | Actinobacteria | p__Actinobacteria |
| Phylum | *Bacteroidetes* | p__*Bacteroidetes* |
| Phylum | *Firmicutes* | p__*Firmicutes* |
| Phylum | *Proteobacteria* | p__*Proteobacteria* |
| Phylum | Tenericutes | p__Tenericutes |
| Class | Beta*Proteobacteria* | *c__*Beta*Proteobacteria* |
| Order | Burkholderiales | o__Burkholderiales |
| Family | Bacteroidaceae | f__Bacteroidaceae |
| Family | Bifidobacteriaceae | f__Bifidobacteriaceae |
| Family | Burkholderiaceae | f__Burkholderiaceae |
| Family | Clostridiaceae | f__Clostridiaceae |
| Family | Desulfovibrionaceae | f__Desulfovibrionaceae |
| Family | Erysipelotrichaceae | f__Erysipelotrichaceae |
| Family | *Fusobacteria*ceae | f__*Fusobacteria*ceae |
| Family | Gemellaceae | f__Gemellaceae |
| Family | Helicobacteriaceae | f__Helicobacteraceae |
| Family | Lachnospiraceae | f__Lachnospiraceae |
| Family | Mogibacteriaceae | f__Mogibacteriaceae |
| Family | norank_Bacteroidales_S24-7 _group | f__Bacteroidales_S24-7_group |
| Family | Peptostreptococcaceae | f__Peptostreptococcaceae |
| Family | Prevotellaceae | f__Prevotellaceae |
| Family | Rikenellaceae | f__Rikenellaceae |
| Family | Ruminococcaceae | f__Ruminococcaceae |
| Family | Staphylococcaceae | f__Staphylococcaceae |
| Family | Turicibacteraceae | f__Turicibacteraceae |
| Family | Turicibacteriaceae | f__Turicibacteriaceae |
| Genus | Actinobacillus_actinomycete mcomitans | g__Actinobacillus_actinomycet emcomitans |
| Genus | Actinobacteria | g__Actinobacteria |
| Genus | Adlercreutzia | g__Adlercreutzia |
| Genus | Alistipes | g__Alistipes |
| Genus | Alternaria | g__Alternaria |
| Genus | Bacteroides | g__Bacteroides |
| Genus | Bifidobacteria | g__Bifidobacteria |
| Genus | Bifidobacterium | g__Bifidobacterium |
| Genus | Bilophila | g__Bilophila |
| Genus | Blautia | g__Blautia |
| Genus | Botrytis | g__Botrytis |
| Genus | Candida | g__Candida |
| Genus | cc115 | g__cc115 |
| Genus | Clostridium | g__Clostridium |
| Genus | Dialister | g__Dialister |
| Genus | E_coli_K99 | g__E_coli_K99 |
| Genus | Escherichia | g__Escherichia |
| Genus | Eubacterium_rectale | g__[Eubacterium]_rectale |
| Genus | Fusarium | g__Fusarium |
| Genus | Gemella | g__Gemella |
| Genus | Lactobacilli | g__Lactobacilli |
| Genus | Malassezia | g__Malassezia |
| Genus | Phascolarctobacterium | g__Phascolarctobacterium |
| Genus | Shigella | g__Shigella |
| Genus | SMB53 | g__SMB53 |
| Genus | Sutterella | g__Sutterella |
| Genus | Tannerella_forsythia | g__Tannerella_forsythia |
| Genus | Turicibacter | g__Turicibacter |
| Genus | | g__Escherichia-Shigella |

**Table 2. A list of flora with significant differences in the relative abundance of intestinal bacteria between AD patients and healthy controls (HC)**

| **Cla ssifi cati on** | **Genus name** | **AD-Mea n(% )** | **HC-Mea n(% )** | **Fold change in AD compared to HC** | **Up- or downregulated in AD compared to HC** |
|---|---|---|---|---|---|
| Phylum | p*__Firmicutes* | 39.41 | 44.07 | 0.8942591 | downregulated |
| Phyl um | p*__Bacteroidetes* | 38.0 3 | 39.9 | 0.9531328 | downregulated |
| Phyl um | p*__Proteobacteria* | 12.8 5 | 8.08 6 | 1.5891665 | upregulated |
| Phyl um | p__Actinobacteria | 5.06 5 | 3.52 2 | 1.4381034 | upregulated |
| Phyl um | p*__Fusobacteria* | 2.02 8 | 1.00 1 | 2.025974 | upregulated |
| Phyl um | p*__Cyanobacteria* | 0.77 71 | 1.22 7 | 0.6333333 | downregulated |
| Phyl um | p*__Verrucomicrobia* | 0.27 26 | 0.75 85 | 0.3593935 | downregulated |
| Clas s | c__Bacteroidia | 37.3 8 | 39.5 1 | 0.9460896 | downregulated |
| Clas s | c__Clostridia | 25.5 | 32.3 3 | 0.7887411 | downregulated |
| Clas s | c*__*Gamma*Proteobacte ria* | 10.1 9 | 4.73 4 | 2.1525137 | upregulated |
| Clas s | c__Bacilli | 5.57 5 | 6.60 6 | 0.8439298 | downregulated |
| Clas s | c__Negativicutes | 6.31 9 | 4.50 7 | 1.4020413 | upregulated |
| Clas s | c__Actinobacteria | 5.79 1 | 3.52 2 | 1.6442362 | upregulated |
| Clas s | c__Fusobacteriia | 2.37 9 | 1.00 1 | 2.3766234 | upregulated |
| Clas s | c*__*Beta*Proteobacteria* | 1.29 8 | 1.43 8 | 0.9026426 | downregulated |
| Clas s | c*__*Alpha*Proteobacteri a* | 1.10 4 | 1.34 9 | 0.818384 | downregulated |
| Ord er | o__Bacteroidales | 37.3 8 | 39.5 1 | 0.9460896 | downregulated |
| Order | o__Clostridiales | 25.43 | 32.24 | 0.7887717 | downregulated |
| Ord er | o__Enterobacteriales | 8.98 3 | 3.45 3 | 2.6015059 | upregulated |
| Ord er | o__Selenomonadales | 6.31 9 | 4.50 7 | 1.4020413 | upregulated |
| Ord er | o__Lactobacillales | 4.80 3 | 5.77 4 | 0.8318324 | downregulated |
| Ord er | o__Bifidobacteriales | 3.88 7 | 1.30 4 | 2.9808282 | upregulated |
| Ord er | o*__Fusobacteria*les | 2.37 9 | 1.00 1 | 2.3766234 | upregulated |
| Ord er | o__Burkholderiales | 1.01 2 | 1.15 8 | 0.8739206 | downregulated |
| Ord er | o__Bacillales | 0.77 07 | 0.82 87 | 0.9300109 | downregulated |
| Fam ily | f__Rikenellaceae | 0.50 28 | 1.01 8 | 0.4939096 | downregulated |
| Fam ily | f__Ktedonobacteraceae | 0.00 018 7 | 0.00 176 9 | 0.1057094 | downregulated |
| Fam ily | f__Nannocystaceae | 0 | 0.00 194 7 | 0 | downregulated |
| Gen us | g__Lachnospiraceae_N K4A136_group | 0.55 82 | 1.47 8 | 0.3776725 | downregulated |
| Gen us | g__Alistipes | 0.42 37 | 0.92 65 | 0.4573125 | downregulated |
| Gen us | g__Ruminococcus_1 | 0.27 83 | 0.91 67 | 0.303589 | downregulated |
| Gen us | g__Ruminococcaceae_ UCG-002 | 0.42 32 | 0.66 21 | 0.6391784 | downregulated |
| Gen us | g__Ruminococcaceae_ UCG-005 | 0.25 49 | 0.58 89 | 0.4328409 | downregulated |
| Genus | g__Coprococcus_2 | 0.1136 | 0.6677 | 0.1701363 | downregulated |
| Gen us | g__Tyzzerella_4 | 0.41 65 | 0.08 026 | 5.1893845 | upregulated |
| Gen us | g__Lachnospiraceae_U CG-001 | 0.04 241 | 0.17 47 | 0.242759 | downregulated |
| Gen us | g__Anaerotruncus | 0.08 045 | 0.13 42 | 0.5994784 | downregulated |
| Gen us | g__Cloacibacterium | 0.00 528 4 | 0.00 185 1 | 2.8546731 | upregulated |
| Gen us | g__norank_f__Ktedono bacteraceae | 0.00 018 7 | 0.00 176 9 | 0.1057094 | downregulated |
| Gen us | g__Nannocystis | 0 | 0.00 194 7 | 0 | downregulated |
| Gen us | g__norank_f__Hydroge nophilaceae | 0.00 143 5 | 0.00 028 94 | 4.9585349 | upregulated |
| Spe cies | s__unclassified_g__Sub doligranulum | 0.11 08 | 0.79 63 | 0.1391435 | downregulated |
| Spe cies | s__unclassified_g__Ali stipes | 0.24 12 | 0.64 16 | 0.3759352 | downregulated |
| Spe cies | s__uncultured_organis m_g__Parasutterella | 0.09 518 | 0.44 42 | 0.2142729 | downregulated |
| Spe cies | s__unclassified_g__Tyz zerella_4 | 0.41 65 | 0.08 026 | 5.1893845 | upregulated |
| Spe cies | s__uncultured_organis m_g__Ruminococcacea e_UCG-005 | 0.16 49 | 0.17 28 | 0.9542824 | downregulated |
| Spe cies | s__uncultured_organis m_g__Anaerotruncus | 0.02 364 | 0.05 794 | 0.4080083 | downregulated |
| Spe cies | s__uncultured_Alistipes _sp._g__Alistipes | 0.02 727 | 0.04 977 | 0.5479204 | downregulated |
| Spe cies | s__Lachnospiraceae_ba cterium_TF01-11 | 0.00 962 8 | 0.05 391 | 0.178594 | downregulated |
| Spe cies | s__unclassified_g__An aerotruncus | 0.01 064 | 0.01 785 | 0.5960784 | downregulated |
| Spe cies | s__unclassified_g__nor ank_o__Mollicutes_RF 9 | 0.00 379 4 | 0.01 637 | 0.2317654 | downregulated |
| Spe cies | s__uncultured_bacteriu m_g__Family_XIII_AD 3011_group | 0.00 809 4 | 0.00 795 1 | 1.0179852 | upregulated |
| Spe cies | s__uncultured_bacteriu m_g__norank_f__Chris tensenellaceae | 0.00 721 4 | 0.00 784 1 | 0.9200357 | downregulated |
| Spe cies | s__uncultured_bacteriu m_g__Cloacibacterium | 0.00 528 4 | 0.00 185 1 | 2.8546731 | upregulated |
| Spe cies | s__uncultured_organis m_g__Peptococcus | 0 | 0.00 637 4 | 0 | downregulated |
| Spe cies | s__Mycobacterium_cel atum_g__Mycobacteriu m | 0.00 115 | 0.00 516 3 | 0.2227387 | downregulated |
| Spe cies | s__unclassified_g__Oce anobacillus | 0.00 482 7 | 0.00 101 8 | 4.7416503 | upregulated |
| Spe cies | s__Alistipes_putredinis _DSM_17216 | 0.00 018 7 | 0.00 356 8 | 0.0524103 | downregulated |
| Spe cies | s__Prevotella_loescheii | 0.00 263 5 | 0.00 046 72 | 5.6399829 | upregulated |
| Spe cies | s__uncultured_bacteriu m_g__norank_o__MBA03 | 0.00 2428 | 0.00 04532 | 5.3574581 | upregulated |
| Spe cies | s__Eubacterium_brachy | 0.00 244 3 | 0.00 016 14 | 15.136307 | upregulated |
| Spe cies | s__uncultured_bacteriu m_adhufec108 | 0 | 0.00 217 3 | 0 | downregulated |
| Spe cies | s__uncultured_Clostridi ales_bacterium_g__nor ank_f__Ktedonobactera ceae | 0.00 018 7 | 0.00 176 9 | 0.1057094 | downregulated |
| Spe cies | s__Nannocystis_pusilla | 0 | 0.00 194 7 | 0 | downregulated |
| Spe cies | s__bacterium_2013Ark 19i | 0.00 143 5 | 0.00 028 94 | 4.9585349 | upregulated |
| Spe cies | s__Auxenochlorella_pr otothecoides_g__noran k | 0.00 146 3 | 0.00 014 47 | 10.110574 | upregulated |
| Spe cies | s*__*uncultured*_Bacteroi detes*_bacterium_g__Di nghuibacter | 0 | 0.00 092 68 | 0 | downregulated |

**Table 3 The following lists present the name of amino acids detected in the blood of WT (2-9 months) and Tg (2-9 months) mice. Related to Figure 5b.**

| **Name of amino acids** |
|---|
| glycine |
| alanine |
| Serine |
| Valine |
| 4-OH proline |
| Aspararine |
| Glutamine |
| Tyrosine |
| Hypotaurine |
| methionine sulfoxide |
| beta-alanine |
| Threonine |
| Leucine |
| Ornithine |
| aspartic acid |
| Methionine |
| Histidine |
| Phenylalanine |
| Arginine |
| Proline |
| Taurine |
| Isoleucine |
| glutamic acid |
| Citrulline |
| Tryptophan |
| Kynurenine |
| GABA |
| Lysine |
| pyroglutamic acid |
| Acetylornithine |
| asymmetric dimethylarginine |
| alpha-aminoadipic acid |
| Carnosine |
| Creatinine |
| Putrescine |
| Serotonin |

**Table 4. ROC analysis of all amino acid markers of amino acids in blood of WT mice and Tg mice in order of selection frequency**

| Amino Acid | Rank Freq. | WT | Tg |
|---|---|---|---|
| phenylalanine | 0.42 | low | high |
| isoleucine | 0.4 | low | high |
| Serotonin | 0.36 | low | high |
| histidine | 0.34 | low | high |
| acetylornithine | 0.32 | high | low |
| Kynurenine | 0.32 | low | high |
| tryptophan | 0.28 | low | high |
| leucine | 0.28 | low | high |
| glutamic acid | 0.26 | low | high |
| pyroglutamic acid | 0.22 | low | high |
| valine | 0.2 | low | high |
| glutamine | 0.2 | low | high |
| beta-alanine | 0.2 | low | high |
| hypotaurine | 0.18 | low | high |
| tyrosine | 0.12 | low | high |
| asymmetric dimethylarginine | 0.12 | high | low |
| glycine | 0.1 | low | high |
| Carnosine | 0.08 | low | high |
| 4-OH proline | 0.08 | low | high |
| aspartic acid | 0.06 | high | low |
| threonine | 0.06 | low | high |
| alpha-aminoadipic acid | 0.04 | low | high |
| ornithine | 0.04 | low | high |
| Creatinine | 0.04 | low | high |
| alanine | 0.04 | low | high |
| GABA | 0.02 | low | high |
| methionine sulfoxide | 0.02 | low | high |
| proline | 0.02 | low | high |

## Claims

1. Use of an agent for regulating therelative abundance of gut microbes in the manufacture of a medicament for treating Alzheimer's disease in a subject.

2. A pharmaceutical composition for treating Alzheimer's disease in a subject comprising an effective amount of an agent for regulating the relative abundance of gut microbes.

3. The use according to claim 1 and the pharmaceutical composition according to claim 2, wherein the gut microbes are selected from one or more of *Firmicutes, Bacteroidetes, Proteobacteria, Actinomycetes, Fusobacteria, Cyanobacteria, Verrucomicrobia,* or a combination thereof.

4. The use according to claim 1 and the pharmaceutical composition according to claim 2, wherein the agent is selected from carbohydrate drugs, gut microbes complexes, or a combination thereof; wherein the carbohydrate drug is selected from monosaccharides, disaccharides, oligosaccharides, polysaccharides, or derivatives thereof, or a combination of them and/or derivatives thereof; preferably oligosaccharides and polysaccharides; more preferably mannuronic acid oligosaccharides or a composition comprising mannuronic acid oligosaccharides; wherein the gut microbes complex comprises one or more selected from *Firmicutes, Bacteroidetes, Proteobacteria, Actinomycetes, Fusobacteria, Cyanobacteria, Verrucomicrobia,* or a combination thereof.

5. The use according to claim 1 and the pharmaceutical composition according to claim 2, wherein the agent regulates the relative abundance of gut microbes of the subject by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; and/or makes the relative abundance of gut microbes of the subject close to or reach the relative abundance of the corresponding gut microbes of the corresponding normal subject.

6. The use according to claim 1 or the pharmaceutical composition according to claim 2, wherein the regulating the relative abundance of gut microbes is to increase the relative abundance of one or more gut microbes and/or reduce the relative abundance of one or more gut microbes.

7. A method for screening a drug candidate that can be used to treat Alzheimer's disease comprising:
a) administering a test agent to *in vivo* or *in vitro* models with gut microbes, and
b) selecting the test agent that regulates the relative abundance of gut microbes as the drug candidate that can be used to treat Alzheimer's disease.

8. The method according to claim 7, further comprising administering OM1 as a positive control to an *in vivo* or *in vitro* model with gut microbes, preferably selecting a test agent that regulates the relative abundance of gut microbes substantially consistently with the OM1.

9. The method according to claim 7, further comprising administering a selected test agent to an *in vivo* or *in vitro* model with gut microbes for verification, wherein the selected test agent regulates the relative abundance of gut microbes by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; and/or makes the relative abundance of gut microbes close to or reach the relative abundance of the corresponding gut microbes in the corresponding normal *in vivo* or *in vitro* model.

10. The method according to claim 7, wherein the gut microbes are selected from one or more of *Firmicutes, Bacteroidetes, Proteobacteria, Actinomycetes, Fusobacteria, Cyanobacteria, Verrucomicrobia,* or a combination thereof.

11. A method for establishing an animal model of Alzheimer's disease comprising administering an agent for regulating the relative abundance of gut microbes to an animal, so that the relative abundance of gut microbes of the animal is regulated by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more; or close to or reach the relative abundance of the corresponding gut microbes of the corresponding animal model having Alzheimer's disease.

12. The method according to claim 11, wherein the gut microbes are selected from one or more of *Firmicutes, Bacteroidetes, Proteobacteria, Actinomycetes, Fusobacteria, Cyanobacteria, Verrucomicrobia,* or a combination thereof.

13. The method according to claim 11, further comprising administering the aggregated Aβ protein to the animal, preferably via hippocampal injection.

14. A method for treating a patient having Alzheimer's disease comprising:
(a) detecting the relative abundance of gut microbes of the patient and comparing it with the relative abundance of the corresponding gut microbes of the corresponding normal population to select gut microorganism of which the relative abundance is different from that of the corresponding gut microorganism of the corresponding normal population;
(b) administering an agent for regulating the relative abundance of gut microbes to the patient to regulate the relative abundance of selected gut microorganism to make them close to or reach the relative abundance of the corresponding gut microorganism of the corresponding normal population.
